# EUROPEAN PATENT APPLICATION

(11) **EP 4 450 502 A1**
(43) Date of publication of application: **23.10.2024**
(21) Application number: 22907545.2
(22) Date of filing: 16.12.2022
(51) Int. Cl.: C07D 471/04, A61K 31/437, A61P 1/00, A61P 1/16, A61P 3/10, A61P 9/00, A61P 9/10, A61P 11/00, A61P 11/02, A61P 11/06, A61P 13/12, A61P 17/00, A61P 17/02, A61P 19/02, A61P 21/00, A61P 29/00, A61P 37/08, A61P 43/00

(54) **AZAINDOLE DERIVATIVE INHIBITING H-PGDS**

(30) Priority: 17.12.2021 JP 2021204998
(71) Applicant: Sato Pharmaceutical Co., Ltd., Tokyo 107-0051 (JP)
(72) Inventor: BABA Motoaki, Tokyo 140-0011 (JP); OKUI Takuma, Tokyo 140-0011 (JP); ITOH Yoshiki, Tokyo 140-0011 (JP)
(74) Representative: Gulde & Partner
(86) International application number: PCT/JP2022/046507
(87) International publication number: WO 2023/113023

(57) **Abstract**

The present invention provides a compound having a hematopoietic prostaglandin D synthase (H-PGDS) inhibitory activity and being useful for preventing or treating a disease involving the enzyme, and a pharmaceutical composition comprising the compound. Specifically, the present invention provides a compound represented by Formula (I) below or a pharmaceutically acceptable salt thereof: 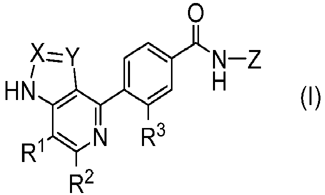 [wherein -X= represents -CH= or the like; =Y- represents =CH- or the like; R¹ and R² each independently represent a hydrogen atom or the like; R³ represents a hydrogen atom or the like; and Z is a group represented by Formula (II): 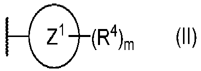 (wherein the wavy line represents the point of attachment to the nitrogen atom; ring Z¹ represents a C₆₋₁₀ aryl group or the like; m represents 0, 1, or 2; and R⁴ represents a halogen atom or the like.)
or Formula (III): 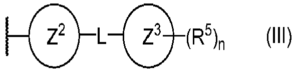 (wherein the wavy line represents the point of attachment to the nitrogen atom; ring Z² represents a C₆₋₁₀ aryl group or the like; L represents a single bond or the like; ring Z³ represents a C₆₋₁₀ aryl group or the like; n represents 0, 1, or 2; and R⁵ represents a halogen atom or the like.]

## Description

### Technical Field

The present invention relates to an azaindole derivative that inhibits H-PGDS and is useful in the pharmaceutical field. Specifically, the present invention relates to an azaindole derivative having a hematopoietic prostaglandin D synthase inhibitory activity and being useful for preventing or treating a disease involving the enzyme, a pharmaceutical composition comprising the compound, and an inhibitor of the enzyme.

### Background Art

Prostaglandin D synthase is an enzyme that converts prostaglandin H₂, which is a common intermediate of various prostaglandins, into prostaglandin D₂.

Among prostaglandin D synthases, hematopoietic prostaglandin D synthase (hematopoietic PGD synthase, H-PGDS) distributed in mast cells, Th2 lymphocytes and the like is known to be involved in various diseases (for example, allergic diseases, inflammatory diseases, neuromuscular related diseases, and ischemic diseases) via the production of prostaglandin D₂.

Therefore, attempts have been made to develop pharmaceutical compounds that treat the various diseases by inhibiting H-PGDS (Patent Literatures 1 to 23 and Non-Patent Literatures 1 to 3).

### Citation List

### Patent Literature

Patent Literature 1: WO 2019/203296
Patent Literature 2: JP 2013-14520 A
Patent Literature 3: WO 2012/033069
Patent Literature 4: WO 2011/090062
Patent Literature 5: WO 2010/104024
Patent Literature 6: WO 2007/007778
Patent Literature 7: JP 2007-51121 A
Patent Literature 8: WO 2020/095215
Patent Literature 9: WO 2019/116256
Patent Literature 10: WO 2018/229629
Patent Literature 11: WO 2018/069863
Patent Literature 12: WO 2017/103851
Patent Literature 13: WO 2011/044307
Patent Literature 14: WO 2008/121670
Patent Literature 15: WO 2007/041634
Patent Literature 16: WO 2011/150457
Patent Literature 17: WO 2010/033977
Patent Literature 18: WO 2009/153721
Patent Literature 19: WO 2009/153720
Patent Literature 20: WO 2008/104869
Patent Literature 21: WO 2008/075172
Patent Literature 22: WO 2005/094805
Patent Literature 23: WO 2017/209272

### Non Patent Literature

Non Patent Literature 1: Bioorg. Med. Chem. Lett. 2012, 22, 3795-3799
Non Patent Literature 2: J. Med. Chem. 2010, 53, 5536-5548 Non Patent Literature 3: Bioorg. Med. Chem. Lett. 2021, 34, 127759

### Summary of Invention

### Technical Problem

An object of the present invention is to provide a novel azaindole derivative having an excellent H-PGDS inhibitory activity and being useful for treating and preventing a disease involving H-PGDS.

### Solution to Problem

In order to solve the above problem, the present inventors have extensively synthesized and studied novel compounds, and as a result, have found that an azaindole derivative represented by Formula (I) below has excellent H-PGDS inhibitory activity, and have completed the present invention.

Thus, the present invention relates to a compound represented by Formula (I): 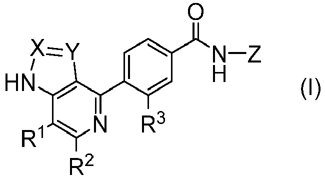 [wherein
-X= represents -CH= or -N=;
=Y- represents =CH- or =N-;
R¹ and R² each independently represent a hydrogen atom, a halogen atom, a cyano group, a hydroxy group, a sulfanyl group, an amino group, a carbamoyl group, a sulfamoyl group, a carboxy group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, or a C₃₋₆ cycloalkyl group;
R³ represents a hydrogen atom or a halogen atom; and
Z is a group represented by Formula (II): 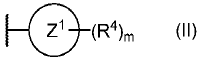 (wherein
   the wavy line represents the point of attachment to the nitrogen atom;
   ring Z¹ represents a C₆₋₁₀ aryl group, a C₃₋₁₀ cycloalkyl group, a 5- to 10-membered heteroaryl group, or a 4- to 10-membered heterocyclyl group;
   m represents 0, 1, or 2; and
   R⁴ represents (when there are multiple R⁴'s, each R⁴ independently represents) a halogen atom, a cyano group, a hydroxy group, a sulfanyl group, an amino group, a carbamoyl group, a sulfamoyl group, a carboxy group, a formyl group, an azide group, a hydrazinyl group, a nitro group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, a C₂₋₇ alkanoyl group, a halo C₂₋₇ alkanoyl group, a hydroxy C₂₋₇ alkanoyl group, a C₂₋₇ alkoxycarbonyl group, a mono C₁₋₆ alkylcarbamoyl group, a di C₁₋₆ alkylcarbamoyl group, a C₁₋₆ alkylsulfonyl group, a C₁₋₆ alkylsulfonyloxy group, a halo C₁₋₆ alkylsulfonyloxy group, a mono C₁₋₆ alkylsulfamoyl group, a di C₁₋₆ alkylsulfamoyl group, a mono C₂₋₇ alkanoylamino group, a (C₂₋₇ alkanoyl) C₁₋₆ alkylamino group, a di C₂₋₇ alkanoylamino group, a mono C₁₋₆ alkylsulfonylamino group, a mono C₂₋₇ alkoxycarbonylamino group, or an oxo group.)
      or Formula (III): 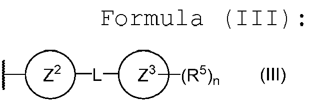 (wherein
      the wavy line represents the point of attachment to the nitrogen atom;
      ring Z² represents a C₆₋₁₀ aryl group, a C₃₋₁₀ cycloalkyl group, a 5- to 10-membered heteroaryl group, or a 4- to 10-membered heterocyclyl group;
      L represents a single bond, a C₁₋₆ alkanediyl group, a hydroxy C₁₋₆ alkanediyl group, a carbonyl group, or a sulfonyl group;
      ring Z³ represents a C₆₋₁₀ aryl group, a C₃₋₁₀ cycloalkyl group, a 5- to 10-membered heteroaryl group, or a 4- to 10-membered heterocyclyl group;
      n represents 0, 1, or 2; and
      R⁵ represents (when there are multiple R⁵'s, each R⁵ independently represents)
      a halogen atom, a cyano group, a hydroxy group, a sulfanyl group, an amino group, a carbamoyl group, a sulfamoyl group, a carboxy group, a formyl group, an azide group, a hydrazinyl group, a nitro group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, a C₂₋₇ alkanoyl group, a halo C₂₋₇ alkanoyl group, a hydroxy C₂₋₇ alkanoyl group, a C₂₋₇ alkoxycarbonyl group, a mono C₁₋₆ alkylcarbamoyl group, a di C₁₋₆ alkylcarbamoyl group, a C₁₋₆ alkylsulfonyl group, a C₁₋₆ alkylsulfonyloxy group, a halo C₁₋₆ alkylsulfonyloxy group, a mono C₁₋₆ alkylsulfamoyl group, a di C₁₋₆ alkylsulfamoyl group, a mono C₂₋₇ alkanoylamino group, a (C₂₋₇ alkanoyl) C₁₋₆ alkylamino group, a di C₂₋₇ alkanoylamino group, a mono C₁₋₆ alkylsulfonylamino group, a mono C₂₋₇ alkoxycarbonylamino group, an oxo group, a C₃₋₆ cycloalkyl group, a cyclic ether group, a cyclic amino group, or a halo cyclic amino group.)] or a pharmaceutically acceptable salt thereof (hereinafter, also referred to as "compound of the present invention").

Further, the present invention relates to a pharmaceutical composition comprising the compound of the present invention, particularly a pharmaceutical composition for use in the treatment or prevention of a disease involving H-PGDS.

Further, the present invention relates to an H-PGDS inhibitor comprising the compound of the present invention.

### Advantageous Effects of Invention

The compound of the present invention has excellent H-PGDS inhibitory activity as shown in Examples described later, and thus is useful as a therapeutic agent or a preventive agent for a disease involving H-PGDS.

### Description of Embodiments

Hereinafter, the definitions of the terms used to specify the compound of the present invention will be described.

Examples of the "halogen atom" in Formula (I) (Formula (II) and Formula (III) in Formula (I) are included. The same applies hereinafter.) include a fluorine atom, a chlorine atom, a bromine atom, and an iodine atom.

The "C₁₋₆ alkyl group" in Formula (I) means a linear or branched alkyl group having 1 to 6 carbon atoms, and examples thereof include a methyl group, an ethyl group, a propyl group, an isopropyl group, a butyl group, an isobutyl group, a sec-butyl group, a tert-butyl group, a pentyl group, an isopentyl group, a neopentyl group, a 1,1-dimethylpropyl group, a 1-methylbutyl group, a 2-methylbutyl group, a 1,2-dimethylpropyl group, a 1-ethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, and a 1,2,2-trimethylpropyl group.

The "C₂₋₆ alkenyl group" in Formula (I) means a linear or branched alkenyl group having 2 to 6 carbon atoms, and examples thereof include a vinyl group, a 1-propenyl group, an allyl group, an isopropenyl group, a 3-butenyl group, a 2-butenyl group, a 1-butenyl group, a 1-methyl-2-propenyl group, a 1-methyl-1-propenyl group, a 1-ethyl-1-ethenyl group, a 2-methyl-2-propenyl group, a 2-methyl-1-propenyl group, a 3-methyl-2-butenyl group, and a 4-pentenyl group.

The "halo C₁₋₆ alkyl group" in Formula (I) means the "C₁₋₆ alkyl group" in which any substitutable positions are substituted with 1 or 2 or more, preferably 1 to 5, same or different "halogen atoms" above, and examples thereof include a fluoromethyl group, a difluoromethyl group, a trifluoromethyl group, a 2-fluoroethyl group, a 1,2-difluoroethyl group, a 2,2,2-trifluoroethyl group, a 2,2,3,3,3-pentafluoropropyl group, a chloromethyl group, a 2-chloroethyl group, a 1,2-dichloroethyl group, a bromomethyl group, and an iodomethyl group.

The "hydroxy C₁₋₆ alkyl group" in Formula (I) means the "C₁₋₆ alkyl group" in which any substitutable positions are substituted with 1 or 2 or more, preferably 1 or 2 hydroxy groups, and examples thereof include a hydroxymethyl group, a 1-hydroxyethyl group, a 1-hydroxypropyl group, a 2-hydroxyethyl group, a 2-hydroxypropyl group, a 1-hydroxypropan-2-yl group (2-hydroxy-1-methylethyl group), a 2-hydroxypropan-2-yl group (1-hydroxy-1-methylethyl group), a 1,2-dihydroxyethyl group, and a 3-hydroxypropyl group.

The "hydroxyhalo C₁₋₆ alkyl group" in Formula (I) means the "halo C₁₋₆ alkyl group" in which any substitutable positions are substituted with 1 or 2 or more, preferably 1 or 2 hydroxy groups, and examples thereof include a 2,2,2-trifluoro-1-hydroxyethyl group and a 2-hydroxy-1,1-difluoroethyl group.

The "C₁₋₆ alkoxy group" in Formula (I) means a group in which a hydrogen atom of a hydroxy group is substituted with the "C₁₋₆ alkyl group", and examples thereof include a methoxy group, an ethoxy group, a propoxy group, an isopropoxy group, a butoxy group, a sec-butoxy group, a tert-butoxy group, a pentyloxy group, an isopentyloxy group, a hexyloxy group, and an isohexyloxy group.

The "halo C₁₋₆ alkoxy group" in Formula (I) means a group in which a hydrogen atom of a hydroxy group is substituted with the "halo C₁₋₆ alkyl group", and examples thereof include a fluoromethoxy group, a difluoromethoxy group, a trifluoromethoxy group, a 2-fluoroethoxy group, a 1,2-difluoroethoxy group, a 2,2,2-trifluoroethoxy group, a chloromethoxy group, a 2-chloroethoxy group, a 1,2-dichloroethoxy group, a bromomethoxy group, and an iodomethoxy group.

The "hydroxy C₁₋₆ alkoxy group" in Formula (I) means the "C₁₋₆ alkoxy group" in which any substitutable positions are substituted with 1 or 2 or more, preferably 1 or 2 hydroxy groups, and examples thereof include a 2-hydroxyethoxy group, a 2-hydroxypropoxy group, a 3-hydroxypropoxy group, and a 2-hydroxy-2-methylpropoxy group.

The " (C₁₋₆ alkoxy) C₁₋₆ alkyl group" in Formula (I) means the "C₁₋₆ alkyl group" in which any substitutable positions are substituted with 1 or 2 or more, preferably 1 or 2, same or different "C₁₋₆ alkoxy groups" above, and examples thereof include a methoxymethyl group, an ethoxymethyl group, a 1-methoxyethyl group, a 1-ethoxyethyl group, a 2-methoxyethyl group, a 2-ethoxyethyl group, a 1-methoxy-1-methylethyl group, a 1,2-dimethoxyethyl group, and a 3-methoxypropyl group.

The " (halo C₁₋₆ alkoxy) C₁₋₆ alkyl group" in Formula (I) means the "C₁₋₆ alkyl group" in which any substitutable positions are substituted with 1 or 2 or more, preferably 1 or 2, same or different "halo C₁₋₆ alkoxy groups" above, and examples thereof include a fluoromethoxymethyl group, a difluoromethoxymethyl group, a trifluoromethoxymethyl group, a 1-(fluoromethoxy)ethyl group, a 1-(difluoromethoxy)ethyl group, a 1-(trifluoromethoxy)ethyl group, a 2-(fluoromethoxy)ethyl group, a 2-(difluoromethoxy)ethyl group, a 2-(trifluoromethoxy)ethyl group, a chloromethoxymethyl group, and a bromomethoxymethyl group.

The "C₁₋₆ alkylthio group" in Formula (I) means a group in which a hydrogen atom of a sulfanyl group is substituted with the "C₁₋₆ alkyl group", and examples thereof include a methylthio group, an ethylthio group, a propylthio group, an isopropylthio group, a butylthio group, a sec-butylthio group, an isobutylthio group, a tert-butylthio group, a pentylthio group, an isopentylthio group, a hexylthio group, and an isohexylthio group.

The "mono C₁₋₆ alkylamino group" in Formula (I) means a group in which one hydrogen atom of an amino group is substituted with the "C₁₋₆ alkyl group", and examples thereof include a methylamino group, an ethylamino group, a propylamino group, an isopropylamino group, a butylamino group, a sec-butylamino group, and a tert-butylamino group.

The "di C₁₋₆ alkylamino group" in Formula (I) means a group in which two hydrogen atoms of an amino group are substituted with the same or different "C₁₋₆ alkyl group" above, and examples thereof include a dimethylamino group, a diethylamino group, a dipropylamino group, a diisopropylamino group, a dibutylamino group, a diisobutylamino group, a dipentylamino group, a dihexylamino group, an N-ethyl-N-methylamino group, an N-methyl-N-propylamino group, an N-butyl-N-methylamino group, an N-methyl-N-pentylamino group, and an N-hexyl-N-methylamino group.

The "mono (halo C₁₋₆ alkyl) amino group" in Formula (I) means a group in which one hydrogen atom of an amino group is substituted with the "halo C₁₋₆ alkyl group", and examples thereof include a 2-fluoroethylamino group, a 2,2-difluoroethylamino group, a 2,2,2-trifluoroethylamino group, and a 2,2,3,3,3-pentafluoropropylamino group.

The "C₃₋₆ cycloalkyl group" in Formula (I) means a monocyclic saturated hydrocarbon group having 3 to 6 carbon atoms, and examples thereof include a cyclopropyl group and a cyclobutyl group.

The "C₆₋₁₀ aryl group" in Formula (I) means an aromatic hydrocarbon group having 6 to 10 carbon atoms, and examples thereof include a phenyl group and a naphthyl group.

The "C₃₋₁₀ cycloalkyl group" in Formula (I) means an alicyclic hydrocarbon group having 3 to 10 carbon atoms.

The "C₃₋₁₀ cycloalkyl group" is any cyclic group selected from a monocyclic ring system, a condensed ring system, a bridged ring system, or a spiro ring system.

Examples of the monocyclic ring system include a cyclopropyl group, a cyclobutyl group, a cyclopentyl group, a cyclopentenyl group, a cyclohexyl group, a cyclohexenyl group, a cycloheptyl group, and a cyclooctyl group.

Examples of the condensed ring system include a bicyclo[3.3.0]octyl group, a bicyclo[4.3.0]nonyl group, and a bicyclo[4.4.0]decyl group (decahydronaphthalenyl group).

Examples of the bridged ring system include a bicyclo[2.2.1]heptyl group, a bicyclo[2.2.2]octyl group, and a bicyclo[3.2.1]octyl group.

Examples of the spiro ring system include a spiro[2.3]hexyl group, a spiro[3.3]heptyl group, a spiro[2.5]octyl group, and a spiro[3.4]octyl group.

The "C₃₋₁₀ cycloalkyl group" also includes a cyclic group in which an alicyclic hydrocarbon group is condensed with a benzene ring (the total number of carbon atoms contained in the ring of the cyclic group can be up to 10). Examples of the cyclic group include an indan-1-yl group, an indan-2-yl group, a 1,2,3,4-tetrahydronaphthalen-1-yl group, and a 1,2,3,4-tetrahydronaphthalen-2-yl group.

The "5- to 10-membered heteroaryl group" in Formula (I) means an aromatic heterocyclic group having 5 to 10 atoms that constitute the ring. The ring of the aromatic heterocyclic group contains at least one heteroatom selected from a nitrogen atom, an oxygen atom, or a sulfur atom (when there are two or more heteroatoms in the ring, the heteroatoms may be the same or different.).

The "5- to 10-membered heteroaryl group" is a cyclic group of either a monocyclic ring system or a condensed ring system.

Examples of the monocyclic ring system include a pyrrolyl group, a furyl group, a thienyl group, an imidazolyl group, a pyrazolyl group, a thiazolyl group, an isothiazolyl group, an oxazolyl group, an isoxazolyl group, a triazolyl group, a tetrazolyl group, a 1,2,3-oxadiazolyl group, a 1,2,4-oxadiazolyl group, a 1,3,4-oxadiazolyl group, a 1,2,5-oxadiazolyl group, a 1,2,3-thiadiazolyl group, a 1,2,4-thiadiazolyl group, a 1,3,4-thiadiazolyl group, a 1,2,5-thiadiazolyl group, a pyridyl group, a pyrazinyl group, a pyrimidinyl group, a pyridazinyl group, a 1,2,4-triazinyl groups, and a 1,3,5-triazinyl group.

Examples of the condensed ring system include an indolyl group, an isoindolyl group, a benzofuranyl group, a benzothiophenyl group, a benzimidazolyl group, a benzoxazolyl group, a benzisoxazolyl group, a benzothiazolyl group, a benzisothiazolyl group, an indazolyl group, an imidazopyridyl group, a purinyl group, a quinolyl group, a quinolizinyl group, an isoquinolyl group, a phthalazinyl group, a naphthyridinyl group, a quinoxalinyl group, a quinazolinyl group, a cinnolinyl group, a pteridinyl group, and a pyrido[3,2-b]pyridyl group.

The "4- to 10-membered heterocyclyl group" in Formula (I) means a non-aromatic heterocyclic group having 4 to 10 atoms that constitute the ring. The ring of the non-aromatic heterocyclic group contains at least one heteroatom selected from a nitrogen atom, an oxygen atom, or a sulfur atom (when there are two or more heteroatoms in the ring, the heteroatoms may be the same or different.).

The "4- to 10-membered heterocyclyl group" is any cyclic group selected from a monocyclic ring system, a condensed ring system, a bridged ring system, or a spiro ring system.

Examples of the monocyclic ring system include an azetidinyl group, a pyrrolidinyl group, a piperidinyl group, an azepanyl group, an oxetanyl group, an oxolanyl group, an oxanyl group, a thianyl group (tetrahydrothiopyranyl group), a pyrazolidinyl group, an imidazolidinyl group, an oxazolidinyl group, an isoxazolidinyl group, a thiazolidinyl group, an isothiazolidinyl group, a dioxolanyl group, a dioxanyl group, a morpholinyl group, a piperazinyl group, a dithianyl group, and a thiomorpholinyl group.

Examples of the condensed ring system include a 3-azabicyclo[3.3.0]octyl group, a 7-azabicyclo[4.3.0]nonyl group (octahydroindolyl group), and an 8-azabicyclo[4.3.0]nonyl group (octahydroisoindolyl group).

Examples of the bridged ring system include a 2-azabicyclo[2.2.1]heptyl group, a 1-azabicyclo[2.2.2]octyl group, and a 2-azabicyclo[2.2.2]octyl group.

Examples of the spiro ring system include a 2-azaspiro[3.3]heptyl group, a 2-azaspiro[3.4]octyl group, and a 2-oxaspiro[3.5]nonyl group.

The "4- to 10-membered heterocyclyl group" also includes a cyclic group in which an alicyclic hydrocarbon group is condensed with a pyridine ring (the total number of atoms contained in the ring of the cyclic group can be up to 10). Examples of the cyclic group include a 2,3-cyclopentenopyridyl group, a 3,4-cyclopentenopyridyl group, a 5,6,7,8-tetrahydroquinolyl group, and a 5,6,7,8-tetrahydroisoquinolyl group.

Further, the "4- to 10-membered heterocyclyl group" also includes a cyclic group in which a non-aromatic heterocyclic group is condensed with a benzene ring (the total number of atoms contained in the ring of the cyclic group can be up to 10). Examples of the cyclic group include a 2,3-dihydro-1H-indolyl group (indolinyl group), a 2,3-dihydro-1H-isoindolyl group (isoindolinyl group), a 1,2,3,4-tetrahydroquinolyl group, a 1,2,3,4-tetrahydroisoquinolyl group, a 2,3-dihydrobenzofuranyl group, a 3,4-dihydro-2H-1-benzopyranyl group (chromanyl group), a 3,4-dihydro-1H-2-benzopyranyl group (isochromanyl group), and a 2,3-dihydrobenzothiophenyl group.

The "C₂₋₇ alkanoyl group" in Formula (I) means a group in which the "C₁₋₆ alkyl group" and a carbonyl group are bonded, that is, an alkanoyl group having 2 to 7 carbon atoms, and examples thereof include an acetyl group, a propanoyl group, a butanoyl group, a 2-methylpropanoyl group, a pentanoyl group, a 3-methylbutanoyl group, and a 2,2-dimethylpropanoyl group.

The "halo C₂₋₇ alkanoyl group" in Formula (I) means the "C₂₋₇ alkanoyl group" in which any substitutable positions are substituted with 1 or 2 or more, preferably 1 to 5, same or different "halogen atoms" above, and examples thereof include a 3,3,3-trifluoropropanoyl group.

The "hydroxy C₂₋₇ alkanoyl group" in Formula (I) means the "C₂₋₇ alkanoyl group" in which any substitutable positions are substituted with 1 or 2 or more, preferably 1 or 2 hydroxy groups, and examples thereof include a 2-hydroxy-2-methylpropanoyl group.

The "C₂₋₇ alkoxycarbonyl group" in Formula (I) means a group in which the "C₁₋₆ alkoxy group" and a carbonyl group are bonded, that is, an alkoxycarbonyl group having 2 to 7 carbon atoms, and examples thereof include a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, and a pentyloxycarbonyl group.

The "mono C₁₋₆ alkylcarbamoyl group" in Formula (I) means a group in which one hydrogen atom of a carbamoyl group is substituted with the "C₁₋₆ alkyl group", and examples thereof include a methylcarbamoyl group, an ethylcarbamoyl group, a propylcarbamoyl group, an isopropylcarbamoyl group, a butylcarbamoyl group, a sec-butylcarbamoyl group, and a tert-butylcarbamoyl group.

The "di C₁₋₆ alkylcarbamoyl group" in Formula (I) means a group in which two hydrogen atoms of a carbamoyl group are substituted with the same or different "C₁₋₆ alkyl group" above, and examples thereof include a dimethylcarbamoyl group, a diethylcarbamoyl group, a dipropylcarbamoyl group, a diisopropylcarbamoyl group, and an N-ethyl-N-methylcarbamoyl group.

The "C₁₋₆ alkylsulfonyl group" in Formula (I) means a group in which the "C₁₋₆ alkyl group" and a sulfonyl group are bonded, and examples thereof include a methylsulfonyl group, an ethylsulfonyl group, a propylsulfonyl group, an isopropylsulfonyl group, a butylsulfonyl group, a sec-butylsulfonyl group, an isobutylsulfonyl group, a tert-butylsulfonyl group, a pentylsulfonyl group, an isopentylsulfonyl group, a hexylsulfonyl group, and an isohexylsulfonyl group.

The "C₁₋₆ alkylsulfonyloxy group" in Formula (I) means a group in which a hydrogen atom of a hydroxy group is substituted with the "C₁₋₆ alkylsulfonyl group", and examples thereof include a methylsulfonyloxy group, an ethylsulfonyloxy group, a propylsulfonyloxy group, and an isopropylsulfonyloxy group.

The "halo C₁₋₆ alkylsulfonyloxy group" in Formula (I) means the "C₁₋₆ alkylsulfonyloxy group" in which any substitutable positions are substituted with 1 or 2 or more, preferably 1 to 3, same or different "halogen atoms" above, and examples thereof include a fluoromethylsulfonyloxy group, a difluoromethylsulfonyloxy group, a trifluoromethylsulfonyloxy group, and a 2,2,2-trifluoroethylsulfonyloxy group.

The "mono C₁₋₆ alkylsulfamoyl group" in Formula (I) means a group in which one hydrogen atom of a sulfamoyl group is substituted with the "C₁₋₆ alkyl group", and examples thereof include a methylsulfamoyl group, an ethylsulfamoyl group, a propylsulfamoyl group, an isopropylsulfamoyl group, a butylsulfamoyl group, a sec-butylsulfamoyl group, and a tert-butylsulfamoyl group.

The "di C₁₋₆ alkylsulfamoyl group" in Formula (I) means a group in which two hydrogen atoms of a sulfamoyl group are substituted with the same or different "C₁₋₆ alkyl group" above, and examples thereof include a dimethylsulfamoyl group, a diethylsulfamoyl group, a dipropylsulfamoyl group, a diisopropylsulfamoyl group, and an N-ethyl-N-methylsulfamoyl group.

The "mono C₂₋₇ alkanoylamino group" in Formula (I) means a group in which one hydrogen atom of an amino group is substituted with the "C₂₋₇ alkanoyl group", and examples thereof include an acetylamino group, a propanoylamino group, a butanoylamino group, a 2-methylpropanoylamino group, a pentanoylamino group, a 3-methylbutanoylamino group, and a 2,2-dimethylpropanoylamino group.

The " (C₂₋₇ alkanoyl) C₁₋₆ alkylamino group" in Formula (I) means a group in which a hydrogen atom bonded to a nitrogen atom of the "mono C₁₋₆ alkylamino group" is substituted with the "C₂₋₇ alkanoyl group", and examples thereof include an N-acetyl-N-methylamino group, an N-methyl-N-propanoylamino group, an N-butanoyl-N-methylamino group, an N-methyl-N-pentanoylamino group, an N-acetyl-N-ethylamino group, and an N-acetyl-N-propylamino group.

The "di C₂₋₇ alkanoylamino group" in Formula (I) means a group in which two hydrogen atoms of an amino group are substituted with the same or different "C₂₋₇ alkanoyl group" above, and examples thereof include a diacetylamino group, a dipropanoylamino group, and an N-acetyl-N-propanoylamino group.

The "mono C₁₋₆ alkylsulfonylamino group" in Formula (I) means a group in which one hydrogen atom of an amino group is substituted with the "C₁₋₆ alkylsulfonyl group", and examples thereof include a methylsulfonylamino group, an ethylsulfonylamino group, a propylsulfonylamino group, an isopropylsulfonylamino group, a butylsulfonylamino group, a sec-butylsulfonylamino group, and a tert-butylsulfonylamino group.

The "mono C₂₋₇ alkoxycarbonylamino group" in Formula (I) means a group in which one hydrogen atom of an amino group is substituted with the "C₂₋₇ alkoxycarbonyl group", and examples thereof include a methoxycarbonylamino group, an ethoxycarbonylamino group, a propoxycarbonylamino group, an isopropoxycarbonylamino group, a butoxycarbonylamino group, an isobutoxycarbonylamino group, a tert-butoxycarbonylamino group, and a pentyloxycarbonylamino group.

The "oxo group" in Formula (I) means an oxygen atom bonded via a double bond (= O). Therefore, when an oxo group is bonded to a carbon atom, the oxo group forms a carbonyl group together with the carbon atom, when one oxo group is bonded to a sulfur atom, the oxo group forms a sulfinyl group together with the sulfur atom, and when two oxo groups are bonded to a sulfur atom, the oxo groups form a sulfonyl group together with the sulfur atom. Examples of the cyclic group in which an oxo group is bonded to a carbon atom that constitutes a ring include a 2-oxopyrrolidin-3-yl group, a 2-oxopiperidin-4-yl group, a 6-oxo-1,6-dihydropyridin-2-yl group, a 1-methyl-6-oxo-1,6-dihydropyridin-2-yl group, a 6-oxo-1,6-dihydropyrimidin-2-yl group, and a 1-methyl-6-oxo-1,6-dihydropyrimidin-2-yl group. Examples of the cyclic group in which an oxo group is bonded to a sulfur atom that constitutes a ring include a 1,1-dioxothian-4-yl group (1,1-dioxotetrahydrothiopyran-4-yl group), a 1,1-dioxoisothiazolidin-5-yl group, and a 1,1-dioxothiomorpholin-4-yl group.

The "C₁₋₆ alkanediyl group" in Formula (I) means a divalent group obtained by removing one hydrogen atom from the "C₁₋₆ alkyl group", and examples thereof include a methylene group, an ethane-1,1-diyl group, an ethane-1,2-diyl group, a propane-1,1-diyl group, a propane-1,2-diyl group, a propane-2,2-diyl group, and a propane-1,3-diyl group.

The "hydroxy C₁₋₆ alkanediyl group" in Formula (I) means the "C₁₋₆ alkanediyl group" in which any substitutable positions are substituted with 1 or 2 or more, preferably 1 or 2 hydroxy groups, and examples thereof include a hydroxymethylene group, a 1-hydroxyethane-1,1-diyl group, a 2-hydroxyethane-1,1-diyl group, a 1-hydroxyethane-1,2-diyl group, a 2-hydroxyethane-1,2-diyl group, a 1,2-dihydroxyethane-1,1-diyl group, and a 1,2-dihydroxyethane-1,2-diyl group.

The "cyclic ether group" in Formula (I) means a group in which carbon atoms that constitute the ring of the "C₃₋₆ cycloalkyl group" are substituted with 1 or 2 oxygen atoms, and examples thereof include an oxiranyl group, an oxetanyl group, an oxolanyl group, a dioxolanyl group, an oxanyl group, and a dioxanyl group.

The "cyclic amino group" in Formula (I) means a group in which carbon atoms that constitute the ring of the "C₃₋₆ cycloalkyl group" are substituted with 1 or 2 nitrogen atoms and a bonding hand is present on the nitrogen atom, and examples thereof include an azetidin-1-yl group, a pyrrolidin-1-yl group, a piperidin-1-yl group, and a piperazin-1-yl group.

The "halo cyclic amino group" in Formula (I) means the "cyclic amino group" in which any substitutable positions are substituted with 1 or 2 or more, preferably 1 to 5, same or different "halogen atoms" above, and examples thereof include a 3-fluoroazetidin-1-yl group, a 3,3-difluoroazetidin-1-yl group, a 3,3-difluoropyrrolidin-1-yl group, a 3,3,4,4-tetrafluoropyrrolidin-1-yl group, and a 3,3-difluoropiperidin-1-yl group.

The "any substitutable position" means a site of a substitutable hydrogen atom on a carbon atom, a nitrogen atom, an oxygen atom, and/or a sulfur atom, the substitution of the hydrogen atom being chemically acceptable, and the substitution resulting in a stable compound.

Each group that constitutes Formula (I) will be described in detail.

"-X=" in Formula (I) represents "-CH=" or "-N=".

"=Y-" in Formula (I) represents "=CH-" or "=N-".

When "-X=" is "-CH=", "=Y-" is preferably "=CH-".

When "-X=" is "-N=", "=Y-" is preferably "=CH-".

R¹ in Formula (I) represents a hydrogen atom, a halogen atom, a cyano group, a hydroxy group, a sulfanyl group, an amino group, a carbamoyl group, a sulfamoyl group, a carboxy group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, or a C₃₋₆ cycloalkyl group, and is preferably a hydrogen atom, a halogen atom, a cyano group, a C₁₋₆ alkyl group, or a halo C₁₋₆ alkyl group.

The halogen atom of R¹ is preferably a fluorine atom or a chlorine atom.

The C₁₋₆ alkyl group of R¹ is preferably a methyl group or an ethyl group.

The C₂₋₆ alkenyl group of R¹ is preferably a vinyl group or an allyl group.

The halo C₁₋₆ alkyl group of R¹ is preferably a difluoromethyl group or a trifluoromethyl group.

The hydroxy C₁₋₆ alkyl group of R¹ is preferably a hydroxymethyl group or a 1-hydroxyethyl group.

The hydroxyhalo C₁₋₆ alkyl group of R¹ is preferably a 2,2,2-trifluoro-1-hydroxyethyl group or a 2-hydroxy-1,1-difluoroethyl group.

The C₁₋₆ alkoxy group of R¹ is preferably a methoxy group or an ethoxy group.

The halo C₁₋₆ alkoxy group of R¹ is preferably a difluoromethoxy group or a trifluoromethoxy group.

The hydroxy C₁₋₆ alkoxy group of R¹ is preferably a 2-hydroxyethoxy group or a 2-hydroxy-2-methylpropoxy group.

The (C₁₋₆ alkoxy) C₁₋₆ alkyl group of R¹ is preferably a methoxymethyl group or an ethoxymethyl group.

The (halo C₁₋₆ alkoxy) C₁₋₆ alkyl group of R¹ is preferably a difluoromethoxymethyl group or a trifluoromethoxymethyl group.

The C₁₋₆ alkylthio group of R¹ is preferably a methylthio group or an ethylthio group.

The mono C₁₋₆ alkylamino group of R¹ is preferably a methylamino group or an ethylamino group.

The di C₁₋₆ alkylamino group of R¹ is preferably a dimethylamino group or a diethylamino group.

The mono (halo C₁₋₆ alkyl) amino group of R¹ is preferably a 2,2-difluoroethylamino group or a 2,2,2-trifluoroethylamino group.

The C₃₋₆ cycloalkyl group of R¹ is preferably a cyclopropyl group or a cyclobutyl group.

R² in Formula (I) represents a hydrogen atom, a halogen atom, a cyano group, a hydroxy group, a sulfanyl group, an amino group, a carbamoyl group, a sulfamoyl group, a carboxy group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, or a C₃₋₆ cycloalkyl group, and is preferably a hydrogen atom or a C₁₋₆ alkyl group.

The halogen atom of R² is preferably a fluorine atom or a chlorine atom.

The C₁₋₆ alkyl group of R² is preferably a methyl group or an ethyl group.

The C₂₋₆ alkenyl group of R² is preferably a vinyl group or an allyl group.

The halo C₁₋₆ alkyl group of R² is preferably a difluoromethyl group or a trifluoromethyl group.

The hydroxy C₁₋₆ alkyl group of R² is preferably a hydroxymethyl group or a 1-hydroxyethyl group.

The hydroxyhalo C₁₋₆ alkyl group of R² is preferably a 2,2,2-trifluoro-1-hydroxyethyl group or a 2-hydroxy-1,1-difluoroethyl group.

The C₁₋₆ alkoxy group of R² is preferably a methoxy group or an ethoxy group.

The halo C₁₋₆ alkoxy group of R² is preferably a difluoromethoxy group or a trifluoromethoxy group.

The hydroxy C₁₋₆ alkoxy group of R² is preferably a 2-hydroxyethoxy group or a 2-hydroxy-2-methylpropoxy group.

The (C₁₋₆ alkoxy) C₁₋₆ alkyl group of R² is preferably a methoxymethyl group or an ethoxymethyl group.

The (halo C₁₋₆ alkoxy) C₁₋₆ alkyl group of R² is preferably a difluoromethoxymethyl group or a trifluoromethoxymethyl group.

The C₁₋₆ alkylthio group of R² is preferably a methylthio group or an ethylthio group.

The mono C₁₋₆ alkylamino group of R² is preferably a methylamino group or an ethylamino group.

The di C₁₋₆ alkylamino group of R² is preferably a dimethylamino group or a diethylamino group.

The mono (halo C₁₋₆ alkyl) amino group of R² is preferably a 2,2-difluoroethylamino group or a 2,2,2-trifluoroethylamino group.

The C₃₋₆ cycloalkyl group of R² is preferably a cyclopropyl group or a cyclobutyl group.

In one aspect of the present invention, R¹ is preferably a hydrogen atom, a halogen atom, a cyano group, a C₁₋₆ alkyl group, or a halo C₁₋₆ alkyl group, and R² is preferably a hydrogen atom.

R³ in Formula (I) represents a hydrogen atom or a halogen atom.

The halogen atom of R³ is preferably a fluorine atom or a chlorine atom.

Z in Formula (I) is a group represented by Formula (II) or Formula (III) below, and preferably a group represented by Formula (II).

The wavy line in Formula (II) represents the point of attachment to the nitrogen atom.

Ring Z¹ in Formula (II) represents a C₆₋₁₀ aryl group, a C₃₋₁₀ cycloalkyl group, a 5- to 10-membered heteroaryl group, or a 4- to 10-membered heterocyclyl group, and is preferably a C₃₋₁₀ cycloalkyl group.

The C₆₋₁₀ aryl group of Z¹ is preferably a phenyl group or a naphthyl group.

The C₃₋₁₀ cycloalkyl group of Z¹ is preferably a cyclohexyl group, a bicyclo[2.2.1]heptyl group, or a bicyclo[2.2.2]octyl group.

The 5- to 10-membered heteroaryl group of Z¹ is preferably a thienyl group or a pyridyl group.

The 4- to 10-membered heterocyclyl group of Z¹ is preferably a pyrrolidinyl group or a piperidinyl group.

In one aspect of the present invention, Z¹ is preferably a group represented by the formula below: 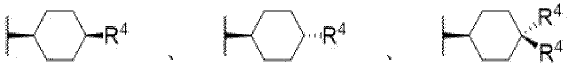 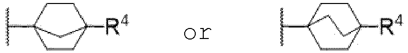 (wherein the wavy line represents the point of attachment to the nitrogen atom and R⁴ is as defined above).

m in Formula (II) represents 0, 1, or 2, and is preferably 1 or 2.

R⁴ in Formula (II) represents (when there are multiple R⁴'s in Formula (II), each R⁴ independently represents) a halogen atom, a cyano group, a hydroxy group, a sulfanyl group, an amino group, a carbamoyl group, a sulfamoyl group, a carboxy group, a formyl group, an azide group, a hydrazinyl group, a nitro group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, a C₂₋₇ alkanoyl group, a halo C₂₋₇ alkanoyl group, a hydroxy C₂₋₇ alkanoyl group, a C₂₋₇ alkoxycarbonyl group, a mono C₁₋₆ alkylcarbamoyl group, a di C₁₋₆ alkylcarbamoyl group, a C₁₋₆ alkylsulfonyl group, a C₁₋₆ alkylsulfonyloxy group, a halo C₁₋₆ alkylsulfonyloxy group, a mono C₁₋₆ alkylsulfamoyl group, a di C₁₋₆ alkylsulfamoyl group, a mono C₂₋₇ alkanoylamino group, a (C₂₋₇ alkanoyl) C₁₋₆ alkylamino group, a di C₂₋₇ alkanoylamino group, a mono C₁₋₆ alkylsulfonylamino group, a mono C₂₋₇ alkoxycarbonylamino group, or an oxo group, and is preferably a cyano group, a hydroxy group, a carbamoyl group, a C₁₋₆ alkyl group, a hydroxy C₁₋₆ alkyl group, or a hydroxy C₁₋₆ alkoxy group.

The halogen atom of R⁴ is preferably a fluorine atom or a chlorine atom.

The C₁₋₆ alkyl group of R⁴ is preferably a methyl group or an ethyl group.

The C₂₋₆ alkenyl group of R⁴ is preferably a vinyl group or an allyl group.

The halo C₁₋₆ alkyl group of R⁴ is preferably a difluoromethyl group or a trifluoromethyl group.

The hydroxy C₁₋₆ alkyl group of R⁴ is preferably a hydroxymethyl group or a 2-hydroxypropan-2-yl group.

The hydroxyhalo C₁₋₆ alkyl group of R⁴ is preferably a 2,2,2-trifluoro-1-hydroxyethyl group or a 2-hydroxy-1,1-difluoroethyl group.

The C₁₋₆ alkoxy group of R⁴ is preferably a methoxy group or an ethoxy group.

The halo C₁₋₆ alkoxy group of R⁴ is preferably a difluoromethoxy group or a trifluoromethoxy group.

The hydroxy C₁₋₆ alkoxy group of R⁴ is preferably a 2-hydroxyethoxy group or a 2-hydroxy-2-methylpropoxy group.

The (C₁₋₆ alkoxy) C₁₋₆ alkyl group of R⁴ is preferably a methoxymethyl group or an ethoxymethyl group.

The (halo C₁₋₆ alkoxy) C₁₋₆ alkyl group of R⁴ is preferably a difluoromethoxymethyl group or a trifluoromethoxymethyl group.

The C₁₋₆ alkylthio group of R⁴ is preferably a methylthio group or an ethylthio group.

The mono C₁₋₆ alkylamino group of R⁴ is preferably a methylamino group or an ethylamino group.

The di C₁₋₆ alkylamino group of R⁴ is preferably a dimethylamino group or a diethylamino group.

The mono (halo C₁₋₆ alkyl) amino group of R⁴ is preferably a 2,2-difluoroethylamino group or a 2,2,2-trifluoroethylamino group.

The C₂₋₇ alkanoyl group of R⁴ is preferably an acetyl group or a propanoyl group.

The halo C₂₋₇ alkanoyl group of R⁴ is preferably a 3,3,3-trifluoropropanoyl group.

The hydroxy C₂₋₇ alkanoyl group of R⁴ is preferably a 2-hydroxy-2-methylpropanoyl group.

The C₂₋₇ alkoxycarbonyl group of R⁴ is preferably a methoxycarbonyl group or an ethoxycarbonyl group.

The mono C₁₋₆ alkylcarbamoyl group of R⁴ is preferably a methylcarbamoyl group or an ethylcarbamoyl group.

The di C₁₋₆ alkylcarbamoyl group of R⁴ is preferably a dimethylcarbamoyl group or a diethylcarbamoyl group.

The C₁₋₆ alkylsulfonyl group of R⁴ is preferably a methylsulfonyl group or an ethylsulfonyl group.

The C₁₋₆ alkylsulfonyloxy group of R⁴ is preferably a methylsulfonyloxy group or an ethylsulfonyloxy group.

The halo C₁₋₆ alkylsulfonyloxy group of R⁴ is preferably a trifluoromethylsulfonyloxy group or a 2,2,2-trifluoroethylsulfonyloxy group.

The mono C₁₋₆ alkylsulfamoyl group of R⁴ is preferably a methylsulfamoyl group or an ethylsulfamoyl group.

The di C₁₋₆ alkylsulfamoyl group of R⁴ is preferably a dimethylsulfamoyl group or a diethylsulfamoyl group.

The mono C₂₋₇ alkanoylamino group of R⁴ is preferably an acetylamino group or a propanoylamino group.

The (C₂₋₇ alkanoyl) C₁₋₆ alkylamino group of R⁴ is preferably an N-acetyl-N-methylamino group or an N-methyl-N-propanoylamino group.

The di C₂₋₇ alkanoylamino group of R⁴ is preferably a diacetylamino group or a dipropanoylamino group.

The mono C₁₋₆ alkylsulfonylamino group of R⁴ is preferably a methylsulfonylamino group or an ethylsulfonylamino group.

The mono C₂₋₇ alkoxycarbonylamino group of R⁴ is preferably a methoxycarbonylamino group or a tert-butoxycarbonylamino group.

The wavy line in Formula (III) represents the point of attachment to the nitrogen atom.

Ring Z² in Formula (III) represents a C₆₋₁₀ aryl group, a C₃₋₁₀ cycloalkyl group, a 5- to 10-membered heteroaryl group, or a 4- to 10-membered heterocyclyl group, and is preferably a 5- to 10-membered heteroaryl group or a 4- to 10-membered heterocyclyl group.

The C₆₋₁₀ aryl group of Z² is preferably a phenyl group or a naphthyl group.

The C₃₋₁₀ cycloalkyl group of Z² is preferably a cyclohexyl group, a bicyclo[2.2.1]heptyl group, or a bicyclo[2.2.2]octyl group.

The 5- to 10-membered heteroaryl group of Z² is preferably a thienyl group or a pyridyl group.

The 4- to 10-membered heterocyclyl group of Z² is preferably a pyrrolidinyl group or a piperidinyl group.

L in Formula (III) represents a single bond, a C₁₋₆ alkanediyl group, a hydroxy C₁₋₆ alkanediyl group, a carbonyl group, or a sulfonyl group, and is preferably a single bond or a carbonyl group.

The C₁₋₆ alkanediyl group of L is preferably a methylene group or an ethane-1,1-diyl group.

The hydroxy C₁₋₆ alkanediyl group of L is preferably a hydroxymethylene group or a 1-hydroxyethane-1,1-diyl group.

Ring Z³ in Formula (III) represents a C₆₋₁₀ aryl group, a C₃₋₁₀ cycloalkyl group, a 5- to 10-membered heteroaryl group, or a 4- to 10-membered heterocyclyl group, and is preferably a C₃₋₁₀ cycloalkyl group, a 5- to 10-membered heteroaryl group, or a 4- to 10-membered heterocyclyl group.

The C₆₋₁₀ aryl group of Z³ is preferably a phenyl group or a naphthyl group.

The C₃₋₁₀ cycloalkyl group of Z³ is preferably a cyclopropyl group or a cyclobutyl group.

The 5- to 10-membered heteroaryl group of Z³ is preferably a pyridyl group or a 1,3,4-thiadiazolyl group.

The 4- to 10-membered heterocyclyl group of Z³ is preferably an oxetanyl group or a morpholinyl group.

n in Formula (III) represents 0, 1, or 2, and is preferably 0 or 1.

R⁵ in Formula (III) represents (when there are multiple R⁵'s in Formula (III), each R⁵ independently represents) a halogen atom, a cyano group, a hydroxy group, a sulfanyl group, an amino group, a carbamoyl group, a sulfamoyl group, a carboxy group, a formyl group, an azide group, a hydrazinyl group, a nitro group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, a C₂₋₇ alkanoyl group, a halo C₂₋₇ alkanoyl group, a hydroxy C₂₋₇ alkanoyl group, a C₂₋₇ alkoxycarbonyl group, a mono C₁₋₆ alkylcarbamoyl group, a di C₁₋₆ alkylcarbamoyl group, a C₁₋₆ alkylsulfonyl group, a C₁₋₆ alkylsulfonyloxy group, a halo C₁₋₆ alkylsulfonyloxy group, a mono C₁₋₆ alkylsulfamoyl group, a di C₁₋₆ alkylsulfamoyl group, a mono C₂₋₇ alkanoylamino group, a (C₂₋₇ alkanoyl) C₁₋₆ alkylamino group, a di C₂₋₇ alkanoylamino group, a mono C₁₋₆ alkylsulfonylamino group, a mono C₂₋₇ alkoxycarbonylamino group, an oxo group, a C₃₋₆ cycloalkyl group, a cyclic ether group, a cyclic amino group, or a halo cyclic amino group, and is preferably a halogen atom or a C₁₋₆ alkyl group.

The halogen atom of R⁵ is preferably a fluorine atom or a chlorine atom.

The C₁₋₆ alkyl group of R⁵ is preferably a methyl group or an ethyl group.

The C₂₋₆ alkenyl group of R⁵ is preferably a vinyl group or an allyl group.

The halo C₁₋₆ alkyl group of R⁵ is preferably a difluoromethyl group or a trifluoromethyl group.

The hydroxy C₁₋₆ alkyl group of R⁵ is preferably a hydroxymethyl group or a 2-hydroxypropan-2-yl group.

The hydroxyhalo C₁₋₆ alkyl group of R⁵ is preferably a 2,2,2-trifluoro-1-hydroxyethyl group or a 2-hydroxy-1,1-difluoroethyl group.

The C₁₋₆ alkoxy group of R⁵ is preferably a methoxy group or an ethoxy group.

The halo C₁₋₆ alkoxy group of R⁵ is preferably a difluoromethoxy group or a trifluoromethoxy group.

The hydroxy C₁₋₆ alkoxy group of R⁵ is preferably a 2-hydroxyethoxy group or a 2-hydroxy-2-methylpropoxy group.

The (C₁₋₆ alkoxy) C₁₋₆ alkyl group of R⁵ is preferably a methoxymethyl group or an ethoxymethyl group.

The (halo C₁₋₆ alkoxy) C₁₋₆ alkyl group of R⁵ is preferably a difluoromethoxymethyl group or a trifluoromethoxymethyl group.

The C₁₋₆ alkylthio group of R⁵ is preferably a methylthio group or an ethylthio group.

The mono C₁₋₆ alkylamino group of R⁵ is preferably a methylamino group or an ethylamino group.

The di C₁₋₆ alkylamino group of R⁵ is preferably a dimethylamino group or a diethylamino group.

The mono (halo C₁₋₆ alkyl) amino group of R⁵ is preferably a 2,2-difluoroethylamino group or a 2,2,2-trifluoroethylamino group.

The C₂₋₇ alkanoyl group of R⁵ is preferably an acetyl group or a propanoyl group.

The halo C₂₋₇ alkanoyl group of R⁵ is preferably a 3,3,3-trifluoropropanoyl group.

The hydroxy C₂₋₇ alkanoyl group of R⁵ is preferably a 2-hydroxy-2-methylpropanoyl group.

The C₂₋₇ alkoxycarbonyl group of R⁵ is preferably a methoxycarbonyl group or an ethoxycarbonyl group.

The mono C₁₋₆ alkylcarbamoyl group of R⁵ is preferably a methylcarbamoyl group or an ethylcarbamoyl group.

The di C₁₋₆ alkylcarbamoyl group of R⁵ is preferably a dimethylcarbamoyl group or a diethylcarbamoyl group. The C₁₋₆ alkylsulfonyl group of R⁵ is preferably a methylsulfonyl group or an ethylsulfonyl group.

The C₁₋₆ alkylsulfonyloxy group of R⁵ is preferably a methylsulfonyloxy group or an ethylsulfonyloxy group.

The halo C₁₋₆ alkylsulfonyloxy group of R⁵ is preferably a trifluoromethylsulfonyloxy group or a 2,2,2-trifluoroethylsulfonyloxy group.

The mono C₁₋₆ alkylsulfamoyl group of R⁵ is preferably a methylsulfamoyl group or an ethylsulfamoyl group.

The di C₁₋₆ alkylsulfamoyl group of R⁵ is preferably a dimethylsulfamoyl group or a diethylsulfamoyl group.

The mono C₂₋₇ alkanoylamino group of R⁵ is preferably an acetylamino group or a propanoylamino group.

The (C₂₋₇ alkanoyl) C₁₋₆ alkylamino group of R⁵ is preferably an N-acetyl-N-methylamino group or an N-methyl-N-propanoylamino group.

The di C₂₋₇ alkanoylamino group of R⁵ is preferably a diacetylamino group or a dipropanoylamino group.

The mono C₁₋₆ alkylsulfonylamino group of R⁵ is preferably a methylsulfonylamino group or an ethylsulfonylamino group.

The mono C₂₋₇ alkoxycarbonylamino group of R⁵ is preferably a methoxycarbonylamino group or a tert-butoxycarbonylamino group.

The C₃₋₆ cycloalkyl group of R⁵ is preferably a cyclopropyl group or a cyclobutyl group.

The cyclic ether group of R⁵ is preferably an oxetanyl group or an oxolanyl group.

The cyclic amino group of R⁵ is preferably an azetidin-1-yl group or a pyrrolidin-1-yl group.

The halo cyclic amino group of R⁵ is preferably a 3,3-difluoroazetidin-1-yl group or a 3,3-difluoropyrrolidin-1-yl group.

Specific examples of the compound of the present invention include those described in Examples, and among them, the following compounds (1) to (49) are preferable.

**[Table 1-1]**

| | |
|---|---|
| (1) | N-(4-Hydroxybicyclo[2.2.2]octan-1-yl)-4-(7-methyl-1H-pyrrolo[3,2-c]pyridin-4-yl)benzamide |
| (2) | N-(trans-4-Hydroxy-4-methylcyclohexyl)-4-(7-methyl-1H-pyrrolo[3,2-c]pyridin-4-yl)benzamide |
| (3) | N-[trans-4-(1-Hydroxycyclopropyl)cyclohexyl]-4-(7-methyl-1H-pyrrolo[3,2-c]pyridin-4-yl)benzamide |
| (4) | N-[trans-4-(2-Hydroxy-2-methylpropoxy)cyclohexyl]-4-(7-methyl-1H-pyrrolo[3,2-c]pyridin-4-yl)benzamide |
| (5) | 3-Fluoro-N-(trans-4-hydroxy-4-methylcyclohexyl)-4-(1H-pyrrolo[3,2-c]pyridin-4-yl)benzamide |
| (6) | 3-Fluoro-N-(4-hydroxybicyclo[2.2.2]octan-1-yl)-4-(1H-pyrrolo[3,2-c]pyridin-4-yl)benzamide |
| (7) | N-(4-Cyanobicyclo[2.2.2]octan-1-yl)-3-fluoro-4-(1H-pyrrolo[3,2-c]pyridin-4-yl)benzamide |
| (8) | 4-[3-Fluoro-4-(1H-pyrrolo[3,2-c]pyridin-4-yl)benzamide]bicycle[2.2.2]octane-1-carboxamide |
| (9) | 3-Fluoro-N-[trans-4-(2-hydroxy-2-methylpropoxy)cyclohexyl]-4-(1H-pyrrolo[3,2-c]pyridin-4-yl)benzamide |
| (10) | 3-Fluoro-N-(4-hydroxybicyclo[2.2.1]heptan-1-yl)-4-(1H-pyrrolo[3,2-c]pyridin-4-yl)benzamide |
| (11) | 3-Fluoro-N-[trans-4-(1-hydroxycyclopropyl)cyclohexyl]-4-(1H-pyrrolo[3,2-c]pyridin-4-yl)benzamide |
| (12) | 3-Fluoro-N-(trans-4-hydroxycyclohexyl)-4-(1H-pyrrolo[3,2-c]pyridin-4-yl)benzamide |
| (13) | 4-(7-Fluoro-1H-pyrrolo[3,2-c]pyridin-4-yl)-N-[trans-4-(2-hydroxypropan-2-yl)cyclohexyl]benzamide |
| (14) | 4-(7-Chloro-1H-pyrrolo[3,2-c]pyridin-4-yl)-N-[trans-4-(2-hydroxypropan-2-yl)cyclohexyl]benzamide |
| (15) | 4-(7-Cyano-1H-pyrrolo[3,2-c]pyridin-4-yl)-N-[trans-4-(2-hydroxypropan-2-yl)cyclohexyl]benzamide |
| (16) | 4-(7-Fluoro-1H-pyrrolo[3,2-c]pyridin-4-yl)-N-(4-hydroxybicyclo[2.2.2]octan-1-yl)benzamide |
| (17) | 4-(7-Chloro-1H-pyrrolo[3,2-c]pyridin-4-yl)-N-(4-hydroxybicyclo[2.2.2]octan-1-yl)benzamide |
| (18) | 4-[7-(Difluoromethyl)-1H-pyrrolo[3,2-c]pyridin-4-yl]-N-[trans-4-(2-hydroxypropan-2-yl)cyclohexyl]benzamide |

**[Table 1-2]**

| | |
|---|---|
| (19) | 4-(7-Fluoro-1H-pyrrolo[3,2-c]pyridin-4-yl)-N-(4-hydroxybicycle[2.2.1]heptan-1-yl)benzamide |
| (20) | 4-(7-Fluoro-1H-pyrrolo[3,2-c]pyridin-4-yl)-N-(trans-4-hydroxycyclohexyl)benzamide |
| (21) | 4-(7-Fluoro-1H-pyrrolo[3,2-c]pyridin-4-yl)-N-(trans-4-hydroxy-4-methylcyclohexyl)benzamide |
| (22) | 4-(7-Fluoro-1H-pyrrolo[3,2-c]pyridin-4-yl)-N-[trans-4-(2-hydroxy-2-methylpropoxy)cyclohexyl]benzamide |
| (23) | 4-(7-Fluoro-1H-pyrrolo[3,2-c]pyridin-4-yl)-N-(cis-4-hydroxycyclohexyl)benzamide |
| (24) | 4-(7-Fluoro-1H-pyrrolo[3,2-c]pyridin-4-yl)-N-(tetrahydropyran-4-yl)benzamide |
| (25) | N-(1,1-Dioxotetrahydrothiopyran-4-yl)-4-(7-fluoro-1H-pyrrolo[3,2-c]pyridin-4-yl)benzamide |
| (26) | N-(trans-4-Cyanocyclohexyl)-4-(7-fluoro-1H-pyrrolo[3,2-c]pyridin-4-yl)benzamide |
| (27) | N-(trans-4-Carbamoylcyclohexyl)-4-(7-fluoro-1H-pyrrolo[3,2-c]pyridin-4-yl)benzamide |
| (28) | 4-(7-Chloro-1H-pyrrolo[3,2-c]pyridin-4-yl)-N-(tetrahydropyran-4-yl)benzamide |
| (29) | 4-(7-Chloro-1H-pyrrolo[3,2-c]pyridin-4-yl)-N-(1,1-dioxotetrahydrothiopyran-4-yl)benzamide |
| (30) | 4-(7-Chloro-1H-pyrrolo[3,2-c]pyridin-4-yl)-N-(trans-4-hydroxycyclohexyl)benzamide |
| (31) | 4-(7-Chloro-1H-pyrrolo[3,2-c]pyridin-4-yl)-N-(cis-4-hydroxy-4-methylcyclohexyl)benzamide |
| (32) | 4-(7-Chloro-1H-pyrrolo[3,2-c]pyridin-4-yl)-N-(4-hydroxybicyclo[2.2.1]heptan-1-yl)benzamide |
| (33) | 4-(7-Chloro-1H-pyrrolo[3,2-c]pyridin-4-yl)-N-(cis-4-hydroxycyclohexyl)benzamide |
| (34) | 4-(7-Chloro-1H-pyrrolo[3,2-c]pyridin-4-yl)-N-(trans-4-hydroxy-4-methylcyclohexyl)benzamide |
| (35) | 3-Fluoro-4-(7-fluoro-1H-pyrrolo[3,2-c]pyridin-4-yl)-N-[trans-4-(2-hydroxypropan-2-yl)cyclohexyl]benzamide |
| (36) | 3-Fluoro-4-(7-fluoro-1H-pyrrolo[3,2-c]pyridin-4-yl)-N-(4-hydroxybicyclo[2.2.2]octan-1-yl)benzamide |
| (37) | 3-Fluoro-4-(7-fluoro-1H-pyrrolo[3,2-c]pyridin-4-yl)-N-(trans-4-hydroxy-4-methylcyclohexyl)benzamide |
| (38) | 3-Fluoro-4-(7-fluoro-1H-pyrrolo[3,2-c]pyridin-4-yl)-N-(tetrahydropyran-4-yl)benzamide |
| (39) | 3-Fluoro-4-(7-fluoro-1H-pyrrolo[3,2-c]pyridin-4-yl)-N-(trans-4-hydroxycyclohexyl)benzamide |

**[Table 1-3]**

| | |
|---|---|
| (40) | 3-Fluoro-4-(7-fluoro-1H-pyrrolo[3,2-c]pyridin-4-yl)-N-(cis-4-hydroxy-4-methylcyclohexyl)benzamide |
| (41) | 3-Fluoro-4-(7-fluoro-1H-pyrrolo[3,2-c]pyridin-4-yl)-N-(4-hydroxybicyclo[2.2.1]heptan-1-yl)benzamide |
| (42) | 3-Fluoro-4-(7-fluoro-1H-pyrrolo[3,2-c]pyridin-4-yl)-N-(cis-4-hydroxycyclohexyl)benzamide |
| (43) | N-[trans-4-(2-Hydroxypropan-2-yl)cyclohexyl]-4-(1H-pyrazolo[4,3-c]pyridin-4-yl)benzamide |
| (44) | N-[trans-4-(2-Hydroxypropan-2-yl)cyclohexyl]-4-(1H-imidazo[4,5-c]pyridin-4-yl)benzamide |
| (45) | 4-(1H-[1,2,3]Triazolo[4,5-c]pyridin-4-yl)-N-[trans-4-(2-hydroxypropan-2-yl)cyclohexyl]benzamide |

**[Table 1-4]**

| | |
|---|---|
| (46) | 4-(7-Fluoro-1H-pyrazolo[4,3-c]pyridin-4-yl)-N-[trans-4-(2-hydroxypropan-2-yl)cyclohexyl]benzamide |
| (47) | 4-(7-Fluoro-1H-pyrazolo[4,3-c]pyridin-4-yl)-N-(4-hydroxybicyclo[2.2.2]octan-1-yl)benzamide |
| (48) | 4-(7-Chloro-1H-pyrazolo[4,3-c]pyridin-4-yl)-N-[trans-4-(2-hydroxypropan-2-yl)cyclohexyl]benzamide |
| (49) | 3-Fluoro-N-[trans-4-(2-hydroxypropan-2-yl)cyclohexyl]-4-(1H-pyrazolo[4,3-c]pyridin-4-yl)benzamide |

Regarding the compound of Formula (I), stereoisomers such as an enantiomer and a diastereomer can exist depending on the kind of the substituent. Unless otherwise stated, the compound of Formula (I) includes all such stereoisomers. The compound of Formula (I) also includes a mixture of such stereoisomers (including racemates).

The compound of Formula (I) may exist as tautomers. Even when only one tautomeric structure is depicted herein, both tautomeric forms are included in the invention.

The compound of Formula (I) includes isotopologues in which 1 or 2 or more atoms in the molecule are substituted with an isotope. In the present specification, an isotope means an atom having the same atomic number and a different mass number. Therefore, "substitution with an isotope" in the present specification means substitution with an atom having the same atomic number but having a mass number different from that normally existing in nature.

For example, the hydrogen atom that constitutes the compound of Formula (I) may be substituted with ²H (D) or ³H (T), and similarly, the carbon atom that constitutes Formula (I) may be substituted with ¹¹C, ¹³C, or ¹⁴C. Isotopologues substituted with stable isotopes such as ²H (D) are useful as therapeutic advantages may be obtained due to higher metabolic stability. On the other hand, isotopologues substituted with radioisotopes such as ³H (T) and ¹⁴C are useful in drug and/or substrate tissue distribution studies.

The isotopologues can be prepared by the methods disclosed herein or similar methods using appropriate reagents containing the corresponding isotopes.

The "pharmaceutically acceptable salt" of the compound of Formula (I) is not particularly limited as long as it is pharmaceutically acceptable. Examples thereof include a base addition salt of a carboxy group, a hydroxy group, or an acidic heteroaryl group when the compound of Formula (I) has a carboxy group, a hydroxy group, or an acidic heteroaryl group (tetrazolyl group and the like), and an acid addition salt of an amino group or a basic heteroaryl group when the compound of Formula (I) has an amino group or a basic heteroaryl group.

Examples of the base addition salt include alkali metal salts (for example, sodium salts and potassium salts); alkaline earth metal salts (for example, calcium salts and magnesium salts); ammonium salts; and organic amine salts (for example, trimethylamine salts, triethylamine salts, dicyclohexylamine salts, ethanolamine salts, diethanolamine salts, triethanolamine salts, procaine salts, and N,N'-dibenzylethylenediamine salts).

Examples of the acid addition salt include inorganic acid salts (for example, hydrochlorides, sulfates, nitrates, phosphates, and perchlorates); organic acid salts (for example, maleates, fumarates, tartrates, citrates, ascorbates, and trifluoroacetates); and sulfonates (for example, methanesulfonates, isethionates, benzenesulfonates, p-toluenesulfonates).

The "pharmaceutically acceptable salt" can be produced according to a method used in the field of organic synthesis. For example, the pharmaceutically acceptable salt can be produced by neutralizing and titrating a solution of a free form of the compound of Formula (I) with an alkaline solution or an acidic solution.

The "pharmaceutically acceptable salt" also includes solvates with pharmaceutically acceptable solvents such as water and ethanol.

Then, the production method of the compound of the present invention will be described. The following production method is an example, and the production method of the compound of the present invention is not limited thereto.

The compound of the present invention can be produced by Production method A or Production method B below.

A step of substituent introduction or functional group transformation and/or a step of protection/deprotection may be inserted between the steps of the production method as necessary.

As a method of substituent introduction or functional group transformation, for example, a method described in a literature [see Comprehensive Organic Transformations, 2nd edition, R. C. Larock, Wiley-VCH (1999)] and the like can be used.

As a protection/deprotection method, for example, a method described in a literature [see Protective Groups in Organic Synthesis, 3rd edition, T.W. Greene, John Wiley & Sons Inc. (1999)] and the like can be used.

The compound used in each step of the production method may form a salt or a solvate as long as the reaction is not hindered.

The isolation/purification may be performed according to a conventional method (extraction, crystallization, recrystallization, distillation, column chromatography and the like).

Production method A: production method of compound of Formula (I) (1) 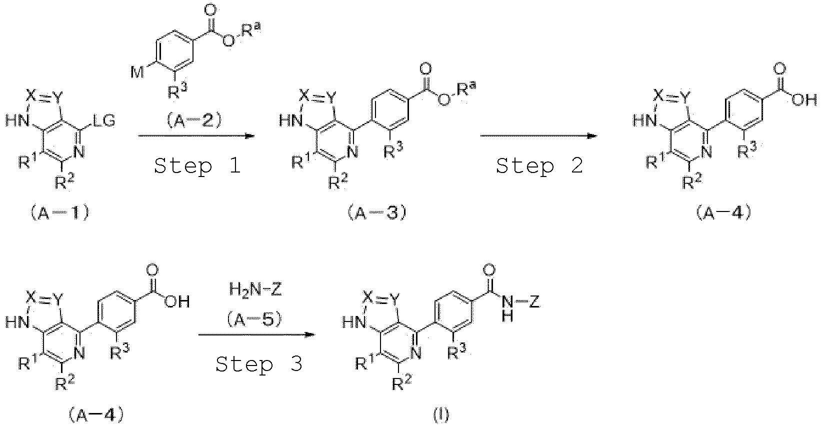 (wherein
-X=, =Y-, R¹, R², R³, and Z are as defined above,
LG represents a leaving group such as a halogen atom or a trifluoromethanesulfonyloxy group,
M represents a boron-containing functional group such as a dihydroxyboryl group (borono group) or a 4,4,5,5-tetramethyl-1,3,2-dioxaboran-2-yl group, and
R^{a} represents a C₁₋₆ alkyl group.)

### Step 1

A compound of Formula (A-1) and a compound of Formula (A-2) are subjected to a coupling reaction in the presence of a metal catalyst and a base to give a compound of Formula (A-3).

As the compound of Formula (A-1) and the compound of Formula (A-2), a commercially available product may be used, or a compound synthesized by a known method or the like may be used.

Examples of the metal catalyst include tetrakis(triphenylphosphine)palladium (0), palladium (II) acetate, tris(dibenzylideneacetone)dipalladium (0), bis[di-tert-butyl(4-dimethylaminophenyl)phosphine]dichloropalladium (II), [1,1'-bis(diphenylphosphino)ferrocene]dichloropalladium (II), [1,1'-bis(diphenylphosphino)ferrocene]dichloropalladium (II) dichloromethane complex, [1,3-bis(2,6-diisopropylphenyl)imidazol-2-ylidene]chloro[3-phenylallyl]palladium (II), and [1,3-bis(2,6-diisopropylphenyl)-4,5-dihydroimidazol-2-ylidene]chloro[3-phenylallyl]palladium (II).

In step 1, a phosphine ligand may be used as necessary. Examples of the phosphine ligand include triphenylphosphine, tris(2-methylphenyl)phosphine, tri(2-furyl)phosphine, tri-tert-butylphosphine, (4-dimethylaminophenyl)di-tert-butylphosphine (Amphos), 2-[di(tert-butyl)phosphino]-1,1'-biphenyl (JohnPhos), 2-[di(tert-butyl)phosphino]-2'-N,N-dimethylamino-1,1'-biphenyl (tBuDavePhos), 2-(dicyclohexylphosphino)-1,1'-biphenyl (CyJohnPhos), 2-(dicyclohexylphosphino)-2'-N,N-dimethylamino-1,1'-biphenyl (DavePhos), 2-dicyclohexylphosphino-2',6'-dimethoxybiphenyl (SPhos), 2-dicyclohexylphosphino-2',4',6'-triisopropylbiphenyl (XPhos), and 4,5-bis(diphenylphosphino)-9,9-dimethylxanthene (Xantphos).

Examples of the base include sodium carbonate, potassium carbonate, cesium carbonate, sodium bicarbonate, tripotassium phosphate, potassium fluoride, cesium fluoride, potassium tert-butoxide, lithium hydroxide, sodium hydroxide, potassium hydroxide, N,N-diisopropylethylamine, and triethylamine.

The reaction solvent is not particularly limited as long as the reaction solvent does not interfere with the reaction, and examples thereof include tetrahydrofuran, 1,4-dioxane, 1,2-dimethoxyethane, toluene, methanol, ethanol, acetonitrile, and water.

The solvent may be used as a mixed solvent in which two or more kinds are combined at an appropriate ratio.

Examples of the mixed solvent include a combination of methanol and water, a combination of ethanol and water, a combination of toluene and water, and a combination of 1,4-dioxane and water.

The amount used of the compound of Formula (A-2) is usually 1 to 3 mol, and preferably 1 to 2 mol relative to 1 mol of the compound of Formula (A-1).

The amount used of the metal catalyst is usually 0.01 to 0.5 mol, and preferably 0.03 to 0.2 mol relative to 1 mol of the compound of Formula (A-1).

The amount used of the base is usually 1 to 10 mol, and preferably 1 to 6 mol relative to 1 mol of the compound of Formula (A-1).

The reaction temperature is usually 20°C to 160°C, and preferably 20°C to 120°C. The reaction can also be performed under microwave irradiation as necessary.

The reaction time is usually 10 minutes to 48 hours, and preferably 30 minutes to 12 hours.

### Step 2

A compound of Formula (A-3) is hydrolyzed in the presence of a base to give a compound of Formula (A-4).

Examples of the base include lithium hydroxide, sodium hydroxide, and potassium hydroxide.

The reaction solvent is not particularly limited as long as the reaction solvent does not interfere with the reaction, and examples thereof include methanol, ethanol, 1-propanol, 2-propanol, 1-butanol, 2-butanol, tert-butanol, tetrahydrofuran, 1,4-dioxane, and water.

The solvent may be used as a mixed solvent in which two or more kinds are combined at an appropriate ratio.

Examples of the mixed solvent include a combination of methanol and water, a combination of ethanol and water, and a combination of tetrahydrofuran and water.

The amount used of the base is usually 1 mol to large excess relative to 1 mol of the compound of Formula (A-3) .

The reaction temperature is usually 0°C to 160°C, and preferably 0°C to 130°C.

The reaction time is usually 10 minutes to 48 hours, and preferably 10 minutes to 5 hours.

### Step 3

A compound of Formula (A-4) and a compound of Formula (A-5) are subjected to a condensation reaction to give a compound of Formula (I).

As the compound of Formula (A-5), a commercially available product may be used, or a compound synthesized by a known method or the like may be used.

In step 3, a condensing agent may be used as necessary. Examples of the condensing agent include N,N'-dicyclohexylcarbodiimide (DCC), 1-ethyl-3-(3-dimethylaminopropyl)carbodiimide hydrochloride (EDC), N,N'-carbonyldiimidazole (CDI), diphenylphosphate azide (DPPA), (1-cyano-2-ethoxy-2-oxoethylideneaminooxy)dimethylaminomorpholinocarbenium hexafluorophosphate (COMU), and O-(7-azabenzotriazol-1-yl)-N,N,N',N'-tetramethyluronium hexafluorophosphate (HATU).

In step 3, a base may be used as necessary. Examples of the base include N,N-diisopropylethylamine, triethylamine, sodium carbonate, potassium carbonate, cesium carbonate, sodium bicarbonate, tripotassium phosphate, potassium fluoride, cesium fluoride, potassium tert-butoxide, lithium hydroxide, sodium hydroxide, and potassium hydroxide.

The reaction solvent is not particularly limited as long as the reaction solvent does not interfere with the reaction, examples thereof include N,N-dimethylformamide, N,N-dimethylacetamide, N-methylpyrrolidone, tetrahydrofuran, 1,4-dioxane, dichloromethane, chloroform, dimethylsulfoxide, acetonitrile, ethyl acetate, toluene, methanol, ethanol, and water.

The amount used of the compound of Formula (A-5) is usually 1 to 5 mol, and preferably 1 to 2 mol relative to 1 mol of the compound of Formula (A-4).

The amount used of the condensing agent is usually 1 to 10 mol, and preferably 1 to 3 mol relative to 1 mol of the compound of Formula (A-4).

The amount used of the base is usually 1 to 20 mol, and preferably 1 to 10 mol relative to 1 mol of the compound of Formula (A-4).

The reaction temperature is usually 0°C to 100°C, and preferably 0°C to 60°C.

The reaction time is usually 10 minutes to 48 hours, and preferably 10 minutes to 30 hours.

Production method B: production method of compound of Formula (I) (2) 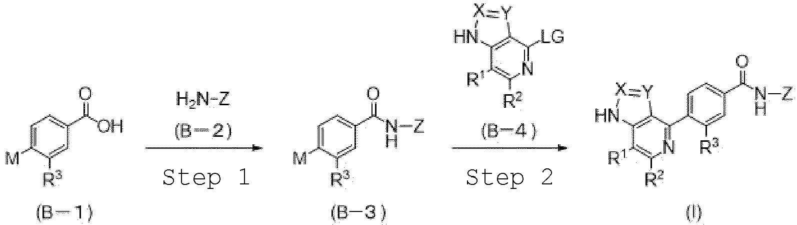 (wherein -X=, =Y-, R¹, R², R³, Z, LG, and M are as defined above.)

### Step 1

A compound of Formula (B-1) and a compound of Formula (B-2) are subjected to a condensation reaction to give a compound of Formula (B-3).

As the compound of Formula (B-1) and the compound of Formula (B-2), a commercially available product may be used, or a compound synthesized by a known method or the like may be used.

In step 1, a condensing agent may be used as necessary. Examples of the condensing agent include N,N'-dicyclohexylcarbodiimide (DCC), 1-ethyl-3-(3-dimethylaminopropyl)carbodiimide hydrochloride (EDC), N,N'-carbonyldiimidazole (CDI), diphenylphosphate azide (DPPA), (1-cyano-2-ethoxy-2-oxoethylideneaminooxy)dimethylaminomorpholinocarbenium hexafluorophosphate (COMU), and O-(7-azabenzotriazol-1-yl)-N,N,N',N'-tetramethyluronium hexafluorophosphate (HATU).

In step 1, a base may be used as necessary. Examples of the base include N,N-diisopropylethylamine, triethylamine, sodium carbonate, potassium carbonate, cesium carbonate, sodium bicarbonate, tripotassium phosphate, potassium fluoride, cesium fluoride, potassium tert-butoxide, lithium hydroxide, sodium hydroxide, and potassium hydroxide.

The reaction solvent is not particularly limited as long as the reaction solvent does not interfere with the reaction, examples thereof include N,N-dimethylformamide, N,N-dimethylacetamide, N-methylpyrrolidone, tetrahydrofuran, 1,4-dioxane, dichloromethane, chloroform, dimethylsulfoxide, acetonitrile, ethyl acetate, toluene, methanol, ethanol, and water.

The amount used of the compound of Formula (B-2) is usually 1 to 5 mol, and preferably 1 to 2 mol relative to 1 mol of the compound of Formula (B-1).

The amount used of the condensing agent is usually 1 to 10 mol, and preferably 1 to 3 mol relative to 1 mol of the compound of Formula (B-1).

The amount used of the base is usually 1 to 20 mol, and preferably 1 to 10 mol relative to 1 mol of the compound of Formula (B-1).

The reaction temperature is usually 0°C to 100°C, and preferably 0°C to 60°C.

The reaction time is usually 10 minutes to 48 hours, and preferably 10 minutes to 30 hours.

### Step 2

A compound of Formula (B-3) and a compound of Formula (B-4) are subjected to a coupling reaction in the presence of a metal catalyst and a base to give a compound of Formula (I).

As the compound of Formula (B-4), a commercially available product may be used, or a compound synthesized by a known method or the like may be used.

Examples of the metal catalyst include tetrakis(triphenylphosphine)palladium (0), palladium (II) acetate, tris(dibenzylideneacetone)dipalladium (0), bis[di-tert-butyl(4-dimethylaminophenyl)phosphine]dichloropalladium (II) (PdCl₂ (Amphos)₂, [1,1'-bis(diphenylphosphino)ferrocene]dichloropalladium (II), [1,1'-bis(diphenylphosphino)ferrocene]dichloropalladium (II) dichloromethane complex, [1,3-bis(2,6-diisopropylphenyl)imidazol-2-ylidene]chloro[3-phenylallyl]palladium (II), and [1,3-bis(2,6-diisopropylphenyl)-4,5-dihydroimidazol-2-ylidene]chloro[3-phenylallyl]palladium (II).

In step 2, a phosphine ligand may be used as necessary. Examples of the phosphine ligand include triphenylphosphine, tris(2-methylphenyl)phosphine, tri(2-furyl)phosphine, tri-tert-butylphosphine, (4-dimethylaminophenyl)di-tert-butylphosphine (Amphos), 2-[di(tert-butyl)phosphino]-1,1'-biphenyl (JohnPhos), 2-[di(tert-butyl)phosphino]-2'-N,N-dimethylamino-1,1'-biphenyl (tBuDavePhos), 2-(dicyclohexylphosphino)-1,1'-biphenyl (CyJohnPhos), 2-(dicyclohexylphosphino)-2'-N,N-dimethylamino-1,1'-biphenyl (DavePhos), 2-dicyclohexylphosphino-2',6'-dimethoxybiphenyl (SPhos), 2-dicyclohexylphosphino-2',4',6'-triisopropylbiphenyl (XPhos), and 4,5-bis(diphenylphosphino)-9,9-dimethylxanthene (Xantphos).

Examples of the base include sodium carbonate, potassium carbonate, cesium carbonate, sodium bicarbonate, tripotassium phosphate, potassium fluoride, cesium fluoride, potassium tert-butoxide, lithium hydroxide, sodium hydroxide, potassium hydroxide, N,N-diisopropylethylamine, and triethylamine.

The reaction solvent is not particularly limited as long as the reaction solvent does not interfere with the reaction, and examples thereof include tetrahydrofuran, 1,4-dioxane, 1,2-dimethoxyethane, toluene, methanol, ethanol, acetonitrile, and water.

The solvent may be used as a mixed solvent in which two or more kinds are combined at an appropriate ratio.

Examples of the mixed solvent include a combination of methanol and water, a combination of ethanol and water, a combination of toluene and water, and a combination of 1,4-dioxane and water.

The amount used of the compound of Formula (B-3) is usually 1 to 3 mol, and preferably 1 to 2 mol relative to 1 mol of the compound of Formula (B-4).

The amount used of the metal catalyst is usually 0.01 to 0.5 mol, and preferably 0.03 to 0.2 mol relative to 1 mol of the compound of Formula (B-4).

The amount used of the base is usually 1 to 10 mol, and preferably 1 to 6 mol relative to 1 mol of the compound of Formula (B-4).

The reaction temperature is usually 20°C to 160°C, and preferably 20°C to 120°C. The reaction can also be performed under microwave irradiation as necessary.

The reaction time is usually 10 minutes to 48 hours, and preferably 30 minutes to 12 hours.

A pharmaceutical composition and an H-PGDS inhibitor comprising the compound of the present invention will be described.

"H-PGDS" refers to hematopoietic prostaglandin D synthase (hematopoietic PGD synthase).

"Inhibiting H-PGDS" refers to deleting or decreasing the activity of H-PGDS as prostaglandin D synthase, for example, inhibiting the activity of H-PGDS under the conditions described in Example 50 described later.

The "H-PGDS inhibitor" refers to an agent for inhibiting H-PGDS.

The "pharmaceutical composition for treating or preventing a disease involving H-PGDS" refers to a pharmaceutical composition for treating or preventing a disease involving H-PGDS by inhibiting H-PGDS.

Examples of the "disease involving H-PGDS" include asthma, chronic obstructive pulmonary disease, allergic rhinitis, sinusitis, eosinophilic pneumonia, atherosclerosis, rheumatoid arthritis, cystic fibrosis, actinic keratosis, chronic urticaria, dermatitis, muscular dystrophy, sarcopenia, disuse muscle atrophy, muscle damage, wounds, dermatomyositis, amyotrophic lateral sclerosis, cerebral infarction, myocardial infarction, ischemic bowel disease, ischemic renal disease, ischemic stomach disease, ischemic liver disease, diabetic ischemic limb, and Buerger's disease.

The pharmaceutical composition and inhibitor of the present invention can be provided as a preparation.

The "preparation" may contain a pharmaceutically acceptable carrier together with the compound of the present invention.

The preparation can be produced using preparation techniques commonly used in the pharmaceutical field.

Examples of the "pharmaceutically acceptable carrier" include solvents (for example, purified water, ethanol, propylene glycol, macrogol, sesame oil, corn oil, and olive oil), excipients (for example, lactose, white sugar, D-mannitol, starch, corn starch, crystalline cellulose, and light anhydrous silicic acid), disintegrants (for example, starch, carboxymethyl cellulose, calcium carboxymethyl cellulose, sodium carboxymethyl starch, and L-hydroxypropyl cellulose), binders (for example, crystalline cellulose, white sugar, D-mannitol, dextrin, hydroxypropyl cellulose, hydroxypropyl methylcellulose, polyvinylpyrrolidone, starch, sucrose, gelatin, methylcellulose, and sodium carboxymethylcellulose), lubricants (for example, magnesium stearate, calcium stearate, talc, and colloidal silica), wetting agents (for example, propylene glycol monostearate, sorbitan monooleate, diethylene glycol monolaurate, and polyoxyethylene lauryl ether), antioxidants (for example, sodium sulfite, potassium sulfite, ascorbic acid, and α-tocopherol), suspending agents (stearyl triethanolamine, sodium lauryl sulfate, lauryl aminopropionic acid, lecithin, benzalkonium chloride, benzethonium chloride, glycerin monostearate, polyvinyl alcohol, polyvinyl pyrrolidone, sodium carboxymethyl cellulose, methyl cellulose, hydroxymethyl cellulose, hydroxyethyl cellulose, and hydroxypropyl cellulose), preservatives (for example, ethyl parahydroxybenzoate, chlorobutanol, benzyl alcohol, sodium dehydroacetate, sorbic acid, and potassium sorbate), buffers (for example, sodium hydrogen phosphate, sodium acetate, sodium carbonate, and sodium citrate), and solubilizers (for example, propylene glycol, D-mannitol, benzyl benzoate, ethanol, triethanolamine, sodium carbonate, and sodium citrate).

The preparation can be present in a variety of dosage forms depending on the use. The preparation may be solid or liquid. The preparation may be an oral preparation or an external preparation. Examples of the preparation include tablets, capsules, powders, granules, liquids, suppositories, ointments, injections, and drops.

The content of the compound of the present invention in the preparation can be appropriately selected depending on the use. For example, in the case of an oral preparation, 0.1 to 100% by mass, preferably 5 to 98% by mass of the compound of the present invention can be compounded as an active ingredient relative to the total mass of the formulation.

The preparation may contain an agent other than the compound of the present invention (hereinafter, also referred to as "concomitant drug"). The concomitant drug can be appropriately selected according to the use.

"Concomitant" means that multiple active ingredients are used in combination. Examples of the concomitant include use as a compounding agent, use as a kit, and an embodiment in which each active ingredient is separately administered in the same or different administration routes.

### Examples

Hereinafter, the present invention will be described in more detail with reference to Examples. These are examples, and the present invention is not limited to the following Examples.

As the various reagents used in Examples, commercial products were used unless otherwise specified.

In Examples, room temperature means 1°C to 40°C.

In silica gel column chromatography, unless otherwise specified, Biotage (registered trademark) SNAP Ultra Silica Cartridge or SNAP KP-Sil Cartridge (Biotage), or CHROMATOREX (registered trademark) Q-PACK SI, Q-PACK NH, or Q-PACK CO₂H (FUJI SILYSIA CHEMICAL LTD.) was used as a column, and ethyl acetate/n-hexane, methanol/chloroform, or methanol/ethyl acetate was used as a mobile phase.

In reversed-phase silica gel column chromatography, Biotage (registered trademark) SNAP Ultra C18 Cartridge (Biotage) was used as a column, and a 0.1% aqueous trifluoroacetic acid solution and a 0.1% trifluoroacetic acid acetonitrile solution were used as mobile phases.

In preparative thin layer chromatography, PLC Silica gel 60 F254 (Merck) was used as a TLC (silica gel plate).

¹H-NMR was measured using ECZ400S (400 MHz, JEOL Ltd.). The chemical shifts of the NMR data were described in parts per million (ppm, δ) based on the residual protons in the deuterated solvent used.

The mass spectrum (MS) was measured by electrospray ionization (ESI) using ACQUITY (registered trademark) SQD (Waters) unless otherwise specified. For mass spectrum (ESI-MS) data measured by ESI, actual measured values are shown. In the case of a salt, a molecular ion peak of a free form is usually observed.

The microwave reaction was performed using Initiator (registered trademark) (Biotage).

The meanings of the abbreviations are shown below.
s: singlet
d: doublet
t: triplet
q: quartet
dd: double doublet
m: multiplet
br: broad
CDCl₃: deuterated chloroform
CD₃OD: deuterated methanol
COMU: (1-cyano-2-ethoxy-2-oxoethylideneaminooxy)dimethylaminomorpholinocarbenium hexafluorophosphate
DMSO-d₆: deuterated dimethyl sulfoxide
DIPEA: N,N-diisopropylethylamine
DMF: N,N-dimethylformamide
DMSO: dimethyl sulfoxide
PdCl₂(Amphos)₂: bis[di-tert-butyl(4-dimethylaminophenyl)phosphine]dichloropalladium (II)
PdCl₂ (dppf): [1,1'-bis(diphenylphosphino)ferrocene]dichloropalladium (II)
PdCl₂(dppf) ·CH₂Cl₂: [1,1'-bis(diphenylphosphino)ferrocene]dichloropalladium (II) dichloromethane complex
THF: tetrahydrofuran

### Example 1

### Synthesis of N-(4-hydroxybicyclo[2.2.2]octan-1-yl)-4-(7-methyl-1H-pyrrolo[3,2-c]pyridin-4-yl)benzamide [1] (hereinafter, referred to as a compound [1])

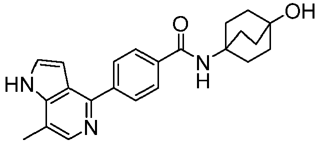

### (1) Synthesis of ethyl 4-(7-methyl-1H-pyrrolo[3,2-c]pyridin-4-yl)benzoate [1-1] (hereinafter, referred to as a compound [1-1])

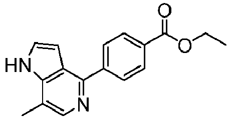

To a solution of 4-chloro-7-methyl-1H-pyrrolo[3,2-c]pyridine (210 mg) in ethanol (1.3 mL)/water (1.3 mL) were added 4-(ethoxycarbonyl)phenylboronic acid (318 mg), potassium carbonate (227 mg), and [1,3-bis(2,6-diisopropylphenyl)-4,5-dihydroimidazol-2-ylidene]chloro[3-phenylallyl]palladium (II) (33.0 mg) at room temperature, and the mixture was stirred at 100°C for 1 hour under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (313 mg) as a yellow solid.

¹H-NMR (400 MHz, CDCl₃) δ: 8.47 (br, 1H), 8.27 (s, 1H), 8.20-8.17 (m, 2H), 8.08-8.05 (m, 2H), 7.35-7.33 (m, 1H), 6.87-6.86 (m, 1H), 4.42 (q, J = 7.1 Hz, 2H), 2.54 (s, 3H), 1.43 (t, J = 7.1 Hz, 3H).

ESI-MS: 281.3[M+H]⁺

### (2) Synthesis of ethyl 4-(7-methyl-1-{[2-(trimethylsilyl)ethoxy]methyl}-1H-pyrrolo[3,2-c]pyridin-4-yl)benzoate [1-2] (hereinafter, referred to as a compound [1-2])

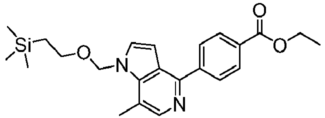

To a solution of the compound [1-1] (209 mg) in THF (2.5 mL) were added 60% sodium hydride (36 mg) and 2-(trimethylsilyl)ethoxymethyl chloride (144 µL) at room temperature, and the mixture was stirred at room temperature for 30 minutes. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (225 mg) as a yellow oil.

ESI-MS: 411.4[M+H]⁺

### (3) Synthesis of 4-(7-methyl-1-{[2-(trimethylsilyl)ethoxy]methyl}-1H-pyrrolo[3,2-c]pyridin-4-yl)benzoate [1-3] (hereinafter, referred to as a compound [1-3])

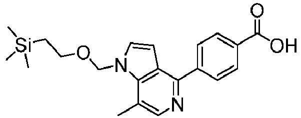

To a solution of the compound [1-2] (225 mg) in methanol (1.4 mL) was added a 2 M aqueous sodium hydroxide solution (1.4 mL) at room temperature, and the mixture was stirred at 68°C for 2 hours. The reaction mixture was concentrated under reduced pressure, then 2 M hydrochloric acid (1.4 mL) was added, and the resulting solid was collected by filtration and dried under reduced pressure to give the title compound (194 mg) as a white solid.

ESI-MS: 383.4[M+H]⁺

### (4) Synthesis of N-(4-hydroxybicyclo[2.2.2]octan-1-yl)-4-(7-methyl-1H-pyrrolo[3,2-c]pyridin-4-yl)benzamide [1]

To a solution of the compound [1-3] (31 mg) in DMF (0.8 mL) were added DIPEA (34 µL) and COMU (39 mg) at room temperature, and the mixture was stirred at room temperature for 5 minutes. To the reaction mixture was added 4-aminobicyclo[2.2.2]octan-1-ol hydrochloride (18 mg) at room temperature, and the mixture was stirred at room temperature for 40 minutes. To the reaction mixture was added a saturated aqueous sodium bicarbonate solution, and the mixture was extracted with ethyl acetate. The organic layer was dried over anhydrous sodium sulfate and concentrated under reduced pressure. To the obtained residue was added a solution of 1 M tetrabutylammonium fluoride in THF (1.4 mL) at room temperature, and the mixture was stirred at 60°C for 5 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (20 mg) as a white solid.

¹H-NMR (400 MHz, DMSO-d₆) δ: 11.73 (br, 1H), 8.09 (d, J = 8.7 Hz, 1H), 8.07-7.97 (m, 2H), 7.89-7.84 (m, 2H), 7.66 (d, J = 8.2 Hz, 1H), 7.54 (d, J = 5.5 Hz, 1H), 6.77 (d, J = 5.5 Hz, 1H), 4.29 (br, 1H), 2.49 (s, 3H), 2.05-1.98 (m, 6H), 1.63-1.58 (m, 6H).

ESI-MS: 376.4[M+H]⁺

### Example 2

Synthesis of N-(trans-4-hydroxy-4-methylcyclohexyl)-4-(7-methyl-1H-pyrrolo[3,2-c]pyridin-4-yl)benzamide [2]

### (hereinafter, referred to as a compound [2])

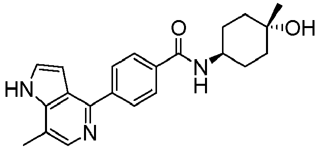

To a solution of the compound [1-3] (31 mg) in DMF (0.8 mL) were added DIPEA (30 µL) and COMU (39 mg) at room temperature, and the mixture was stirred at room temperature for 5 minutes. To the reaction mixture was added trans-4-amino-1-methylcyclohexanol hydrochloride (13 mg) at room temperature, and the mixture was stirred at room temperature for 40 minutes. To the reaction mixture was added a saturated aqueous sodium bicarbonate solution, and the mixture was extracted with ethyl acetate. The organic layer was dried over anhydrous sodium sulfate and concentrated under reduced pressure. To the obtained residue was added a solution of 1 M tetrabutylammonium fluoride in THF (990 µL) at room temperature, and the mixture was stirred at 60°C for 4.5 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (18 mg) as a white solid.

¹H-NMR (400 MHz, DMSO-d₆) δ: 11.76 (br, 1H), 8.22 (d, J = 7.8 Hz, 1H), 8.10 (s, 1H), 8.04 (d, J = 7.3 Hz, 2H), 7.95 (d, J = 7.3 Hz, 2H), 7.55 (d, J = 1.8 Hz, 1H), 6.80-6.79 (m, 1H), 4.29 (s, 1H), 3.85-3.75 (m, 1H), 2.49 (s, 3H), 1.78-1.75 (m, 2H), 1.61-1.43 (m, 6H), 1.16 (s, 3H).

ESI-MS: 364.4[M+H]⁺

### Example 3

Synthesis of N-[trans-4-(1-hydroxycyclopropyl)cyclohexyl]-4-(7-methyl-1H-pyrrolo[3,2-c]pyridin-4-yl)benzamide [3] (hereinafter, referred to as a compound [3])

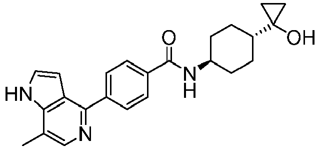

### (1) Synthesis of methyl trans-4-(dibenzylamino)cyclohexane-1-carboxylate [3-1] (hereinafter, referred to as a compound [3-1])

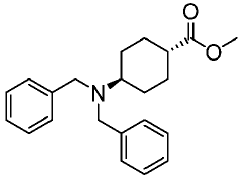

To a solution of methyl trans-4-aminocyclohexanecarboxylate hydrochloride (387 mg) in acetonitrile (6.7 mL) were added potassium carbonate (1.11 g) and benzyl bromide (598 µL) at room temperature, and the mixture was stirred at 80°C for 9 hours. The reaction mixture was filtered, and the filtrate was concentrated under reduced pressure. The obtained residue was purified by reversed-phase silica gel column chromatography to give the title compound (344 mg) as a white solid.

¹H-NMR (400 MHz, CDCl₃) δ: 7.36-7.35 (m, 4H), 7.30-7.26 (m, 4H), 7.22-7.18 (m, 2H), 3.63-3.62 (m, 7H), 2.55-2.49 (m, 1H), 2.23-2.17 (m, 1H), 2.02-1.95 (m, 4H), 1.43-1.30 (m, 4H).

ESI-MS: 338.6[M+H]⁺

### (2) Synthesis of 1-[trans-4-(dibenzylamino)cyclohexyl]cyclopropan-1-ol [3-2] (hereinafter, referred to as a compound [3-2])

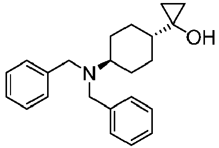

To a solution of the compound [3-1] (238 mg) in THF (23.5 mL) was added titanium tetraisopropoxide (620 µL) at room temperature under an argon atmosphere. To this reaction mixture, a solution of 1 M ethyl magnesium bromide in THF (4.23 mL) was added dropwise over 5 minutes, and then the mixture was stirred at room temperature for 21 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (186 mg) as a white solid.

¹H-NMR (400 MHz, CDCl₃) δ: 7.38-7.36 (m, 4H), 7.30-7.26 (m, 4H), 7.22-7.18 (m, 2H), 3.62 (s, 4H), 2.54-2.47 (m, 1H), 1.99-1.95 (m, 2H), 1.83-1.80 (m, 2H), 1.62 (s, 1H), 1.42-1.32 (m, 2H), 1.26-1.16 (m, 2H), 0.93-0.85 (m, 1H), 0.68-0.66 (m, 2H), 0.42-0.39 (m, 2H).

ESI-MS: 336.1[M+H]⁺

### (3) Synthesis of 1-(trans-4-aminocyclohexyl)cyclopropan-1-ol [3-3] (hereinafter, referred to as a compound [3-3])

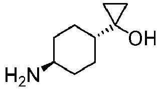

To a solution of the compound [3-2] (185 mg) in ethanol (6.9 mL) was added 20% palladium hydroxide-activated carbon (37.0 mg) at room temperature, and the mixture was stirred at room temperature for 17 hours under a hydrogen atmosphere. The reaction mixture was filtered, and the filtrate was concentrated under reduced pressure to give the title compound (81.8 mg) as a white solid.

¹H-NMR (400 MHz, CDCl₃) δ: 2.67-2.59 (m, 1H), 1.94-1.90 (m, 2H), 1.81-1.76 (m, 2H), 1.41-1.30 (m, 2H), 1.13-1.03 (m, 2H), 0.95-0.87 (m, 1H), 0.72-0.69 (m, 2H), 0.46-0.43 (m, 2H).

ESI-MS: 156.3[M+H]⁺

### (4) Synthesis of N-[trans-4-(1-hydroxycyclopropyl)cyclohexyl]-4-(7-methyl-1H-pyrrolo[3,2-c]pyridin-4-yl)benzamide [3]

To a solution of the compound [1-3] (32 mg) in DMF (0.8 mL) were added DIPEA (30 µL) and COMU (39 mg) at room temperature, and the mixture was stirred at room temperature for 5 minutes. To the reaction mixture was added the compound [3-3] (13 mg) at room temperature, and the mixture was stirred at room temperature for 1 hour. To the reaction mixture was added a saturated aqueous sodium bicarbonate solution, and the mixture was extracted with ethyl acetate. The organic layer was dried over anhydrous sodium sulfate and concentrated under reduced pressure. To the obtained residue was added a solution of 1 M tetrabutylammonium fluoride in THF (960 µL) at room temperature, and the mixture was stirred at 60°C for 4.5 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (18 mg) as a white solid.

¹H-NMR (400 MHz, CD₃OD) δ: 8.05 (s, 1H), 7.97-7.93 (m, 4H), 7.45 (d, J = 3.2 Hz, 1H), 6.77 (d, J = 3.2 Hz, 1H), 3.91-3.88 (m, 1H), 2.55 (s, 3H), 2.10-2.07 (m, 2H), 1.90-1.86 (m, 2H), 1.55-1.40 (m, 4H), 1.05-0.94 (m, 1H), 0.64 (dd, J = 6.6, 4.8 Hz, 2H), 0.45 (dd, J = 6.6, 4.8 Hz, 2H) .

ESI-MS: 390.4[M+H]⁺

### Example 4

### Synthesis of N-[trans-4-(2-hydroxy-2-methylpropoxy)cyclohexyl]-4-(7-methyl-1H-pyrrolo[3,2-c]pyridin-4-yl)benzamide [4] (hereinafter, referred to as a compound [4])

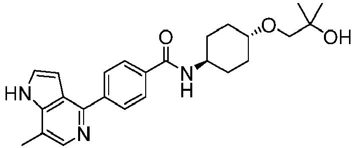

### (1) Synthesis of 4-(7-methyl-1H-pyrrolo[3,2-c]pyridin-4-yl)benzoate [4-1] (hereinafter, referred to as a compound [4-1])

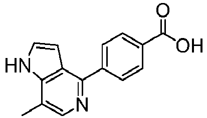

To a solution of the compound [1-1] (650 mg) in methanol (5.8 mL) was added a 2 M aqueous sodium hydroxide solution (5.8 mL) at room temperature, and the mixture was stirred at 68°C for 30 minutes. The reaction mixture was concentrated under reduced pressure, then 2 M hydrochloric acid (5.8 mL) was added, and the resulting solid was collected by filtration and dried under reduced pressure to give the title compound (496 mg) as a white solid.

ESI-MS: 253.3[M+H]⁺

### (2) Synthesis of trans-4-(dibenzylamino)cyclohexan-1-ol [4-2] (hereinafter, referred to as a compound [4-2])

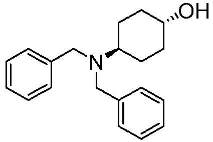

To a solution of trans-4-aminocyclohexan-1-ol (3.00 g) in ethanol (50.0 mL) were added benzyl bromide (6.80 mL) and sodium bicarbonate (8.75 g) at room temperature, and the mixture was stirred at 80°C for 45 minutes. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (2.00 g) as a white solid.

¹H-NMR (400 MHz, CDCl₃) δ: 7.37-7.35 (m, 4H), 7.30-7.26 (m, 4H), 7.22-7.18 (m, 2H), 3.64 (s, 4H), 3.57-3.52 (m, 1H), 2.56-2.48 (m, 1H), 2.14-1.97 (m, 2H), 1.94-1.88 (m, 2H), 1.48-1.38 (m, 2H), 1.23-1.14 (m, 2H).

ESI-MS: 296.3[M+H]⁺

### (3) Synthesis of tert-butyl 2-{[trans-4-(dibenzylamino)cyclohexyl]oxy}acetate [4-3] (hereinafter, referred to as a compound [4-3])

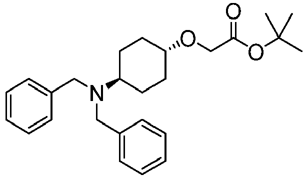

To a solution of the compound [4-2] (2.0 g) in DMF (13 mL) were added 60% sodium hydride (320 mg) and tert-butyl bromoacetate (1.5 mL) at room temperature, and the mixture was stirred at 55°C for 2 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (700 mg) as a yellow oil.

¹H-NMR (400 MHz, CDCl₃) δ: 7.36-7.34 (m, 4H), 7.30-7.26 (m, 4H), 7.21-7.18 (m, 2H), 3.96 (s, 2H), 3.60 (s, 4H), 3.27-3.20 (m, 1H), 2.56-2.49 (m, 1H), 2.10-2.07 (m, 2H), 1.93-1.90 (m, 2H), 1.46-1.43 (m, 13H).

ESI-MS: 410.4[M+H]⁺

### (4) Synthesis of 1-{[trans-4-(dibenzylamino)cyclohexyl]oxy}-2-methylpropan-2-ol [4-4] (hereinafter, referred to as a compound [4-4])

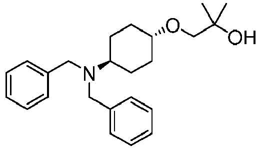

To a solution of the compound [4-3] (700 mg) in THF (3.40 mL) was added a solution of 3 M methylmagnesium bromide in diethyl ether (1.70 mL) at 0°C, and the mixture was stirred at room temperature for 3 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (285 mg) as a yellow oil.

¹H-NMR (400 MHz, CDCl₃) δ: 7.37-7.35 (m, 4H), 7.31-7.27 (m, 4H), 7.22-7.19 (m, 2H), 3.61 (s, 4H), 3.24 (s, 2H), 3.22-3.15 (m, 1H), 2.56-2.50 (m, 1H), 2.08-2.04 (m, 2H), 1.93-1.90 (m, 2H), 1.40-1.31 (m, 2H), 1.20-1.10 (m, 8H) .

ESI-MS: 368.4[M+H]⁺

### (5) Synthesis of 1-[(trans-4-aminocyclohexyl)oxy]-2-methylpropan-2-ol [4-5] (hereinafter, referred to as a compound [4-5])

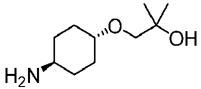

To a solution of the compound [4-4] (285 mg) in ethanol (1.50 mL) was added 20% palladium hydroxide-activated carbon (21.0 mg) at room temperature, and the mixture was stirred at room temperature for 4 hours under a hydrogen atmosphere. The reaction mixture was filtered and concentrated under reduced pressure to give the title compound (140 mg) as a black oil.

¹H-NMR (400 MHz, CDCl₃) δ: 3.27-3.21 (m, 3H), 2.74-2.68 (m, 1H), 2.03-1.99 (m, 2H), 1.89-1.86 (m, 2H), 1.34-1.08 (m, 10H).

ESI-MS: 188.3[M+H]⁺

### (6) Synthesis of N-[trans-4-(2-hydroxy-2-methylpropoxy)cyclohexyl]-4-(7-methyl-1H-pyrrolo[3,2-c]pyridin-4-yl)benzamide [4]

To a solution of the compound [4-1] (49 mg) in DMF (1.7 mL) were added COMU (80 mg), DIPEA (173 µL), and the compound [4-5] (35 mg) at room temperature, and the mixture was stirred at room temperature for 30 minutes. Further, to the reaction mixture was added the compound [4-5] (15 mg) at room temperature, and the mixture was stirred at room temperature for 19 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by reversed-phase silica gel column chromatography to give the title compound (12 mg) as a white solid.

¹H-NMR (400 MHz, DMSO-d₆) δ: 11.73 (br, 1H), 8.26 (d, J = 7.8 Hz, 1H), 8.10 (s, 1H), 8.04 (d, J = 8.2 Hz, 2H), 7.95 (d, J = 8.2 Hz, 2H), 7.56-7.54 (m, 1H), 6.79-6.78 (m, 1H), 4.21 (s, 1H), 3.82-3.77 (m, 1H), 3.24-3.17 (m, 3H), 2.49 (s, 3H), 2.03-2.00 (m, 2H), 1.89-1.86 (m, 2H), 1.45-1.35 (m, 2H), 1.29-1.21 (m, 2H), 1.06 (s, 6H).

ESI-MS: 422.6[M+H]⁺

### Example 5

### Synthesis of 3-fluoro-N-(trans-4-hydroxy-4-methylcyclohexyl)-4-(1H-pyrrolo[3,2-c]pyridin-4-yl)benzamide [5] (hereinafter, referred to as a compound [5])

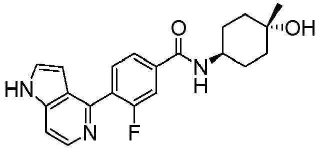

### (1) Synthesis of methyl 3-fluoro-4-(1H-pyrrolo[3,2-c]pyridin-4-yl)benzoate [5-1] (hereinafter, referred to as a compound [5-1])

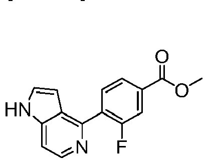

To a solution of 4-chloro-1H-pyrrolo[3,2-c]pyridine (153 mg) in methanol (6.6 mL)/water (3.3 mL) were added 2-fluoro-4-(methoxycarbonyl)phenylboronic acid (218 mg), potassium carbonate (152 mg), and [1,3-bis(2,6-diisopropylphenyl)-4,5-dihydroimidazol-2-ylidene]chloro[3-phenylallyl]palladium (II) (32.5 mg) at room temperature, and the mixture was stirred at 80°C for 2 hours under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (113 mg) as a white solid.

¹H-NMR (400 MHz, CDCl₃) δ: 8.60 (br, 1H), 8.48 (d, J = 6.0 Hz, 1H), 7.99-7.96 (m, 1H), 7.91-7.83 (m, 2H), 7.38 (d, J = 6.0 Hz, 1H), 7.31-7.30 (m, 1H), 6.60-6.59 (m, 1H), 3.97 (s, 3H).

ESI-MS: 271.3[M+H]⁺

### (2) Synthesis of 3-fluoro-4-(1H-pyrrolo[3,2-c]pyridin-4-yl)benzoic acid [5-2] (hereinafter, referred to as a compound [5-2])

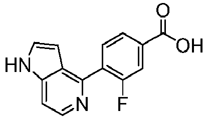

To a solution of the compound [5-1] (113 mg) in methanol (4.2 mL) was added a 2 M aqueous sodium hydroxide solution (2.1 mL) at room temperature, and the mixture was stirred at room temperature for 30 minutes. The reaction mixture was concentrated under reduced pressure and 2 M hydrochloric acid was added. The resulting solid was collected by filtration, washed with water, and then dried under reduced pressure to give the title compound (68.5 mg) as a white solid.

¹H-NMR (400 MHz, DMSO-d₆) δ: 8.45 (d, J = 6.4 Hz, 1H), 8.01-7.86 (m, 5H), 6.72 (s, 1H).

ESI-MS: 257.2[M+H]⁺

### (3) Synthesis of 3-fluoro-N-(trans-4-hydroxy-4-methylcyclohexyl)-4-(1H-pyrrolo[3,2-c]pyridin-4-yl)benzamide [5]

To a solution of the compound [5-2] (12 mg) in DMF (0.6 mL) were added DIPEA (32 µL) and COMU (22 mg) at room temperature, and the mixture was stirred at room temperature for 5 minutes. To the reaction mixture was added trans-4-amino-1-methylcyclohexanol hydrochloride (10 mg) at room temperature, and the mixture was stirred at room temperature for 1 hour. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (3.3 mg) as a white solid.

¹H-NMR (400 MHz, CD₃OD) δ: 8.25 (d, J = 5.9 Hz, 1H), 7.79-7.70 (m, 3H), 7.49 (d, J = 5.9 Hz, 1H), 7.43 (d, J = 3.2 Hz, 1H), 6.48-6.46 (m, 1H), 3.96-3.92 (m, 1H), 1.96-1.92 (m, 2H), 1.77-1.74 (m, 2H), 1.67-1.57 (m, 4H), 1.29 (s, 3H).

ESI-MS: 368.4[M+H]⁺

### Example 6

### Synthesis of 3-fluoro-N-(4-hydroxybicyclo[2.2.2]octan-1-yl)-4-(1H-pyrrolo[3,2-c]pyridin-4-yl)benzamide [6] (hereinafter, referred to as a compound [6])

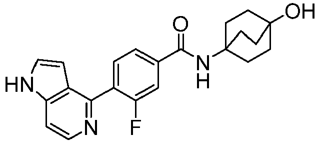

To a solution of the compound [5-2] (44 mg) in DMF (0.9 mL) were added DIPEA (70 µL) and COMU (81 mg) at room temperature, and the mixture was stirred at room temperature for 5 minutes. To the reaction mixture was added 4-aminobicyclo[2.2.2]octan-1-ol hydrochloride (40 mg) at room temperature, and the mixture was stirred at room temperature for 30 minutes. To the reaction mixture was added a saturated aqueous sodium bicarbonate solution, and the mixture was extracted with ethyl acetate. The obtained residue was purified by silica gel column chromatography to give the title compound (5.3 mg) as a white solid.

¹H-NMR (400 MHz, CD₃OD) δ: 8.24 (d, J = 5.9 Hz, 1H), 7.69-7.63 (m, 3H), 7.49 (d, J = 5.9 Hz, 1H), 7.43-7.41 (m, 1H), 6.46-6.45 (m, 1H), 2.21-2.17 (m, 6H), 1.81-1.77 (m, 6H) .

ESI-MS: 380.4[M+H]⁺

### Example 7

### Synthesis of N-(4-cyanobicyclo[2.2.2]octan-1-yl)-3-fluoro-4-(1H-pyrrolo[3,2-c]pyridin-4-yl)benzamide [7] (hereinafter, referred to as a compound [7])

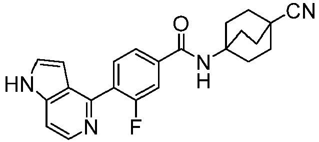

To a solution of the compound [5-2] (15 mg) in DMF (0.6 mL) were added DIPEA (20 µL) and COMU (25 mg) at room temperature, and the mixture was stirred at room temperature for 5 minutes. To the reaction mixture was added 4-aminobicyclo[2.2.2]octane-1-carbonitrile hydrochloride (11 mg) at room temperature, and the mixture was stirred at room temperature for 30 minutes. To the reaction mixture was added a saturated aqueous sodium bicarbonate solution, and the mixture was extracted with ethyl acetate. The obtained residue was purified by silica gel column chromatography to give the title compound (6.2 mg) as a white solid.

¹H-NMR (400 MHz, CD₃OD) δ: 8.24 (d, J = 5.5 Hz, 1H), 7.70-7.65 (m, 3H), 7.49-7.46 (m, 1H), 7.42 (d, J = 3.2 Hz, 1H), 6.45-6.44 (m, 1H), 2.15-2.09 (m, 12H).

ESI-MS: 389.4[M+H]⁺

### Example 8

### Synthesis of 4-[3-fluoro-4-(1H-pyrrolo[3,2-c]pyridin-4-yl)benzamide]bicyclo[2.2.2]octane-1-carboxamide [8] (hereinafter, referred to as a compound [8])

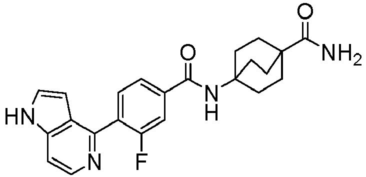

To a solution of the compound [7] (5.2 mg) in tert-butanol (0.3 mL) was added potassium tert-butoxide (18 mg) at room temperature, and the mixture was stirred at 80°C for 47 hours. The reaction mixture was concentrated under reduced pressure. The obtained residue was purified by silica gel column chromatography to give the title compound (2.6 mg) as a white solid.

¹H-NMR (400 MHz, CD₃OD) δ: 8.25-8.23 (m, 1H), 7.70-7.65 (m, 3H), 7.49-7.48 (m, 1H), 7.42 (d, J = 3.0 Hz, 1H), 6.46-6.45 (m, 1H), 2.12-2.10 (m, 6H), 1.94-1.91 (m, 6H).

ESI-MS: 407.4[M+H]⁺

### Example 9

### Synthesis of 3-fluoro-N-[trans-4-(2-hydroxy-2-methylpropoxy)cyclohexyl]-4-(1H-pyrrolo[3,2-c]pyridin-4-yl)benzamide [9] (hereinafter, referred to as a compound [9])

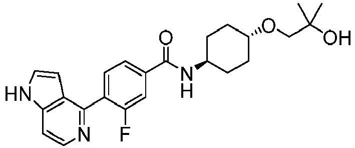

To a solution of the compound [5-2] (45 mg) in DMF (1.8 mL) were added COMU (75 mg), DIPEA (30 µL), and the compound [4-5] (33 mg) at room temperature, and the mixture was stirred at room temperature for 3 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (11 mg) as a yellow solid.

¹H-NMR (400 MHz, DMSO-d₆) δ: 11.68 (br, 1H), 8.37 (d, J = 7.8 Hz, 1H), 8.31-8.28 (m, 1H), 7.82-7.72 (m, 3H), 7.50-7.45 (m, 2H), 8.39-8.38 (m, 1H), 4.21 (s, 1H), 3.80-3.74 (m, 1H), 3.18-3.15 (m, 3H), 2.03-2.01 (m, 2H), 1.90-1.87 (m, 2H), 1.41-1.31 (m, 2H), 1.29-1.24 (m, 2H), 1.06 (s, 6H).

ESI-MS: 426.5[M+H]⁺

### Example 10

### Synthesis of 3-fluoro-N-(4-hydroxybicyclo[2.2.1]heptan-1-yl)-4-(1H-pyrrolo[3,2-c]pyridin-4-yl)benzamide [10] (hereinafter, referred to as a compound [10])

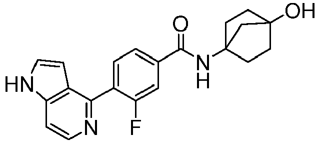

To a solution of the compound [5-2] (30 mg) in DMF (1.1 mL) were added COMU (50 mg), DIPEA (48 µL), and 4-aminobicyclo[2.2.1]heptan-1-ol hydrochloride (25 mg) at room temperature, and the mixture was stirred at room temperature for 21 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (19 mg) as a yellow solid.

¹H-NMR (400 MHz, CD₃OD) δ: 8.26-8.24 (m, 1H), 7.79-7.70 (m, 3H), 7.50-7.47 (m, 1H), 7.44-7.42 (m, 1H), 6.47 (d, J = 2.7 Hz, 1H), 2.13-2.03 (m, 6H), 1.88-1.82 (m, 2H), 1.74-1.69 (m, 2H).

ESI-MS: 366.4[M+H]⁺

### Example 11

### Synthesis of 3-fluoro-N-[trans-4-(1-hydroxycyclopropyl)cyclohexyl]-4-(1H-pyrrolo[3,2-c]pyridin-4-yl)benzamide [11] (hereinafter, referred to as a compound [11])

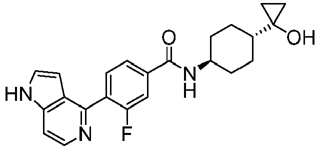

To a solution of the compound [5-2] (53 mg) in DMF (2.0 mL) were added DIPEA (36 µL) and COMU (90 mg) at room temperature, and the mixture was stirred at room temperature for 5 minutes. To the reaction mixture was added the compound [3-3] (33 mg) at room temperature, and the mixture was stirred at room temperature for 2 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (5.8 mg) as a white solid.

¹H-NMR (400 MHz, DMSO-d₆) δ: 11.69 (br, 1H), 8.38 (d, J = 8.2 Hz, 1H), 8.29 (d, J = 5.9 Hz, 1H), 7.83-7.72 (m, 3H), 7.51-7.50 (m, 1H), 7.45 (d, J = 5.5 Hz, 1H), 6.39-6.38 (m, 1H), 4.88 (s, 1H), 3.76-3.70 (m, 1H), 1.92-1.89 (m, 2H), 1.76-1.70 (m, 2H), 1.36-1.30 (m, 4H), 0.92-0.86 (m, 1H), 0.49-0.47 (m, 2H), 0.36-0.34 (m, 2H).

ESI-MS: 394.4[M+H]⁺

### Example 12

### Synthesis of 3-fluoro-N-(trans-4-hydroxycyclohexyl)-4-(1H-pyrrolo[3,2-c]pyridin-4-yl)benzamide [12] (hereinafter, referred to as a compound [12])

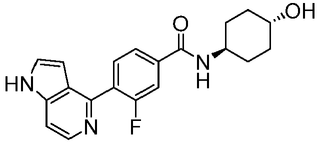

To a solution of the compound [5-2] (30 mg) in DMF (1.1 mL) were added DIPEA (20 µL) and COMU (50 mg) at room temperature, and the mixture was stirred at room temperature for 5 minutes. To the reaction mixture was added trans-4-aminocyclohexanol (18 mg) at room temperature, and the mixture was stirred at room temperature for 10 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (11 mg) as an orange solid.

¹H-NMR (400 MHz, CD₃OD) δ: 8.26-8.24 (m, 1H), 7.80-7.68 (m, 3H), 7.51 (d, J = 5.5 Hz, 1H), 7.44-7.43 (m, 1H), 6.49-6.48 (m, 1H), 3.88-3.85 (m, 1H), 3.60-3.52 (m, 1H), 2.03-2.00 (m, 4H), 1.52-1.35 (m, 4H).

ESI-MS: 354.4[M+H]⁺

### Example 13

### Synthesis of 4-(7-fluoro-1H-pyrrolo[3,2-c]pyridin-4-yl)-N-[trans-4-(2-hydroxypropan-2-yl)cyclohexyl]benzamide [13] (hereinafter, referred to as a compound [13])

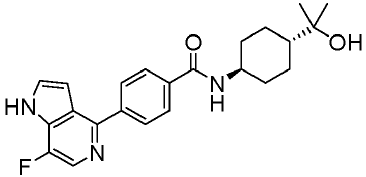

### (1) Synthesis of N-[trans-4-(2-hydroxypropan-2-yl)cyclohexyl]-4-(4,4,5,5-tetramethyl-1,3,2-dioxaborolan-2-yl)benzamide [13-1] (hereinafter, referred to as a compound [13-1])

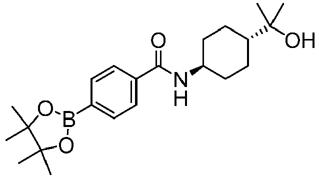

To a solution of 4-(4,4,5,5-tetramethyl-1,3,2-dioxaborolan-2-yl)benzoic acid (744 mg) in DMF (10 mL) were added DIPEA (561 µL) and COMU (1.41 g) at room temperature, and the mixture was stirred at room temperature for 5 minutes. To the reaction mixture was added 2-(trans-4-aminocyclohexyl)propan-2-ol (613 mg) at room temperature, and the mixture was stirred at room temperature for 4 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (977 mg) as a white solid.

¹H-NMR (400 MHz, CDCl₃) δ: 7.85 (d, J = 8.2 Hz, 2H), 7.72 (d, J = 7.8 Hz, 2H), 5.92 (d, J = 8.2 Hz, 1H), 4.01-3.84 (m, 1H), 2.27-2.09 (m, 2H), 2.03-1.84 (m, 2H), 1.45-1.11 (m, 23H).

ESI-MS: 388.5[M+H]⁺

### (2) Synthesis of 4-(7-fluoro-1H-pyrrolo[3,2-c]pyridin-4-yl)-N-[trans-4-(2-hydroxypropan-2-yl)cyclohexyl]benzamide [13]

To a solution of 4-bromo-7-fluoro-1H-pyrrolo[3,2-c]pyridine (22 mg) in 1,4-dioxane (0.90 mL)/water (0.10 mL) were added the compound [13-1] (50 mg), cesium carbonate (98 mg), and PdCl₂(Amphos)₂ (7.1 mg) at room temperature, and the mixture was stirred at 100°C for 2 hours under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (23 mg) as a white solid.

¹H-NMR (400 MHz, DMSO-d₆) δ: 12.35 (br, 1H), 8.29-8.27 (m, 2H), 8.02 (d, J = 8.4 Hz, 2H), 7.97 (d, J = 8.4 Hz, 2H), 7.65 (d, J = 3.2 Hz, 1H), 6.88-6.87 (m, 1H), 4.04 (s, 1H), 3.77-3.68 (m, 1H), 1.92-1.82 (m, 4H), 1.37-1.28 (m, 2H), 1.21-1.04 (m, 9H).

ESI-MS: 396.5[M+H]⁺

### Example 14

### Synthesis of 4-(7-chloro-1H-pyrrolo[3,2-c]pyridin-4-yl)-N-[trans-4-(2-hydroxypropan-2-yl)cyclohexyl]benzamide [14] (hereinafter, referred to as a compound [14])

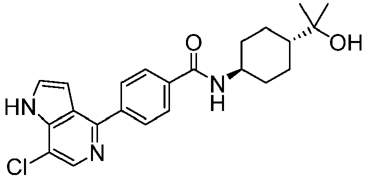

To a solution of 4-bromo-7-chloro-1H-pyrrolo[3,2-c]pyridine (23 mg) in 1,4-dioxane (0.90 mL)/water (0.10 mL) were added the compound [13-1] (50 mg), cesium carbonate (98 mg), and PdCl₂(Amphos)₂ (7.1 mg) at room temperature, and the mixture was stirred at 100°C for 45 minutes under an argon atmosphere. To the reaction mixture was added water, and the mixture was extracted with ethyl acetate. The organic layer was washed with a saturated aqueous sodium chloride solution and concentrated under reduced pressure. The obtained residue was purified by silica gel column chromatography to give the title compound (29 mg) as a white solid.

¹H-NMR (400 MHz, DMSO-d₆) δ: 12.23 (br, 1H), 8.34 (s, 1H), 8.29 (d, J = 7.6 Hz, 1H), 8.04 (d, J = 8.8 Hz, 2H), 7.98 (d, J = 8.8 Hz, 2H), 7.65 (d, J = 3.2 Hz, 1H), 6.89 (d, J = 3.2 Hz, 1H), 4.03 (s, 1H), 3.78-3.68 (m, 1H), 1.92-1.82 (m, 4H), 1.37-1.28 (m, 2H), 1.21-1.04 (m, 9H).

ESI-MS: 412.5[M+H]⁺

### Example 15

### Synthesis of 4-(7-cyano-1H-pyrrolo[3,2-c]pyridin-4-yl)-N-[trans-4-(2-hydroxypropan-2-yl)cyclohexyl]benzamide [15] (hereinafter, referred to as a compound [15])

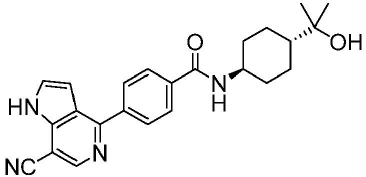

To a solution of the compound [14] (18 mg) in N,N-dimethylacetamide (2.2 mL) were added zinc cyanide (22 mg), tris(dibenzylideneacetone)dipalladium(0) (24 mg), and 2-dicyclohexylphosphino-2',4',6'-triisopropylbiphenyl (25 mg) at room temperature, and the mixture was stirred at 130°C for 30 minutes under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (14 mg) as a white solid.

¹H-NMR (400 MHz, DMSO-d₆) δ: 8.74 (s, 1H), 8.33 (d, J = 8.0 Hz, 1H), 8.11 (d, J = 8.4 Hz, 2H), 8.01 (d, J = 8.4 Hz, 2H), 7.73 (d, J = 3.2 Hz, 1H), 6.96 (d, J = 3.2 Hz, 1H), 4.04 (s, 1H), 3.78-3.68 (m, 1H), 1.92-1.82 (m, 4H), 1.37-1.28 (m, 2H), 1.21-1.04 (m, 9H).

ESI-MS: 403.5[M+H]⁺

### Example 16

### Synthesis of 4-(7-fluoro-1H-pyrrolo[3,2-c]pyridin-4-yl)-N-(4-hydroxybicyclo[2.2.2]octan-1-yl)benzamide [16] (hereinafter, referred to as a compound [16])

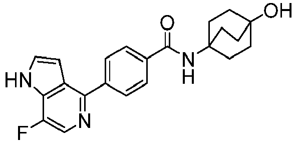

### (1) Synthesis of N-(4-hydroxybicyclo[2.2.2]octan-1-yl)-4-(4,4,5,5-tetramethyl-1,3,2-dioxaborolan-2-yl)benzamide [16-1] (hereinafter, referred to as a compound [16-1])

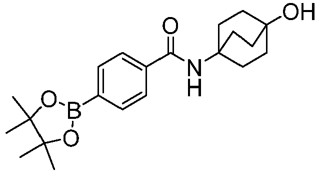

To a solution of 4-(4,4,5,5-tetramethyl-1,3,2-dioxaborolan-2-yl)benzoic acid (248 mg) in DMF (5.0 mL) were added DIPEA (374 µL) and COMU (471 mg) at room temperature, and the mixture was stirred at room temperature for 5 minutes. To the reaction mixture was added 4-aminobicyclo[2.2.2]octan-1-ol hydrochloride (178 mg) at room temperature, and the mixture was stirred at room temperature for 1 hour. To the reaction mixture was added DIPEA (374 µL) at room temperature, and the mixture was further stirred at room temperature for 40 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (87 mg) as a white solid.

¹H-NMR (400 MHz, CDCl₃) δ: 7.83 (d, J = 8.4 Hz, 2H), 7.66 (d, J = 8.4 Hz, 2H), 5.77 (br, 1H), 2.18-2.14 (m, 6H), 1.83-1.79 (m, 6H), 1.35 (s, 12H), 1.23 (s, 1H).

ESI-MS: 372.5[M+H]⁺

### (2) Synthesis of 4-(7-fluoro-1H-pyrrolo[3,2-c]pyridin-4-yl)-N-(4-hydroxybicyclo[2.2.2]octan-1-yl)benzamide [16]

To a solution of 4-bromo-7-fluoro-1H-pyrrolo[3,2-c]pyridine (11 mg) in 1,4-dioxane (0.90 mL)/water (0.10 mL) were added the compound [16-1] (24 mg), cesium carbonate (98 mg), and PdCl₂(Amphos)₂ (7.1 mg) at room temperature, and the mixture was stirred at 100°C for 3 hours under an argon atmosphere. To the reaction mixture was added water, and the mixture was extracted with ethyl acetate. The organic layer was washed with a saturated aqueous sodium chloride solution and concentrated under reduced pressure. The obtained residue was purified by silica gel column chromatography to give the title compound (2.7 mg) as a white solid.

¹H-NMR (400 MHz, DMSO-d₆) δ: 12.35 (br, 1H), 8.27 (d, J = 2.4 Hz, 1H), 7.98 (d, J = 8.8 Hz, 2H), 7.89 (d, J = 8.8 Hz, 2H), 7.70 (s, 1H), 7.66-7.64 (m, 1H), 6.87-6.84 (m, 1H), 4.30 (s, 1H), 2.07-2.03 (m, 6H), 1.64-1.60 (m, 6H).

ESI-MS: 380.5[M+H]⁺

### Example 17

### Synthesis of 4-(7-chloro-1H-pyrrolo[3,2-c]pyridin-4-yl)-N-(4-hydroxybicyclo[2.2.2]octan-1-yl)benzamide [17] (hereinafter, referred to as a compound [17])

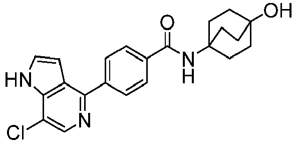

To a solution of 4-bromo-7-chloro-1H-pyrrolo[3,2-c]pyridine (12 mg) in 1,4-dioxane (0.90 mL)/water (0.10 mL) were added the compound [16-1] (24 mg), cesium carbonate (98 mg), and PdCl₂(Amphos)₂ (7.1 mg) at room temperature, and the mixture was stirred at 100°C for 4 hours under an argon atmosphere. To the reaction mixture was added water, and the mixture was extracted with ethyl acetate. The organic layer was washed with a saturated aqueous sodium chloride solution and concentrated under reduced pressure. The obtained residue was purified by silica gel column chromatography to give the title compound (7.0 mg) as a white solid.

¹H-NMR (400 MHz, DMSO-d₆) δ: 12.24 (br, 1H), 8.33 (s, 1H), 8.01 (d, J = 8.8 Hz, 2H), 7.90 (d, J = 8.8 Hz, 2H), 7.71 (s, 1H), 7.65 (d, J = 3.2 Hz, 1H), 6.87 (d, J = 3.2 Hz, 1H), 4.30 (s, 1H), 2.07-2.03 (m, 6H), 1.64-1.60 (m, 6H) .

ESI-MS: 396.5[M+H]⁺

### Example 18

### Synthesis of 4-[7-(difluoromethyl)-1H-pyrrolo[3,2-c]pyridin-4-yl]-N-[trans-4-(2-hydroxypropan-2-yl)cyclohexyl]benzamide [18] (hereinafter, referred to as a compound [18])

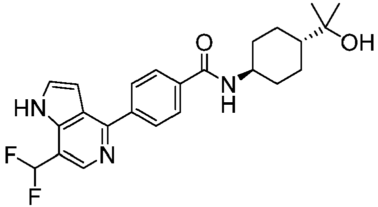

### (1) Synthesis of 4-bromo-7-chloro-1-(4-methoxybenzyl)-1H-pyrrolo[3,2-c]pyridine [18-1] (hereinafter, referred to as a compound [18-1])

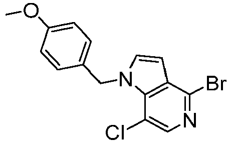

To a solution of 4-bromo-7-chloro-1H-pyrrolo[3,2-c]pyridine (116 mg) in DMF (5.0 mL) were added 4-methoxybenzyl chloride (81.7 µL) and cesium carbonate (326 mg) at room temperature, and the mixture was stirred at room temperature for 2 hours. To the reaction mixture was added 4-methoxybenzyl chloride (20.4 µL) at room temperature, and the mixture was further stirred at room temperature for 24 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (177 mg) as a colorless oil.

ESI-MS: 351.3[M+H]⁺

### (2) Synthesis of ethyl 4-[7-chloro-1-(4-methoxybenzyl)-1H-pyrrolo[3,2-c]pyridin-4-yl]benzoate [18-2] (hereinafter, referred to as a compound [18-2])

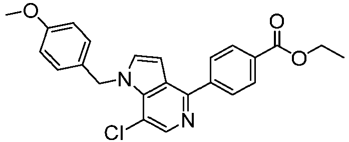

To a solution of the compound [18-1] (173 mg) in 1,2-dimethoxyethane (4.9 mL) were added 4-(ethoxycarbonyl)phenylboronic acid (124 mg), cesium fluoride (150 mg), and PdCl₂(dppf)·CH₂Cl₂ (40.3 mg) at room temperature, and the mixture was stirred at 100°C for 3 hours under an argon atmosphere. To the reaction mixture were added 4-(ethoxycarbonyl)phenylboronic acid (19.1 mg), cesium fluoride (22.5 mg), PdCl₂(dppf)·CH₂Cl₂ (40.3 mg), and 1,2-dimethoxyethane (4.9 mL) at room temperature, and the mixture was further stirred at 100°C for 15 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (126 mg) as a colorless oil.

ESI-MS: 421.4[M+H]⁺

### (3) Synthesis of ethyl 4-[1-(4-methoxybenzyl)-7-vinyl-1H-pyrrolo[3,2-c]pyridin-4-yl]benzoate [18-3] (hereinafter, referred to as a compound [18-3])

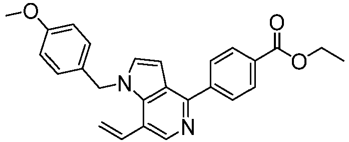

To a solution of the compound [18-2] (95.6 mg) in 1-propanol (4.5 mL) were added potassium vinyl trifluoroborate (122 mg), PdCl₂(Amphos)₂ (32.2 mg), and triethylamine (127 µL) at room temperature, and the mixture was stirred at 110°C for 30 minutes under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (54.9 mg) as a yellow solid.

ESI-MS: 413.5[M+H]⁺

### (4) Synthesis of ethyl 4-[7-formyl-1-(4-methoxybenzyl)-1H-pyrrolo[3,2-c]pyridin-4-yl]benzoate [18-4] (hereinafter, referred to as a compound [18-4])

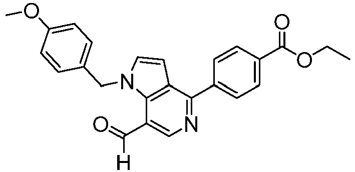

To a solution of the compound [18-3] (54.9 mg) in acetone (5.3 mL)/water (1.3 mL) were added 2% aqueous osmium tetroxide solution (1.02 mL) and sodium periodate (285 mg) at room temperature, and the mixture was stirred at room temperature for 5 hours. To the reaction mixture were added a saturated aqueous sodium bicarbonate solution and a saturated aqueous sodium thiosulfate solution, and the mixture was extracted with ethyl acetate. The organic layer was dried over anhydrous sodium sulfate and concentrated under reduced pressure. The obtained residue was purified by silica gel column chromatography to give the title compound (32.9 mg) as a yellow solid.

ESI-MS: 415.5[M+H]⁺

### (5) Synthesis of ethyl 4-[7-(difluoromethyl)-1-(4-methoxybenzyl)-1H-pyrrolo[3,2-c]pyridin-4-yl]benzoate [18-5] (hereinafter, referred to as a compound [18-5])

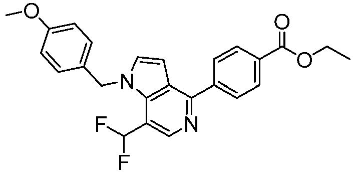

To a solution of the compound [18-4] (31.6 mg) in dichloromethane (1.9 mL) was added diethylaminosulfur trifluoride (50 µL) at room temperature, and the mixture was stirred at room temperature for 20 minutes. To the reaction mixture was added diethylaminosulfur trifluoride (100 µL) at room temperature, and the mixture was further stirred at room temperature for 1.5 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (30.9 mg) as a colorless oil.

ESI-MS: 437.5[M+H]⁺

### (6) Synthesis of ethyl 4-[7-(difluoromethyl)-1H-pyrrolo[3,2-c]pyridin-4-yl]benzoate [18-6] (hereinafter, referred to as a compound [18-6])

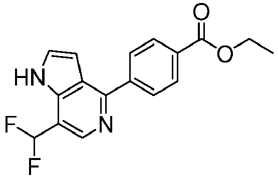

To a solution of the compound [18-5] (28.2 mg) in acetonitrile (2.56 mL)/water (0.64 mL) was added cerium (IV) ammonium nitrate (177 mg) at room temperature, and the mixture was stirred at room temperature for 2.5 hours. To the reaction mixture were added a saturated aqueous sodium bicarbonate solution and a saturated aqueous sodium thiosulfate solution, and the mixture was extracted with ethyl acetate. The organic layer was dried over anhydrous sodium sulfate and concentrated under reduced pressure. The obtained residue was purified by silica gel column chromatography to give the title compound (15.8 mg) as a pale pink solid.

ESI-MS: 317.4[M+H]⁺

### (7) Synthesis of 4-[7-(difluoromethyl)-1H-pyrrolo[3,2-c]pyridin-4-yl] benzoic acid [18-7] (hereinafter, referred to as a compound [18-7])

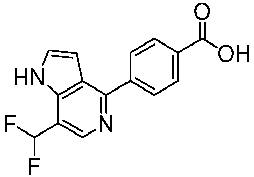

To a solution of the compound [18-6] (15.2 mg) in methanol (2.4 mL) was added a 2 M aqueous sodium hydroxide solution (1.2 mL) at room temperature, and the mixture was stirred at room temperature for 30 minutes. The reaction mixture was concentrated under reduced pressure and 2 M hydrochloric acid was added. The resulting solid was collected by filtration, washed with water, and then dried under reduced pressure to give the title compound (11.2 mg) as a white solid.

ESI-MS: 289.4[M+H]⁺

### (8) Synthesis of 4-[7-(difluoromethyl)-1H-pyrrolo[3,2-c]pyridin-4-yl]-N-[trans-4-(2-hydroxypropan-2-yl)cyclohexyl]benzamide [18]

To a solution of the compound [18-7] (5.8 mg) in DMF (2.0 mL) were added DIPEA (6.8 µL), COMU (17 mg), and 2-(trans-4-aminocyclohexyl)propan-2-ol (7.9 mg) at room temperature, and the mixture was stirred at room temperature for 30 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (6.8 mg) as a white solid.

¹H-NMR (400 MHz, DMSO-d₆) δ: 12.06 (br, 1H), 8.48 (s, 1H), 8.31 (d, J = 8.0 Hz, 1H), 8.07 (d, J = 8.8 Hz, 2H), 8.00 (d, J = 8.8 Hz, 2H), 7.62 (d, J = 3.2 Hz, 1H), 7.42 (t, J = 54.4 Hz, 1H), 6.89 (d, J = 3.2 Hz, 1H), 4.04 (s, 1H), 3.77-3.69 (m, 1H), 1.92-1.82 (m, 4H), 1.37-1.28 (m, 2H), 1.22-1.04 (m, 9H).

ESI-MS: 428.5[M+H]⁺

### Example 19

### Synthesis of 4-(7-fluoro-1H-pyrrolo[3,2-c]pyridin-4-yl)-N-(4-hydroxybicyclo[2.2.1]heptan-1-yl)benzamide [19] (hereinafter, referred to as a compound [19])

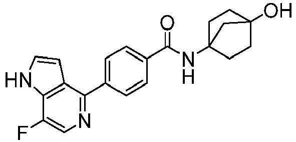

### (1) Synthesis of ethyl 4-(7-fluoro-1H-pyrrolo[3,2-c]pyridin-4-yl)benzoate [19-1] (hereinafter, referred to as a compound [19-1])

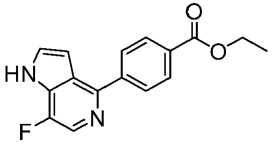

To a solution of 4-bromo-7-fluoro-1H-pyrrolo[3,2-c]pyridine (205 mg) in ethanol (6.4 mL)/water (3.2 mL) were added 4-(ethoxycarbonyl)phenylboronic acid (204 mg), potassium carbonate (171 mg), and [1,3-bis(2,6-diisopropylphenyl)-4,5-dihydroimidazol-2-ylidene]chloro[3-phenylallyl]palladium (II) (31.2 mg) at room temperature, and the mixture was stirred at 80°C for 1 hour under an argon atmosphere. Further, to the reaction mixture were added 4-(ethoxycarbonyl)phenylboronic acid (200 mg), potassium carbonate (342 mg), and [1,3-bis(2,6-diisopropylphenyl)-4,5-dihydroimidazol-2-ylidene]chloro[3-phenylallyl]palladium (II) (32 mg) at 80°C, and the mixture was stirred at 80°C for 1 hour. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (240 mg) as a white solid.

¹H-NMR (400 MHz, DMSO-d₆) δ: 12.40 (br, 1H), 8.29 (d, J = 2.3 Hz, 1H), 8.13-8.08 (m, 4H), 7.68 (d, J = 3.2 Hz, 1H), 6.92-6.90 (m, 1H), 4.35 (q, J = 7.0 Hz, 2H), 1.35 (t, J = 7.0 Hz, 3H).

ESI-MS: 285.3[M+H]⁺

### (2) Synthesis of 4-(7-fluoro-1H-pyrrolo[3,2-c]pyridin-4-yl)benzoic acid [19-2] (hereinafter, referred to as a compound [19-2])

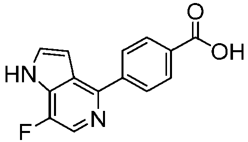

To a solution of the compound [19-1] (240 mg) in ethanol (2.1 mL) was added a 2 M aqueous sodium hydroxide solution (2.1 mL) at room temperature, and the mixture was stirred at 70°C for 10 minutes. The reaction mixture was concentrated under reduced pressure and 2 M hydrochloric acid (2.1 mL) was added. The resulting solid was collected by filtration, washed with water, and then dried under reduced pressure to give the title compound (210 mg) as a yellow solid.

¹H-NMR (400 MHz, DMSO-d₆) δ: 7.91-7.86 (m, 4H), 7.59 (d, J = 4.1 Hz, 1H), 7.36 (d, J = 2.3 Hz, 1H), 6.54-6.52 (m, 1H).

ESI-MS: 257.3[M+H]⁺

### (3) Synthesis of 4-(7-fluoro-1H-pyrrolo[3,2-c]pyridin-4-yl)-N-(4-hydroxybicyclo[2.2.1]heptan-1-yl)benzamide [19]

To a solution of the compound [19-2] (30 mg) in DMF (1.0 mL) were added COMU (55 mg), DIPEA (51 µL), and 4-aminobicyclo[2.2.1]heptan-1-ol hydrochloride (27 mg) at room temperature, and the mixture was stirred at room temperature for 30 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by preparative thin layer chromatography to give the title compound (3.7 mg) as a yellow solid.

¹H-NMR (400 MHz, CD₃OD) δ: 8.14 (d, J = 3.2 Hz, 1H), 7.93-7.92 (m, 4H), 7.52 (d, J = 3.2 Hz, 1H), 6.84-6.82 (m, 1H), 2.15-2.05 (m, 6H), 1.90-1.80 (m, 2H), 1.75-1.70 (m, 2H) .

ESI-MS: 366.4[M+H]⁺

### Example 20

### Synthesis of 4-(7-fluoro-1H-pyrrolo[3,2-c]pyridin-4-yl)-N-(trans-4-hydroxycyclohexyl)benzamide [20] (hereinafter, referred to as a compound [20])

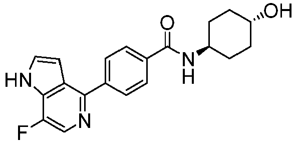

To a solution of the compound [19-2] (30 mg) in DMF (1.0 mL) were added DIPEA (20 µL) and COMU (50 mg) at room temperature, and the mixture was stirred at room temperature for 5 minutes. To the reaction mixture was added trans-4-aminocyclohexanol (18 mg) at room temperature, and the mixture was stirred at room temperature for 2 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by preparative thin layer chromatography to give the title compound (8.2 mg) as a yellow solid.

¹H-NMR (400 MHz, DMSO-d₆) δ: 12.38 (br, 1H), 8.27-8.26 (m, 2H), 8.01 (d, J = 7.3 Hz, 2H), 7.96 (d, J = 8.7 Hz, 2H), 7.66-7.65 (m, 1H), 6.88-6.87 (m, 1H), 4.55 (d, J = 4.1 Hz, 1H), 3.79-3.68 (m, 1H), 3.42-3.36 (m, 1H), 1.87-1.81 (m, 4H), 1.40-1.32 (m, 2H), 1.30-1.20 (m, 2H).

ESI-MS: 354.4[M+H]⁺

### Example 21

### Synthesis of 4-(7-fluoro-1H-pyrrolo[3,2-c]pyridin-4-yl)-N-(trans-4-hydroxy-4-methylcyclohexyl)benzamide [21] (hereinafter, referred to as a compound [21])

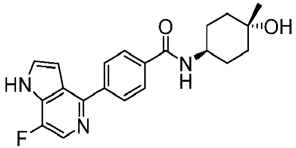

To a solution of the compound [19-2] (30 mg) in DMF (1.0 mL) were added DIPEA (20 µL) and COMU (50 mg) at room temperature, and the mixture was stirred at room temperature for 5 minutes. To the reaction mixture was added trans-4-amino-1-methylcyclohexanol hydrochloride (20 mg) at room temperature, and the mixture was stirred at room temperature for 21 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by preparative thin layer chromatography to give the title compound (14 mg) as a yellow solid.

¹H-NMR (400 MHz, DMSO-d₆) δ: 12.38 (br, 1H), 8.26-8.23 (m, 2H), 8.02 (d, J = 7.3 Hz, 2H), 7.96 (d, J = 8.2 Hz, 2H), 7.65 (d, J = 3.2 Hz, 1H), 6.87-6.86 (m, 1H), 4.28 (s, 1H), 3.86-3.80 (m, 1H), 1.78-1.75 (m, 2H), 1.61-1.59 (m, 2H), 1.48-1.41 (m, 4H), 1.16 (s, 3H).

ESI-MS: 368.4[M+H]⁺

### Example 22

### Synthesis of 4-(7-fluoro-1H-pyrrolo[3,2-c]pyridin-4-yl)-N-[trans-4-(2-hydroxy-2-methylpropoxy)cyclohexyl]benzamide [22] (hereinafter, referred to as a compound [22])

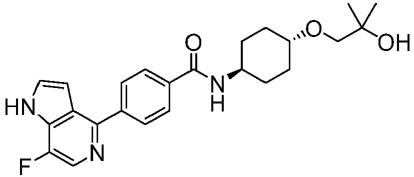

To a solution of the compound [19-2] (21 mg) in DMF (1.0 mL) were added COMU (35 mg), DIPEA (18 µL), and the compound [4-5] (20 mg) at room temperature, and the mixture was stirred at room temperature for 17 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by preparative thin layer chromatography to give the title compound (5.6 mg) as a yellow solid.

¹H-NMR (400 MHz, DMSO-d₆) δ: 12.37 (br, 1H), 8.30-8.27 (m, 2H), 8.02 (d, J = 7.8 Hz, 2H), 7.98-7.95 (m, 2H), 7.66 (d, J = 2.7 Hz, 1H), 6.88-6.87 (m, 1H), 4.22 (s, 1H), 3.82-3.72 (m, 1H), 3.19-3.17 (m, 3H), 2.03-1.99 (m, 2H), 1.89-1.86 (m, 2H), 1.42-1.33 (m, 2H), 1.28-1.22 (m, 2H), 1.06 (s, 6H).

ESI-MS: 426.4[M+H]⁺

### Example 23

### Synthesis of 4-(7-fluoro-1H-pyrrolo[3,2-c]pyridin-4-yl)-N-(cis-4-hydroxycyclohexyl)benzamide [23] (hereinafter, referred to as a compound [23])

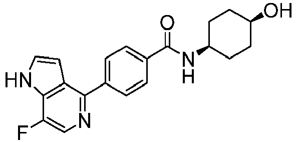

To a solution of the compound [19-2] (30 mg) in DMF (1.0 mL) were added DIPEA (48 µL) and COMU (50 mg) at room temperature, and the mixture was stirred at room temperature for 5 minutes. To the reaction mixture was added cis-4-aminocyclohexanol hydrochloride (23 mg) at room temperature, and the mixture was stirred at room temperature for 20 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (12 mg) as a white solid.

¹H-NMR (400 MHz, DMSO-d₆) δ: 12.35 (br, 1H), 8.30-8.27 (m, 2H), 8.00-7.99 (m, 4H), 7.66-7.65 (m, 1H), 6.88-6.87 (m, 1H), 4.36 (s, 1H), 3.83-3.78 (m, 2H), 1.80-1.67 (m, 4H), 1.56-1.47 (m, 4H).

ESI-MS: 354.4[M+H]⁺

### Example 24

### Synthesis of 4-(7-fluoro-1H-pyrrolo[3,2-c]pyridin-4-yl)-N-(tetrahydropyran-4-yl)benzamide [24] (hereinafter, referred to as a compound [24])

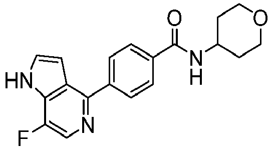

To a solution of the compound [19-2] (30 mg) in DMF (1.0 mL) were added DIPEA (22 µL) and COMU (50 mg) at room temperature, and the mixture was stirred at room temperature for 5 minutes. To the reaction mixture was added oxan-4-amine (16 µL) at room temperature, and the mixture was stirred at room temperature for 18 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (13 mg) as a yellow solid.

¹H-NMR (400 MHz, DMSO-d₆) δ: 12.37 (br, 1H), 8.41-8.38 (m, 1H), 8.28-8.25 (m, 1H), 8.03-7.95 (m, 4H), 7.65 (d, J = 7.5 Hz, 1H), 6.89-6.87 (m, 1H), 4.07-4.02 (m, 1H), 3.89-3.83 (m, 2H), 3.42-3.37 (m, 2H), 1.79-1.74 (m, 2H), 1.63-1.52 (m, 2H).

ESI-MS: 340.3[M+H]⁺

### Example 25

### Synthesis of N-(1,1-dioxotetrahydrothiopyran-4-yl)-4-(7-fluoro-1H-pyrrolo[3,2-c]pyridin-4-yl)benzamide [25] (hereinafter, referred to as a compound [25])

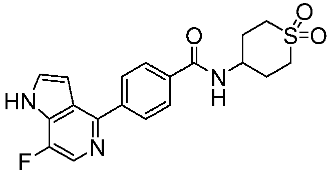

To a solution of the compound [19-2] (20 mg) in DMF (1.0 mL) were added DIPEA (15 µL) and COMU (33 mg) at room temperature, and the mixture was stirred at room temperature for 5 minutes. To the reaction mixture was added 1,1-dioxothian-4-amine (15 mg) at room temperature, and the mixture was stirred at room temperature for 17 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (7.6 mg) as a white solid.

¹H-NMR (400 MHz, DMSO-d₆) δ: 12.41 (br, 1H), 8.52 (d, J = 7.8 Hz, 1H), 8.31-8.30 (m, 1H), 8.05-7.99 (m, 4H), 7.68-7.67 (m, 1H), 6.89-6.88 (m, 1H), 4.27-4.18 (m, 1H), 3.32-3.30 (m, 2H), 3.15-3.10 (m, 2H), 2.14-2.09 (m, 4H).

ESI-MS: 388.3[M+H]⁺

### Example 26

### Synthesis of N-(trans-4-cyanocyclohexyl)-4-(7-fluoro-1H-pyrrolo[3,2-c]pyridin-4-yl)benzamide [26] (hereinafter, referred to as a compound [26])

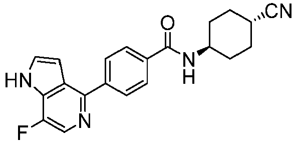

To a solution of the compound [19-2] (30 mg) in DMF (1.0 mL) were added DIPEA (44 µL) and COMU (50 mg) at room temperature, and the mixture was stirred at room temperature for 5 minutes. To the reaction mixture was added trans-4-cyanocyclohexylamine hydrochloride (21 mg) at room temperature, and the mixture was stirred at room temperature for 19 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (14.6 mg) as a yellow solid.

¹H-NMR (400 MHz, DMSO-d₆) δ: 12.38 (br, 1H), 8.37-8.27 (m, 2H), 8.03-7.95 (m, 4H), 7.66 (d, J = 3.2 Hz, 1H), 6.87 (d, J = 3.2 Hz, 1H), 3.85-3.78 (m, 1H), 2.70-2.64 (m, 1H), 2.08-2.05 (m, 2H), 1.89-1.86 (m, 2H), 1.65-1.57 (m, 2H), 1.43-1.31 (m, 2H).

ESI-MS: 363.4[M+H]⁺

### Example 27

### Synthesis of N-(trans-4-carbamoylcyclohexyl)-4-(7-fluoro-1H-pyrrolo[3,2-c]pyridin-4-yl)benzamide [27] (hereinafter, referred to as a compound [27])

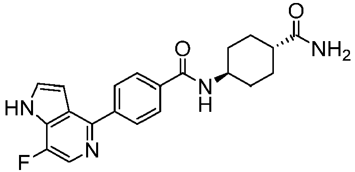

To a solution of the compound [26] (10 mg) in tert-butanol (0.9 mL) was added potassium tert-butoxide (37 mg) at room temperature, and the mixture was stirred at 80°C for 18 hours. The reaction mixture was concentrated under reduced pressure. The obtained residue was purified by silica gel column chromatography to give the title compound (4.8 mg) as a white solid.

¹H-NMR (400 MHz, CD₃OD) δ: 8.14-8.13 (m, 1H), 7.98-7.93 (m, 4H), 7.52-7.51 (m, 1H), 6.84 (d, J = 3.7 Hz, 1H), 3.97-3.88 (m, 1H), 2.25-2.21 (m, 1H), 2.12-2.08 (m, 2H), 1.98-1.95 (m, 2H), 1.68-1.60 (m, 2H), 1.50-1.43 (m, 2H).

ESI-MS: 381.5[M+H]⁺

### Example 28

### Synthesis of 4-(7-chloro-1H-pyrrolo[3,2-c]pyridin-4-yl)-N-(tetrahydropyran-4-yl)benzamide [28] (hereinafter, referred to as a compound [28])

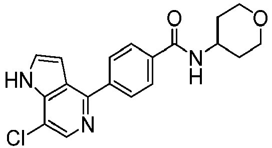

### (1) Synthesis of methyl 4-(7-chloro-1H-pyrrolo[3,2-c]pyridin-4-yl)benzoate [28-1] (hereinafter, referred to as a compound [28-1])

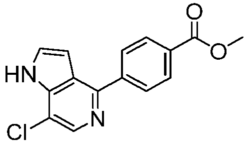

To a solution of 4-bromo-7-chloro-1H-pyrrolo[3,2-c]pyridine (232 mg) in 1,4-dioxane (9.0 mL)/water (1.0 mL) were added 4-(methoxycarbonyl)phenylboronic acid (198 mg), cesium carbonate (424 mg), and PdCl₂(dppf)·CH₂Cl₂ (81.7 mg) at room temperature, and the mixture was stirred at 100°C for 3 hours under an argon atmosphere. To the reaction mixture was added water, and the mixture was extracted with ethyl acetate. The organic layer was washed with a saturated aqueous sodium chloride solution and concentrated under reduced pressure. The obtained residue was purified by silica gel column chromatography to give the title compound (224 mg) as a white solid.

¹H-NMR (400 MHz, DMSO-d₆) δ: 12.28 (br, 1H), 8.36 (s, 1H), 8.16-8.10 (m, 4H), 7.68 (d, J = 3.2 Hz, 1H), 6.93 (d, J = 3.2 Hz, 1H), 3.89 (s, 3H).

ESI-MS: 287.3[M+H]⁺

### (2) Synthesis of 4-(7-chloro-1H-pyrrolo[3,2-c]pyridin-4-yl)benzoic acid [28-2] (hereinafter, referred to as a compound [28-2])

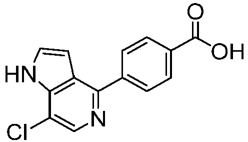

To a solution of the compound [28-1] (223 mg) in methanol (7.8 mL) was added a 2 M aqueous sodium hydroxide solution (3.9 mL) at room temperature, and the mixture was stirred at 60°C for 1 hour. The reaction mixture was concentrated under reduced pressure and 2 M hydrochloric acid was added. The resulting solid was collected by filtration, washed with water, and then dried under reduced pressure to give the title compound (211 mg) as a yellow white solid.

¹H-NMR (400 MHz, CD₃OD) δ: 8.33 (s, 1H), 8.22 (d, J = 8.0 Hz, 2H), 8.01 (d, J = 8.0 Hz, 2H), 7.63 (d, J = 3.2 Hz, 1H), 6.94 (d, J = 3.2 Hz, 1H).

ESI-MS: 273.2[M+H]⁺

### (3) Synthesis of 4-(7-chloro-1H-pyrrolo[3,2-c]pyridin-4-yl)-N-(tetrahydro-2H-pyran-4-yl)benzamide [28]

To a solution of the compound [28-2] (20 mg) in DMF (3.6 mL) were added DIPEA (25 µL), COMU (63 mg), and oxan-4-amine (19 µL) at room temperature, and the mixture was stirred at room temperature for 30 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (21 mg) as a white solid.

¹H-NMR (400 MHz, DMSO-d₆) δ: 12.25 (br, 1H), 8.42 (d, J = 7.6 Hz, 1H), 8.34 (s, 1H), 8.06 (d, J = 8.4 Hz, 2H), 8.00 (d, J = 8.4 Hz, 2H), 7.66 (d, J = 3.2 Hz, 1H), 6.90 (d, J = 3.2 Hz, 1H), 4.08-3.98 (m, 1H), 3.90-3.87 (m, 2H), 3.42-3.36 (m, 2H), 1.79-1.76 (m, 2H), 1.65-1.55 (m, 2H).

ESI-MS: 356.3[M+H]⁺

### Example 29

### Synthesis of 4-(7-chloro-1H-pyrrolo[3,2-c]pyridin-4-yl)-N-(1,1-dioxotetrahydrothiopyran-4-yl)benzamide [29] (hereinafter, referred to as a compound [29])

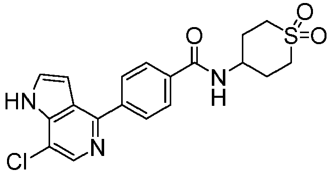

To a solution of the compound [28-2] (20 mg) in DMF (3.6 mL) were added DIPEA (25 µL) and COMU (63 mg) at room temperature, and the mixture was stirred at room temperature for 5 minutes. To the reaction mixture was added 1,1-dioxothian-4-amine (27 mg) at room temperature, and the mixture was stirred at room temperature for 4 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (20 mg) as a white solid.

¹H-NMR (400 MHz, DMSO-d₆) δ: 12.27 (br, 1H), 8.55 (d, J = 7.6 Hz, 1H), 8.34 (s, 1H), 8.07 (d, J = 8.8 Hz, 2H), 8.01 (d, J = 8.8 Hz, 2H), 7.66 (d, J = 3.2 Hz, 1H), 6.90 (d, J = 3.2 Hz, 1H), 4.29-4.20 (m, 1H), 3.32-3.28 (m, 2H), 3.16-3.12 (m, 2H), 2.17-2.06 (m, 4H).

ESI-MS: 404.3[M+H]⁺

### Example 30

### Synthesis of 4-(7-chloro-1H-pyrrolo[3,2-c]pyridin-4-yl)-N-(trans-4-hydroxycyclohexyl)benzamide [30] (hereinafter, referred to as a compound [30])

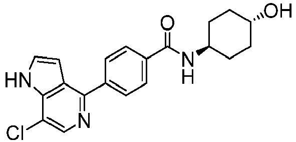

To a solution of the compound [28-2] (20 mg) in DMF (3.6 mL) were added DIPEA (25 µL) and COMU (63 mg) at room temperature, and the mixture was stirred at room temperature for 5 minutes. To the reaction mixture was added trans-4-aminocyclohexanol (21 mg) at room temperature, and the mixture was stirred at room temperature for 5 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (16 mg) as a white solid.

¹H-NMR (400 MHz, DMSO-d₆) δ: 12.24 (br, 1H), 8.34 (s, 1H), 8.28 (d, J = 7.6 Hz, 1H), 8.04 (d, J = 8.8 Hz, 2H), 7.98 (d, J = 8.8 Hz, 2H), 7.65 (d, J = 3.2 Hz, 1H), 6.89 (d, J = 3.2 Hz, 1H), 4.56 (d, J = 4.4 Hz, 1H), 3.79-3.70 (m, 1H), 3.43-3.36 (m, 1H), 1.87-1.82 (m, 4H), 1.44-1.34 (m, 2H), 1.30-1.21 (m, 2H).

ESI-MS: 370.3[M+H]⁺

### Example 31

### Synthesis of 4-(7-chloro-1H-pyrrolo[3,2-c]pyridin-4-yl)-N-(cis-4-hydroxy-4-methylcyclohexyl)benzamide [31] (hereinafter, referred to as a compound [31])

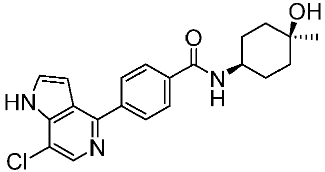

To a solution of the compound [28-2] (20 mg) in DMF (3.6 mL) were added DIPEA (25 µL) and COMU (63 mg) at room temperature, and the mixture was stirred at room temperature for 5 minutes. To the reaction mixture was added cis-4-amino-1-methylcyclohexanol (24 mg) at room temperature, and the mixture was stirred at room temperature for 6 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (17 mg) as a white solid.

¹H-NMR (400 MHz, DMSO-d₆) δ: 12.23 (br, 1H), 8.34 (s, 1H), 8.32 (d, J = 8.4 Hz, 1H), 8.05-7.99 (m, 4H), 7.65 (d, J = 3.2 Hz, 1H), 6.90 (d, J = 2.8 Hz, 1H), 4.04 (s, 1H), 3.79-3.70 (m, 1H), 1.80-1.71 (m, 2H), 1.59-1.57 (m, 4H), 1.40-1.32 (m, 2H), 1.12 (s, 3H).

ESI-MS: 384.4[M+H]⁺

### Example 32

### Synthesis of 4-(7-chloro-1H-pyrrolo[3,2-c]pyridin-4-yl)-N-(4-hydroxybicyclo[2.2.1]heptan-1-yl)benzamide [32] (hereinafter, referred to as a compound [32])

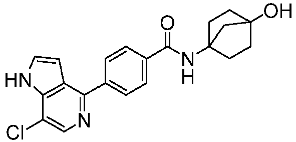

To a solution of the compound [28-2] (20 mg) in DMF (3.6 mL) were added DIPEA (125 µL) and COMU (63 mg) at room temperature, and the mixture was stirred at room temperature for 5 minutes. To the reaction mixture was added 4-aminobicyclo[2.2.1]heptan-1-ol hydrochloride (30 mg) at room temperature, and the mixture was stirred at room temperature for 30 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (15 mg) as a white solid.

¹H-NMR (400 MHz, DMSO-d₆) δ: 12.23 (br, 1H), 8.40 (s, 1H), 8.33 (s, 1H), 8.03 (d, J = 8.8 Hz, 2H), 7.97 (d, J = 8.8 Hz, 2H), 7.65 (d, J = 3.2 Hz, 1H), 6.89 (d, J = 2.8 Hz, 1H), 4.92 (s, 1H), 2.04-1.97 (m, 2H), 1.92-1.85 (m, 4H), 1.73-1.66 (m, 2H), 1.58-1.52 (m, 2H).

ESI-MS: 382.4[M+H]⁺

### Example 33

### Synthesis of 4-(7-chloro-1H-pyrrolo[3,2-c]pyridin-4-yl)-N-(cis-4-hydroxycyclohexyl)benzamide [33] (hereinafter, referred to as a compound [33])

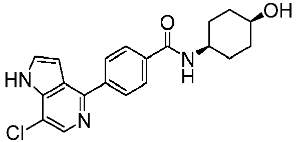

To a solution of the compound [28-2] (20 mg) in DMF (3.6 mL) were added DIPEA (125 µL) and COMU (63 mg) at room temperature, and the mixture was stirred at room temperature for 5 minutes. To the reaction mixture was added cis-4-aminocyclohexanol hydrochloride (28 mg) at room temperature, and the mixture was stirred at room temperature for 30 minutes. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (13 mg) as a white solid.

¹H-NMR (400 MHz, DMSO-d₆) δ: 12.23 (br, 1H), 8.34 (s, 1H), 8.30 (d, J = 7.2 Hz, 1H), 8.05-7.99 (m, 4H), 7.66-7.65 (m, 1H), 6.90-6.89 (m, 1H), 4.37 (d, J = 2.8 Hz, 1H), 3.85-3.77 (m, 2H), 1.83-1.67 (m, 4H), 1.57-1.46 (m, 4H).

ESI-MS: 370.4[M+H]⁺

### Example 34

### Synthesis of 4-(7-chloro-1H-pyrrolo[3,2-c]pyridin-4-yl)-N-(trans-4-hydroxy-4-methylcyclohexyl)benzamide [34] (hereinafter, referred to as a compound [34])

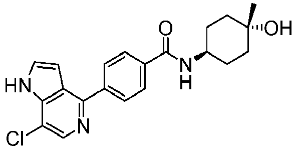

To a solution of the compound [28-2] (20 mg) in DMF (3.6 mL) were added DIPEA (25 µL) and COMU (63 mg) at room temperature, and the mixture was stirred at room temperature for 5 minutes. To the reaction mixture was added trans-4-amino-1-methylcyclohexanol hydrochloride (24 mg) at room temperature, and the mixture was stirred at room temperature for 15 minutes. To the reaction mixture was added DIPEA (100 µL) at room temperature, and the mixture was further stirred at room temperature for 15 minutes. To the reaction mixture was added a saturated aqueous sodium bicarbonate solution, and the mixture was extracted with ethyl acetate. The organic layer was washed with a saturated aqueous sodium chloride solution and concentrated under reduced pressure. The obtained residue was purified by silica gel column chromatography to give the title compound (13 mg) as a white solid.

¹H-NMR (400 MHz, DMSO-d₆) δ: 12.24 (br, 1H), 8.34 (s, 1H), 8.26 (d, J = 8.0 Hz, 1H), 8.05 (d, J = 8.8 Hz, 2H), 7.97 (d, J = 8.8 Hz, 2H), 7.65 (d, J = 3.2 Hz, 1H), 6.89 (d, J = 3.2 Hz, 1H), 4.29 (s, 1H), 3.86-3.79 (m, 1H), 1.78-1.75 (m, 2H), 1.61-1.41 (m, 6H), 1.16 (s, 3H).

ESI-MS: 384.4[M+H]⁺

### Example 35

### Synthesis of 3-fluoro-4-(7-fluoro-1H-pyrrolo[3,2-c]pyridin-4-yl)-N-[trans-4-(2-hydroxypropan-2-yl)cyclohexyl]benzamide [35] (hereinafter, referred to as a compound [35])

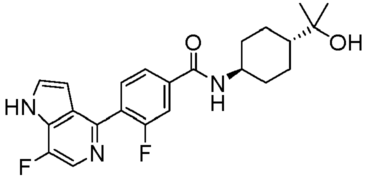

### (1) Synthesis of methyl 3-fluoro-4-(7-fluoro-1H-pyrrolo[3,2-c]pyridin-4-yl)benzoate [35-1] (hereinafter, referred to as a compound [35-1])

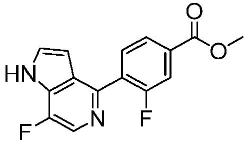

To a solution of 4-bromo-7-fluoro-1H-pyrrolo[3,2-c]pyridine (227 mg) in methanol (6.6 mL)/water (3.3 mL) were added 2-fluoro-4-(methoxycarbonyl)phenylboronic acid (272 mg), potassium carbonate (190 mg), and [1,3-bis(2,6-diisopropylphenyl)-4,5-dihydroimidazol-2-ylidene]chloro[3-phenylallyl]palladium (II) (34.3 mg) at room temperature, and the mixture was stirred at 80°C for 1 hour under an argon atmosphere. To the reaction mixture was added water, and the mixture was extracted with ethyl acetate. The organic layer was washed with a saturated aqueous sodium chloride solution and concentrated under reduced pressure. The obtained residue was suspended in ethyl acetate/methanol and the solid was collected by filtration to give the title compound (216 mg) as a white solid.

¹H-NMR (400 MHz, DMSO-d₆) δ: 12.39 (br, 1H), 8.30 (d, J = 2.4 Hz, 1H), 7.93-7.91 (m, 1H), 7.86-7.80 (m, 2H), 7.62 (d, J = 3.2 Hz, 1H), 6.52-6.49 (m, 1H), 3.91 (s, 3H).

ESI-MS: 289.3[M+H]⁺

### (2) Synthesis of 3-fluoro-4-(7-fluoro-1H-pyrrolo[3,2-c]pyridin-4-yl)benzoic acid [35-2] (hereinafter, referred to as a compound [35-2])

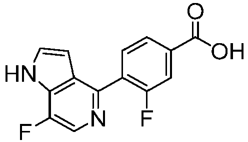

To a solution of the compound [35-1] (216 mg) in methanol (7.5 mL) was added a 2 M aqueous sodium hydroxide solution (3.8 mL) at room temperature, and the mixture was stirred at 60°C for 1 hour. The reaction mixture was concentrated under reduced pressure and 2 M hydrochloric acid was added. The resulting solid was collected by filtration, washed with water, and then dried under reduced pressure to give the title compound (195 mg) as a white solid.

¹H-NMR (400 MHz, DMSO-d₆) δ: 12.47 (br, 1H), 8.35 (s, 1H), 7.92-7.90 (m, 1H), 7.84-7.78 (m, 2H), 7.65 (br, 1H), 6.55 (br, 1H).

ESI-MS: 275.3[M+H]⁺

### (3) Synthesis of 3-fluoro-4-(7-fluoro-1H-pyrrolo[3,2-c]pyridin-4-yl)-N-[trans-4-(2-hydroxypropan-2-yl)cyclohexyl]benzamide [35]

To a solution of the compound [35-2] (14 mg) in DMF (2.6 mL) were added DIPEA (18 µL), COMU (44 mg), and 2-(trans-4-aminocyclohexyl)propan-2-ol (20 mg) at room temperature, and the mixture was stirred at room temperature for 3 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (7.6 mg) as a white solid.

¹H-NMR (400 MHz, DMSO-d₆) δ: 12.35 (br, 1H), 8.37 (d, J = 8.4 Hz, 1H), 8.29 (d, J = 2.0 Hz, 1H), 7.83-7.79 (m, 2H), 7.75-7.71 (m, 1H), 7.61 (d, J = 2.8 Hz, 1H), 6.49-6.47 (m, 1H), 4.04 (s, 1H), 3.76-3.67 (m, 1H), 1.92-1.82 (m, 4H), 1.36-1.27 (m, 2H), 1.22-1.04 (m, 9H).

ESI-MS: 414.5[M+H]⁺

### Example 36

### Synthesis of 3-fluoro-4-(7-fluoro-1H-pyrrolo[3,2-c]pyridin-4-yl)-N-(4-hydroxybicyclo[2.2.2]octan-1-yl)benzamide [36] (hereinafter, referred to as a compound [36])

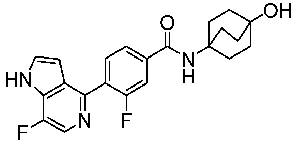

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added 4-aminobicyclo[2.2.2]octan-1-ol hydrochloride (13 mg) at room temperature, and the mixture was stirred at room temperature for 26 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (13 mg) as a white solid.

¹H-NMR (400 MHz, DMSO-d₆) δ: 12.34 (br, 1H), 8.28 (d, J = 2.4 Hz, 1H), 7.80-7.67 (m, 4H), 7.60 (d, J = 3.2 Hz, 1H), 6.47-6.45 (m, 1H), 4.31 (s, 1H), 2.06-2.02 (m, 6H), 1.64-1.60 (m, 6H).

ESI-MS: 398.4[M+H]⁺

### Example 37

### Synthesis of 3-fluoro-4-(7-fluoro-1H-pyrrolo[3,2-c]pyridin-4-yl)-N-(trans-4-hydroxy-4-methylcyclohexyl)benzamide [37] (hereinafter, referred to as a compound [37])

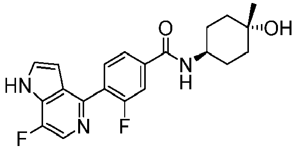

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added trans-4-amino-1-methylcyclohexanol hydrochloride (9.7 mg) at room temperature, and the mixture was stirred at room temperature for 26 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (13 mg) as a white solid.

¹H-NMR (400 MHz, DMSO-d₆) δ: 12.34 (br, 1H), 8.33 (d, J = 7.6 Hz, 1H), 8.29 (d, J = 2.8 Hz, 1H), 7.82-7.78 (m, 2H), 7.75-7.71 (m, 1H), 7.61 (d, J = 3.2 Hz, 1H), 6.49-6.47 (m, 1H), 4.30 (s, 1H), 3.86-3.79 (m, 1H), 1.79-1.76 (m, 2H), 1.62-1.58 (m, 2H), 1.53-1.41 (m, 4H), 1.16 (s, 3H).

ESI-MS: 386.4[M+H]⁺

### Example 38

### Synthesis of 3-fluoro-4-(7-fluoro-1H-pyrrolo[3,2-c]pyridin-4-yl)-N-(tetrahydropyran-4-yl)benzamide [38] (hereinafter, referred to as a compound [38])

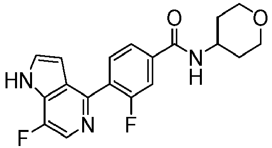

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added oxan-4-amine (7.8 µL) at room temperature, and the mixture was stirred at room temperature for 23 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (4.5 mg) as a white solid.

¹H-NMR (400 MHz, DMSO-d₆) δ: 12.35 (br, 1H), 8.50 (d, J = 7.6 Hz, 1H), 8.29 (d, J = 4.0 Hz, 1H), 7.84-7.80 (m, 2H), 7.76-7.72 (m, 1H), 7.61 (d, J = 4.8 Hz, 1H), 6.51-6.46 (m, 1H), 4.07-3.98 (m, 1H), 3.90-3.87 (m, 2H), 3.42-3.33 (m, 2H), 1.79-1.76 (m, 2H), 1.64-1.54 (m, 2H).

ESI-MS: 358.4[M+H]⁺

### Example 39

### Synthesis of 3-fluoro-4-(7-fluoro-1H-pyrrolo[3,2-c]pyridin-4-yl)-N-(trans-4-hydroxycyclohexyl)benzamide [39] (hereinafter, referred to as a compound [39])

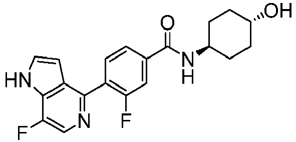

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added trans-4-aminocyclohexanol (8.6 mg) at room temperature, and the mixture was stirred at room temperature for 23 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (6.0 mg) as a white solid.

¹H-NMR (400 MHz, DMSO-d₆) δ: 12.37 (br, 1H), 8.37 (d, J = 7.6 Hz, 1H), 8.29 (d, J = 2.4 Hz, 1H), 7.82-7.78 (m, 2H), 7.75-7.71 (m, 1H), 7.61 (d, J = 3.2 Hz, 1H), 6.49-6.47 (m, 1H), 4.58 (d, J = 4.0 Hz, 1H), 3.79-3.69 (m, 1H), 3.44-3.33 (m, 1H), 1.88-1.82 (m, 4H), 1.44-1.33 (m, 2H), 1.29-1.20 (m, 2H).

ESI-MS: 372.4[M+H]⁺

### Example 40

### Synthesis of 3-fluoro-4-(7-fluoro-1H-pyrrolo[3,2-c]pyridin-4-yl)-N-(cis-4-hydroxy-4-methylcyclohexyl)benzamide [40] (hereinafter, referred to as a compound [40])

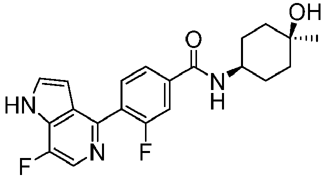

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added cis-4-amino-1-methylcyclohexanol (9.7 mg) at room temperature, and the mixture was stirred at room temperature for 25 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (6.0 mg) as a white solid.

¹H-NMR (400 MHz, DMSO-d₆) δ: 12.35 (br, 1H), 8.41 (d, J = 7.6 Hz, 1H), 8.29 (d, J = 2.0 Hz, 1H), 7.84-7.81 (m, 2H), 7.73-7.70 (m, 1H), 7.61 (d, J = 3.2 Hz, 1H), 6.49-6.47 (m, 1H), 4.06 (s, 1H), 3.78-3.69 (m, 1H), 1.80-1.71 (m, 2H), 1.59-1.56 (m, 4H), 1.39-1.32 (m, 2H), 1.12 (s, 3H).

ESI-MS: 386.4[M+H]⁺

### Example 41

### Synthesis of 3-fluoro-4-(7-fluoro-1H-pyrrolo[3,2-c]pyridin-4-yl)-N-(4-hydroxybicyclo[2.2.1]heptan-1-yl)benzamide [41] (hereinafter, referred to as a compound [41])

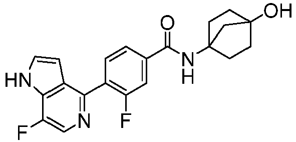

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added 4-aminobicyclo[2.2.1]heptan-1-ol hydrochloride (12 mg) at room temperature, and the mixture was stirred at room temperature for 25 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (6.5 mg) as a white solid.

¹H-NMR (400 MHz, DMSO-d₆) δ: 12.35 (br, 1H), 8.50 (s, 1H), 8.28 (d, J = 1.6 Hz, 1H), 7.81-7.78 (m, 2H), 7.72-7.69 (m, 1H), 7.61 (d, J = 3.2 Hz, 1H), 6.48-6.46 (m, 1H), 4.93 (s, 1H), 2.02-1.96 (m, 2H), 1.92-1.84 (m, 4H), 1.73-1.66 (m, 2H), 1.58-1.52 (m, 2H).

ESI-MS: 384.4[M+H]⁺

### Example 42

### Synthesis of 3-fluoro-4-(7-fluoro-1H-pyrrolo[3,2-c]pyridin-4-yl)-N-(cis-4-hydroxycyclohexyl)benzamide [42] (hereinafter, referred to as a compound [42])

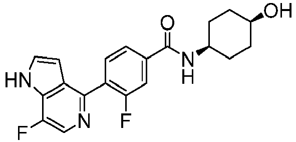

To a solution of the compound [35-2] (14 mg) in DMF (2.5 mL) were added DIPEA (64 µL) and COMU (32 mg) at room temperature, and the mixture was stirred at room temperature for 5 minutes. To the reaction mixture was added cis-4-aminocyclohexanol hydrochloride (11 mg) at room temperature, and the mixture was stirred at room temperature for 28 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (4.5 mg) as a white solid.

¹H-NMR (400 MHz, DMSO-d₆) δ: 12.35 (br, 1H), 8.40 (d, J = 7.2 Hz, 1H), 8.29 (d, J = 2.4 Hz, 1H), 7.84-7.82 (m, 2H), 7.74-7.70 (m, 1H), 7.61 (d, J = 3.2 Hz, 1H), 6.50-6.47 (m, 1H), 4.39 (d, J = 2.4 Hz, 1H), 3.85-3.75 (m, 2H), 1.83-1.67 (m, 4H), 1.58-1.46 (m, 4H).

ESI-MS: 372.4[M+H]⁺

### Example 43

### Synthesis of N-[trans-4-(2-hydroxypropan-2-yl)cyclohexyl]-4-(1H-pyrazolo[4,3-c]pyridin-4-yl)benzamide [43] (hereinafter, referred to as a compound [43])

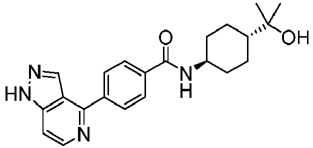

### (1) Synthesis of 4-chloro-1-{[2-(trimethylsilyl)ethoxy]methyl}-1H-pyrazolo[4,3-c]pyridine [43-1-a] (hereinafter, referred to as a compound [43-1-a]) and 4-chloro-2-{[2-(trimethylsilyl)ethoxy]methyl}-2H-pyrazolo[4,3-c]pyridine [43-1-b] (hereinafter, referred to as a compound [43-1-b])

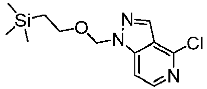

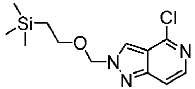

To a solution of 4-chloro-1H-pyrazolo[4,3-c]pyridine (50 mg) in THF (1.0 mL) were added 60% sodium hydride (16 mg) and 2-(trimethylsilyl)ethoxymethyl chloride (63 µL) at room temperature, and the mixture was stirred at room temperature for 45 minutes. To the reaction mixture was added water, and the mixture was extracted with ethyl acetate. The organic layer was dried over anhydrous sodium sulfate and concentrated under reduced pressure. The obtained residue was purified by silica gel column chromatography to give the title compounds [43-1-a] and [43-1-b] as a 1 : 1 mixture (50 mg).
Compound [43-1-a]
ESI-MS: 284.3[M+H]⁺
Compound [43-1-b]
ESI-MS: 284.3[M+H]⁺

### (2) Synthesis of N-[trans-4-(2-hydroxypropan-2-yl)cyclohexyl]-4-(1-{[2-(trimethylsilyl)ethoxy]methyl}-1H-pyrazolo[4,3-c]pyridin-4-yl)benzamide [43-2-a] (hereinafter, referred to as a compound [43-2-a]) and N-[trans-4-(2-hydroxypropan-2-yl)cyclohexyl]-4-(2-{[2-(trimethylsilyl)ethoxy]methyl}-2H-pyrazolo[4,3-c]pyridin-4-yl)benzamide [43-2-b] (hereinafter, referred to as a compound [43-2-b])

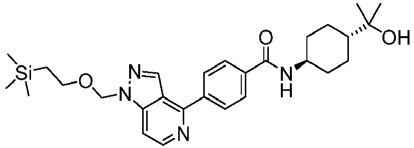

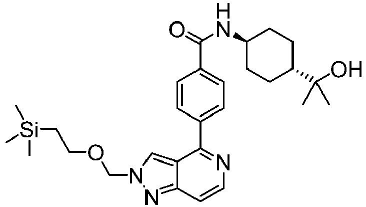

To a solution of the 1 : 1 mixture of the compounds [43-1-a] and [43-1-b] (50 mg) in ethanol (0.9 mL)/water (0.9 mL) were added the compound [13-1] (46 mg), potassium carbonate (32 mg), and [1,3-bis(2,6-diisopropylphenyl)imidazol-2-ylidene]chloro[3-phenylallyl]palladium (II) (5.7 mg) at room temperature, and the mixture was stirred at 80°C for 17 hours under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compounds [43-2-a] and [43-2-b] as a 1 : 1 mixture (51 mg).
Compound [43-2-a]
ESI-MS: 509.5[M+H]⁺
Compound [43-2-b]
ESI-MS: 509.5[M+H]⁺

### (3) Synthesis of N-[trans-4-(2-hydroxypropan-2-yl)cyclohexyl]-4-(1H-pyrazolo[4,3-c]pyridin-4-yl)benzamide [43]

To the 1 : 1 mixture of the compounds [43-2-a] and [43-2-b] (51 mg) was added a solution of 1 M tetrabutylammonium fluoride in THF (1.0 mL) at room temperature, and the mixture was stirred at 60°C for 21 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (22.1 mg) as a white solid.

¹H-NMR (400 MHz, CD₃OD) δ: 8.41-8.39 (m, 2H), 8.10-8.05 (m, 2H), 8.03-7.99 (m, 2H), 7.56 (d, J = 5.9 Hz, 1H), 3.90-3.81 (m, 1H), 2.11-2.07 (m, 2H), 1.98-1.95 (m, 2H), 1.45-1.17 (m, 11H).

ESI-MS: 379.5[M+H]⁺

### Example 44

### Synthesis of N-[trans-4-(2-hydroxypropan-2-yl)cyclohexyl]-4-(1H-imidazo[4,5-c]pyridin-4-yl)benzamide [44] (hereinafter, referred to as a compound [44])

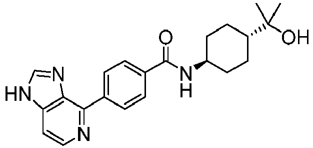

The title compound was synthesized by a method according to Example 43, using 4-chloro-1H-imidazo[4,5-c]pyridine in place of 4-chloro-1H-pyrazolo[4,3-c]pyridine.

¹H-NMR (400 MHz, CD₃OD) δ: 8.44-8.40 (m, 2H), 8.33-8.27 (m, 2H), 8.00-7.99 (m, 2H), 7.65-7.64 (m, 1H), 3.90-3.84 (m, 1H), 2.11-2.08 (m, 2H), 1.98-1.95 (m, 2H), 1.48-1.20 (m, 11H).

ESI-MS: 379.4[M+H]⁺

### Example 45

### Synthesis of 4-(1H-[1,2,3]triazolo[4,5-c]pyridin-4-yl)-N-[trans-4-(2-hydroxypropan-2-yl)cyclohexyl]benzamide [45] (hereinafter, referred to as a compound [45])

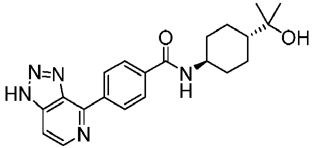

The title compound was synthesized by a method according to Example 43, using 4-chloro-3H-[1,2,3]triazolo[4,5-c]pyridine hydrochloride in place of 4-chloro-1H-pyrazolo[4,3-c]pyridine.

¹H-NMR (400 MHz, DMSO-d₆) δ: 8.85-8.80 (m, 2H), 8.60 (d, J = 5.9 Hz, 1H), 8.33 (d, J = 8.2 Hz, 1H), 8.04 (d, J = 8.2 Hz, 2H), 7.83 (d, J = 5.5 Hz, 1H), 4.03 (s, 1H), 3.76-3.72 (m, 1H), 1.95-1.83 (m, 4H), 1.38-1.05 (m, 11H).

ESI-MS: 380.5[M+H]⁺

### Example 46

### Synthesis of 4-(7-fluoro-1H-pyrazolo[4,3-c]pyridin-4-yl)-N-[trans-4-(2-hydroxypropan-2-yl)cyclohexyl]benzamide [46] (hereinafter, referred to as a compound [46])

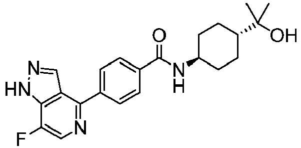

### (1) Synthesis of 4-chloro-7-fluoro-1-(4-methoxybenzyl)-1H-pyrazolo[4,3-c]pyridine [46-1-a] (hereinafter, referred to as a compound [46-1-a]) and 4-chloro-7-fluoro-2-(4-methoxybenzyl)-2H-pyrazolo[4,3-c]pyridine [46-1-b] (hereinafter, referred to as a compound [46-1-b])

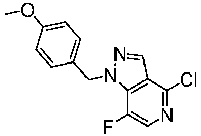

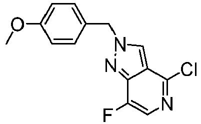

To a solution of 4-chloro-7-fluoro-1H-pyrazolo[4,3-c]pyridine (30 mg) in DMF (1.8 mL) were added 4-methoxybenzyl chloride (29 µL) and cesium carbonate (86 mg) at room temperature, and the mixture was stirred at room temperature for 4.5 hours. To the reaction mixture were added 4-methoxybenzyl chloride (7.2 µL) and cesium carbonate (29 mg) at room temperature, and the mixture was further stirred at room temperature for 30 minutes. To the reaction mixture was added water, and the mixture was extracted with ethyl acetate. The organic layer was dried over anhydrous sodium sulfate and concentrated under reduced pressure. The obtained residue was purified by silica gel column chromatography to give both the title compound [46-1-a] (20.7 mg) and the title compound [46-1-b] (29.1 mg) as white solids.

### Compound [46-1-a]

¹H-NMR (400 MHz, CDCl₃) δ: 8.14 (d, J = 2.0 Hz, 1H), 8.01 (d, J = 3.2 Hz, 1H), 7.28-7.27 (m, 2H), 6.85 (d, J = 8.8 Hz, 2H), 5.61 (s, 2H), 3.77 (s, 3H).

ESI-MS: 292.2[M+H]⁺

### Compound [46-1-b]

¹H-NMR (400 MHz, CDCl₃) δ: 8.00 (d, J = 2.4 Hz, 1H), 7.90 (d, J = 2.8 Hz, 1H), 7.34 (d, J = 8.8 Hz, 2H), 6.95 (d, J = 8.4 Hz, 2H), 5.57 (s, 2H), 3.83 (s, 3H).

ESI-MS: 292.2[M+H]⁺

### (2) Synthesis of 4-[7-fluoro-1-(4-methoxybenzyl)-1H-pyrazolo[4,3-c]pyridin-4-yl]-N-[trans-4-(2-hydroxypropan-2-yl)cyclohexyl]benzamide [46-2] (hereinafter, referred to as a compound [46-2])

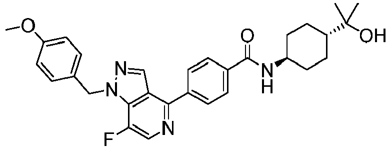

To a solution of the compound [46-1-a] (13.5 mg) in ethanol (0.30 mL)/water (0.15 mL) were added the compound [13-1] (26.7 mg), potassium carbonate (25 mg), and [1,3-bis(2,6-diisopropylphenyl)-4,5-dihydroimidazol-2-ylidene]chloro[3-phenylallyl]palladium (II) (2.9 mg) at room temperature, and the mixture was stirred at 80°C for 45 minutes under an argon atmosphere. To the reaction mixture was added water, and the mixture was extracted with ethyl acetate. The organic layer was dried over anhydrous sodium sulfate and concentrated under reduced pressure to give the title compound [46-2] (26.5 mg) as a crude product.

¹H-NMR (400 MHz, CDCl₃) δ: 8.33 (d, J = 3.2 Hz, 1H), 8.26 (d, J = 2.0 Hz, 1H), 7.99 (d, J = 8.0 Hz, 2H), 7.92 (d, J = 8.8 Hz, 2H), 7.31 (d, J = 8.8 Hz, 2H), 6.86 (d, J = 8.8 Hz, 2H), 5.67 (s, 2H), 4.00-3.92 (m, 1H), 3.77 (s, 3H), 2.22-2.20 (m, 2H), 2.05-1.93 (m, 2H), 1.40-1.20 (m, 11H).

ESI-MS: 517.5[M+H]⁺

### (3) Synthesis of 4-(7-fluoro-1H-pyrazolo[4,3-c]pyridin-4-yl)-N-[trans-4-(2-hydroxypropan-2-yl)cyclohexyl]benzamide [46]

To a solution of the compound [46-2] (8.0 mg) in acetonitrile (0.45 mL)/water (0.15 mL) was added cerium (IV) ammonium nitrate (42 mg) at room temperature, and the mixture was stirred at room temperature for 2 hours. To the reaction mixture were added a saturated aqueous sodium bicarbonate solution and a saturated aqueous sodium thiosulfate solution, and the mixture was extracted with ethyl acetate. The organic layer was dried over anhydrous sodium sulfate and concentrated under reduced pressure. The obtained residue was purified by silica gel column chromatography to give the title compound (1.0 mg) as a white solid.

¹H-NMR (400 MHz, CD₃OD) δ: 8.49 (d, J = 3.2 Hz, 1H), 8.33-8.32 (m, 1H), 8.06 (d, J = 8.4 Hz, 2H), 8.00 (d, J = 8.8 Hz, 2H), 3.90-3.81 (m, 1H), 2.10-2.07 (m, 2H), 1.97-1.94 (m, 2H), 1.44-1.17 (m, 11H).

ESI-MS: 397.4[M+H]⁺

### Example 47

### Synthesis of 4-(7-fluoro-1H-pyrazolo[4,3-c]pyridin-4-yl)-N-(4-hydroxybicyclo[2.2.2]octan-1-yl)benzamide [47] (hereinafter, referred to as a compound [47])

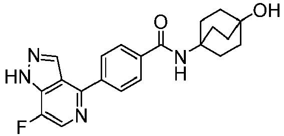

The title compound was synthesized by a method according to Example 46, using the compound [16-1] in place of the compound [13-1] in the step (2).

¹H-NMR (400 MHz, DMSO-d₆) δ: 8.62 (d, J = 3.2 Hz, 1H), 8.44-8.43 (m, 1H), 8.09 (d, J = 8.0 Hz, 2H), 7.93 (d, J = 8.4 Hz, 2H), 7.76 (s, 1H), 4.31 (s, 1H), 2.07-2.00 (m, 6H), 1.64-1.60 (m, 6H).

ESI-MS: 381.3[M+H]⁺

### Example 48

### Synthesis of 4-(7-chloro-1H-pyrazolo[4,3-c]pyridin-4-yl)-N-[trans-4-(2-hydroxypropan-2-yl)cyclohexyl]benzamide [48] (hereinafter, referred to as a compound [48])

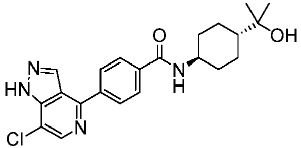

### (1) Synthesis of 7-chloro-1H-pyrazolo[4,3-c]pyridine [48-1] (hereinafter, referred to as a compound [48-1])

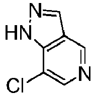

To a solution of 4,5-dichloropyridine-3-carbaldehyde (540 mg) in ethanol (4.0 mL) was added hydrazine monohydrate (596 µL) at room temperature, and the mixture was stirred at 80°C for 18 hours. To the reaction mixture was added water, and the resulting solid was collected by filtration, washed with water, and then dried under reduced pressure to give the title compound (351 mg) as a pale yellow solid.

¹H-NMR (400 MHz, CDCl₃) δ: 10.55 (br, 1H), 9.05 (s, 1H), 8.46 (s, 1H), 8.28 (s, 1H).

ESI-MS: 154.1[M+H]⁺

### (2) Synthesis of 7-chloro-1-(4-methoxybenzyl)-1H-pyrazolo[4,3-c]pyridine [48-2-a] (hereinafter, referred to as a compound [48-2-a]) and 7-chloro-2-(4-methoxybenzyl)-2H-pyrazolo[4,3-c]pyridine [48-2-b] (hereinafter, referred to as a compound [48-2-b])

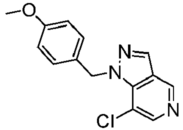

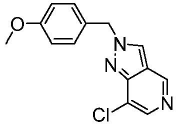

To a solution of the compound [48-1] (350 mg) in DMF (11 mL) were added 4-methoxybenzyl chloride (373 µL) and cesium carbonate (1.49 g) at room temperature, and the mixture was stirred at room temperature for 29 hours. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound [48-2-a] (259 mg) as a white solid and the title compound [48-2-b] (301 mg) as a colorless oil.

### Compound [48-2-a]

¹H-NMR (400 MHz, CDCl₃) δ: 8.96 (s, 1H), 8.37 (s, 1H), 8.20 (s, 1H), 7.21 (d, J = 8.4 Hz, 2H), 6.84 (d, J = 8.4 Hz, 2H), 5.88 (s, 2H), 3.77 (s, 3H).

### Compound [48-2-b]

¹H-NMR (400 MHz, CDCl₃) δ: 8.97 (s, 1H), 8.30 (s, 1H), 8.00 (s, 1H), 7.33 (d, J = 8.8 Hz, 2H), 6.94 (d, J = 8.8 Hz, 2H), 5.61 (s, 2H), 3.82 (s, 3H).

### (3) Synthesis of 7-chloro-1-(4-methoxybenzyl)-1H-pyrazolo[4,3-c]pyridine=5-oxide [48-3] (hereinafter, referred to as a compound [48-3])

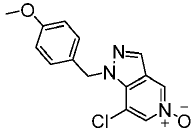

To a solution of the compound [48-2-a] (263 mg) in chloroform (4.8 mL) was added m-chloroperbenzoic acid (249 mg) at room temperature, and the mixture was stirred at room temperature for 20 hours. To the reaction mixture were added a saturated aqueous sodium bicarbonate solution and a saturated aqueous sodium thiosulfate solution, and the mixture was extracted with ethyl acetate. The organic layer was dried over anhydrous sodium sulfate and concentrated under reduced pressure. The obtained residue was purified by silica gel column chromatography to give the title compound (233 mg) as a white solid.

¹H-NMR (400 MHz, CDCl₃) δ: 8.59 (d, J = 1.2 Hz, 1H), 8.18 (d, J = 1.2 Hz, 1H), 8.07 (s, 1H), 7.19 (d, J = 8.8 Hz, 2H), 6.85 (d, J = 8.8 Hz, 2H), 5.82 (s, 2H), 3.78 (s, 3H) .

ESI-MS: 290.2[M+H]⁺

### (4) Synthesis of 4-bromo-7-chloro-1-(4-methoxybenzyl)-1H-pyrazolo[4,3-c]pyridine [48-4] (hereinafter, referred to as a compound [48-4])

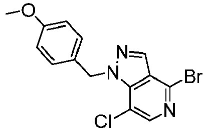

To a solution of the compound [48-3] (171 mg) in acetonitrile (11.8 mL) was added phosphoryl bromide (678 mg) at room temperature, and the mixture was stirred at 70°C for 1.5 hours. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (152 mg) as a white solid.

¹H-NMR (400 MHz, CDCl₃) δ: 8.13 (s, 1H), 8.11 (s, 1H), 7.20 (d, J = 8.8 Hz, 2H), 6.84 (d, J = 8.8 Hz, 2H), 5.85 (s, 2H), 3.77 (s, 3H).

ESI-MS: 352.0[M+H]⁺

### (5) Synthesis of 4-[7-chloro-1-(4-methoxybenzyl)-1H-pyrazolo[4,3-c]pyridin-4-yl]-N-[trans-4-(2-hydroxypropan-2-yl)cyclohexyl]benzamide [48-5] (hereinafter, referred to as a compound [48-5])

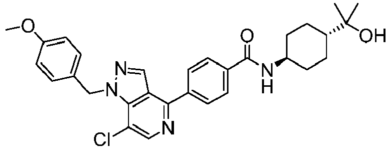

To a solution of the compound [48-4] (102 mg) in 1,4-dioxane (5.2 mL)/water (0.6 mL) were added the compound [13-1] (123 mg), cesium carbonate (113 mg), and PdCl₂(dppf) ·CH₂Cl₂ (11.8 mg) at room temperature, and the mixture was stirred at 100°C for 19 hours under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (89.6 mg) as a white solid.

¹H-NMR (400 MHz, CDCl₃) δ: 8.45 (s, 1H), 8.31 (s, 1H), 8.02 (d, J = 8.8 Hz, 2H), 7.93 (d, J = 8.8 Hz, 2H), 7.26-7.23 (m, 2H), 6.87-6.84 (m, 2H), 5.99 (d, J = 8.0 Hz, 1H), 5.93 (s, 2H), 3.98-3.95 (m, 1H), 3.77 (s, 3H), 2.26-2.18 (m, 2H), 1.95-1.91 (m, 2H), 1.29-1.21 (m, 11H).

ESI-MS: 533.3[M+H]⁺

### (6) Synthesis of 4-(7-chloro-1H-pyrazolo[4,3-c]pyridin-4-yl)-N-[trans-4-(2-hydroxypropan-2-yl)cyclohexyl]benzamide [48]

To a solution of the compound [48-5] (42 mg) in acetonitrile (1.3 mL)/water (0.3 mL) was added cerium (IV) ammonium nitrate (218 mg) at room temperature, and the mixture was stirred at room temperature for 3 hours. To the reaction mixture were added a saturated aqueous sodium bicarbonate solution and a saturated aqueous sodium thiosulfate solution, and the mixture was extracted with ethyl acetate. The organic layer was dried over anhydrous sodium sulfate and concentrated under reduced pressure. The obtained residue was purified by silica gel column chromatography to give the title compound (17 mg) as a white solid.

¹H-NMR (400 MHz, CD₃OD) δ: 8.51 (s, 1H), 8.42 (s, 1H), 8.10 (d, J = 8.4 Hz, 2H), 8.01 (d, J = 8.4 Hz, 2H), 3.90-3.82 (m, 1H), 2.10-2.07 (m, 2H), 1.97-1.94 (m, 2H), 1.46-1.17 (m, 11H).

ESI-MS: 413.2[M+H]⁺

### Example 49

### Synthesis of 3-fluoro-N-[trans-4-(2-hydroxypropan-2-yl)cyclohexyl]-4-(1H-pyrazolo[4,3-c]pyridin-4-yl)benzamide [49] (hereinafter, referred to as a compound [49])

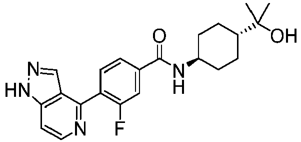

### (1) Synthesis of 4-chloro-1-(4-methoxybenzyl)-1H-pyrazolo[4,3-c]pyridine [49-1-a] (hereinafter, referred to as a compound [49-1-a]) and 4-chloro-2-(4-methoxybenzyl)-2H-pyrazolo[4,3-c]pyridine [49-1-b] (hereinafter, referred to as a compound [49-1-b])

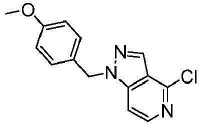

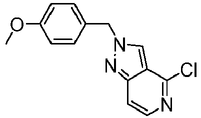

To a solution of 4-chloro-1H-pyrazolo[4,3-c]pyridine (77 mg) in DMF (2.5 mL) were added 4-methoxybenzyl chloride (82 µL) and cesium carbonate (325 mg) at room temperature, and the mixture was stirred at room temperature for 29 hours. To the reaction mixture was added water, and the mixture was extracted with ethyl acetate. The organic layer was dried over anhydrous sodium sulfate and concentrated under reduced pressure. The obtained residue was purified by silica gel column chromatography to give both the title compound [49-1-a] (93 mg) and the title compound [49-1-b] (50 mg) as white solids.

### Compound [49-1-a]

¹H-NMR (400 MHz, CDCl₃) δ: 8.17 (s, 1H), 8.12 (d, J = 6.4 Hz, 1H), 7.18-7.15 (m, 3H), 6.85 (d, J = 8.8 Hz, 2H), 5.52 (s, 2H), 3.78 (s, 3H).

### Compound [49-1-b]

¹H-NMR (400 MHz, CDCl₃) δ: 8.04 (s, 1H), 8.02 (d, J = 6.4 Hz, 1H), 7.47 (d, J = 6.4 Hz, 1H), 7.31 (d, J = 8.8 Hz, 2H), 6.94 (d, J = 8.8 Hz, 2H), 5.54 (s, 2H), 3.82 (s, 3H).

### (2) Synthesis of methyl 3-fluoro-4-[1-(4-methoxybenzyl)-1H-pyrazolo[4,3-c]pyridin-4-yl]benzoate [49-2] (hereinafter, referred to as a compound [49-2])

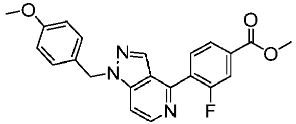

To a solution of the compound [49-1-a] (90 mg) in 1,4-dioxane (2.9 mL)/water (0.4 mL) were added 2-fluoro-4-(methoxycarbonyl)phenylboronic acid (72 mg), cesium carbonate (129 mg), and PdCl₂ (dppf) ·CH₂Cl₂ (14 mg) at room temperature, and the mixture was stirred at 100°C for 1.5 hours under an argon atmosphere. To the reaction mixture was added water, and the mixture was extracted with ethyl acetate. The organic layer was dried over anhydrous sodium sulfate and concentrated under reduced pressure. The obtained residue was purified by silica gel column chromatography to give the title compound (108 mg) as a white solid.

¹H-NMR (400 MHz, CDCl₃) δ: 8.51 (d, J = 6.0 Hz, 1H), 8.11 (d, J = 4.4 Hz, 1H), 7.99 (d, J = 7.6 Hz, 1H), 7.94-7.87 (m, 2H), 7.29 (d, J = 6.0 Hz, 1H), 7.21 (d, J = 8.4 Hz, 2H), 6.86 (d, J = 8.4 Hz, 2H), 5.57 (s, 2H), 3.98 (s, 3H), 3.78 (s, 3H).

ESI-MS: 392.2[M+H]⁺

### (3) Synthesis of 3-fluoro-4-[1-(4-methoxybenzyl)-1H-pyrazolo[4,3-c]pyridin-4-yl]benzoic acid [49-3] (hereinafter, referred to as a compound [49-3])

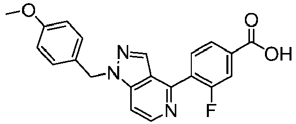

To a solution of the compound [49-2] (108 mg) in methanol (2.8 mL)/THF (1.4 mL) was added a 2 M aqueous sodium hydroxide solution (1.4 mL) at room temperature, and the mixture was stirred at room temperature for 24 hours. The reaction mixture was concentrated under reduced pressure and 2 M hydrochloric acid was added. The resulting solid was collected by filtration, washed with water, and then dried under reduced pressure to give the title compound (102 mg) as a white solid.

¹H-NMR (400 MHz, CD₃OD) δ: 8.44 (d, J = 6.0 Hz, 1H), 8.19 (d, J = 2.8 Hz, 1H), 8.03 (dd, J = 8.0, 1.2 Hz, 1H), 7.95-7.93 (m, 1H), 7.85 (dd, J = 7.6, 7.2 Hz, 1H), 7.70 (d, J = 6.0 Hz, 1H), 7.25 (d, J = 8.8 Hz, 2H), 6.87 (d, J = 8.8 Hz, 2H), 5.64 (s, 2H), 3.75 (s, 3H).

ESI-MS: 378.2[M+H]⁺

### (4) Synthesis of 3-fluoro-N-[trans-4-(2-hydroxypropan-2-yl)cyclohexyl]-4-[1-(4-methoxybenzyl)-1H-pyrazolo[4,3-c]pyridin-4-yl]benzamide [49-4] (hereinafter, referred to as a compound [49-4])

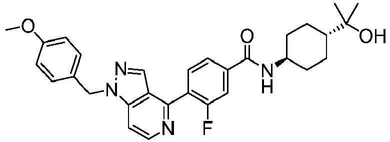

To a solution of the compound [49-3] (38 mg) in DMF (1.0 mL) were added DIPEA (19 µL) and COMU (47 mg) at room temperature, and the mixture was stirred at room temperature for 5 minutes. To the reaction mixture was added 2-(trans-4-aminocyclohexyl)propan-2-ol (24 mg) at room temperature, and the mixture was stirred at room temperature for 1 hour. To the reaction mixture was added a saturated aqueous sodium bicarbonate solution, and the mixture was extracted with ethyl acetate. The organic layer was dried over anhydrous sodium sulfate and concentrated under reduced pressure. The obtained residue was purified by silica gel column chromatography to give the title compound (41 mg) as a white solid.

¹H-NMR (400 MHz, DMSO-d₆) δ: 8.50 (d, J = 6.4 Hz, 1H), 8.41 (d, J = 7.6 Hz, 1H), 8.17 (d, J = 3.6 Hz, 1H), 7.87-7.82 (m, 4H), 7.26 (d, J = 8.8 Hz, 2H), 6.88 (d, J = 8.8 Hz, 2H), 5.64 (s, 2H), 4.04 (s, 1H), 3.77-3.67 (m, 4H), 1.92-1.89 (m, 2H), 1.85-1.82 (m, 2H), 1.36-1.04 (m, 11H).

ESI-MS: 517.3[M+H]⁺

### (5) Synthesis of 3-fluoro-N-[trans-4-(2-hydroxypropan-2-yl)cyclohexyl]-4-(1H-pyrazolo[4,3-c]pyridin-4-yl)benzamide [49]

To a solution of the compound [49-4] (39 mg) in acetonitrile (1.2 mL)/water (0.3 mL) was added cerium (IV) ammonium nitrate (205 mg) at room temperature, and the mixture was stirred at room temperature for 4 hours. To the reaction mixture were added a saturated aqueous sodium bicarbonate solution and a saturated aqueous sodium thiosulfate solution, and the mixture was extracted with ethyl acetate. The organic layer was dried over anhydrous sodium sulfate and concentrated under reduced pressure. The obtained residue was purified by silica gel column chromatography to give the title compound (15 mg) as a white solid.

¹H-NMR (400 MHz, CD₃OD) δ: 8.45 (d, J = 5.6 Hz, 1H), 8.15 (d, J = 2.4 Hz, 1H), 7.83-7.78 (m, 3H), 7.62 (d, J = 6.4 Hz, 1H), 3.89-3.82 (m, 1H), 2.09-2.07 (m, 2H), 1.97-1.95 (m, 2H), 1.46-1.15 (m, 11H).

ESI-MS: 397.2[M+H]⁺

### Example 50

The H-PGDS inhibitory activity of each Example compound was measured according to the method described in WO 2007/041634.

Recombinant human H-PGDS (final concentration: 1.0 nM) expressed in E. coli was mixed in an assay buffer containing 20 mM Tris-HCl (pH 8.0), 2 mM magnesium chloride, 2 mM reduced glutathione, and 0.5 mg/mL γ-globulin, and 150 µL of the mixture was dispensed into an 8-strip PCR tube (BM Equipment Co., Ltd.). Test compounds (Example compounds) (16.5 µL) at various concentrations dissolved in DMSO were added and the mixture was preincubated for 15 minutes on ice.

Prostaglandin H₂ (final concentration: 25 µM) (Cayman Chemical) was dispensed into another 8-strip PCR tube, and the solvent was dried and solidified using a vacuum concentrator (Thermo Fisher Scientific). To the dried and solidified prostaglandin H₂ was added 50 µL of the assay buffer containing H-PGDS and each test compound after the preincubation, and the mixture was reacted on ice for 1 minute. 40 µL of this mixture was added to 40 µL of a stop solution (50 mM aqueous citric acid solution containing iron chloride at a final concentration of 4 mg/mL) to stop the reaction. Then, the reaction solution was deproteinized, added to a 96 square deep well plate (SHONANMARUHACHI STEC Co., Ltd.), and the prostaglandin D₂ concentration in the reaction solution was measured by LC-MS/MS. ACQUITY (registered trademark) TQD (Waters) was used as a measuring instrument.

Prostaglandin D₂ production activity (%) in the group with each test compound when a value obtained by subtracting the amount of production of prostaglandin D₂ in the group without enzyme, which is non-specific production of prostaglandin D₂, from the amount of production of prostaglandin D₂ in the group without test compound (DMSO-addition group) in each 8-strip PCR tube is 100% was calculated. From the calculated activity value, the test compound concentration that inhibits prostaglandin D₂ production by 50% (IC₅₀) was determined. The results are shown in Table 1 below.

**Table 1**

| [Table 2-1] | |
|---|---|
| Example number of test compound | H-PGDS inhibitory activity (IC₅₀, nM) |
| Example 1 | 0.99 |
| Example 2 | 1.3 |
| Example 3 | 1.9 |
| Example 4 | 1.5 |
| Example 5 | 1.0 |
| Example 6 | 1.1 |
| Example 7 | 1.3 |
| Example 8 | 0.75 |
| Example 9 | 1.2 |
| Example 10 | 1.0 |
| Example 11 | 3.9 |
| Example 12 | 0.88 |
| Example 13 | 1.4 |
| Example 14 | 1.2 |
| Example 15 | 3.4 |
| Example 16 | 0.99 |
| Example 17 | 0.69 |
| Example 18 | 2.8 |
| Example 19 | 1.4 |
| Example 20 | 0.94 |
| Example 21 | 1.7 |
| Example 22 | 2.1 |
| Example 23 | 0.79 |
| Example 24 | 0.86 |
| Example 25 | 0.70 |
| Example 26 | 1.3 |
| Example 27 | 2.3 |
| Example 28 | 1.7 |
| Example 29 | 0.85 |
| Example 30 | 0.68 |
| Example 31 | 1.0 |
| Example 32 | 0.91 |

**[Table 2-2]**

| Example number of test compound | H-PGDS inhibitory activity (IC₅₀, nM) |
|---|---|
| Example 33 | 0.77 |
| Example 34 | 0.86 |
| Example 35 | 1.2 |
| Example 36 | 0.71 |
| Example 37 | 0.95 |
| Example 38 | 0.85 |
| Example 39 | 0.65 |
| Example 40 | 1.1 |
| Example 41 | 0.89 |
| Example 42 | 0.92 |
| Example 43 | 2.4 |
| Example 44 | 4.0 |
| Example 45 | 24 |

**[Table 2-3]**

| Example number of test compound | H-PGDS inhibitory activity (IC₅₀, nM) |
|---|---|
| Example 46 | 5.3 |
| Example 47 | 14 |
| Example 48 | 5.9 |
| Example 49 | 0.78 |

The test results indicate that the compound of the present invention exhibits H-PGDS inhibitory activity.

The correspondence relation between each compound of Examples 1 to 49 and Formula (I) is shown below.

**[Table 3-1]**

| | -X= | =Y- | R¹ | R² | R³ | Z |
|---|---|---|---|---|---|---|
| Example 1 | -CH= | -CH- | Methyl group | Hydrogen atom | Hydrogen atom | 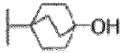 |
| Example 2 | -CH= | -CH- | Methyl group | Hydrogen atom | Hydrogen atom | 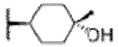 |
| Example 3 | -CH= | -CH- | Methyl group | Hydrogen atom | Hydrogen atom | 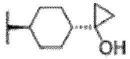 |
| Example 4 | -CH= | -CH- | Methyl group | Hydrogen atom | Hydrogen atom | 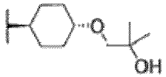 |
| Example 5 | -CH= | -CH- | Hydrogen atom | Hydrogen atom | Fluorine atom | 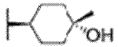 |
| Example 6 | -CH= | -CH- | Hydrogen atom | Hydrogen atom | Fluorine atom | 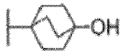 |
| Example 7 | -CH= | -CH- | Hydrogen atom | Hydrogen atom | Fluorine atom | 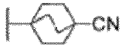 |

**[Table 3-2]**

| | -X= | =Y- | R¹ | R² | R³ | Z |
|---|---|---|---|---|---|---|
| Example 8 | -CH= | -CH- | Hydrogen atom | Hydrogen atom | Fluorine atom | 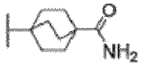 |
| Example 9 | -CH= | -CH- | Hydrogen atom | Hydrogen atom | Fluorine atom | 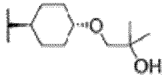 |
| Example 10 | -CH= | -CH- | Hydrogen atom | Hydrogen atom | Fluorine atom | 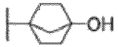 |
| Example 11 | -CH= | -CH- | Hydrogen atom | Hydrogen atom | Fluorine atom | 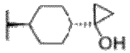 |
| Example 12 | -CH= | -CH- | Hydrogen atom | Hydrogen atom | Fluorine atom | 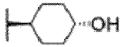 |
| Example 13 | -CH= | -CH- | Fluorine atom | Hydrogen atom | Hydrogen atom | 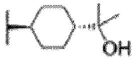 |
| Example 14 | -CH= | -CH- | Chlorine atom | Hydrogen atom | Hydrogen atom | 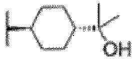 |

**[Table 3-3]**

| | -X= | =Y- | R¹ | R² | R³ | Z |
|---|---|---|---|---|---|---|
| Example 15 | -CH= | -CH- | Cyano group | Hydrogen atom | Hydrogen atom | 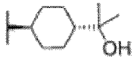 |
| Example 16 | -CH= | -CH- | Fluorine atom | Hydrogen atom | Hydrogen atom | 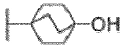 |
| Example 17 | -CH= | -CH- | Chlorine atom | Hydrogen atom | Hydrogen atom | 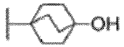 |
| Example 18 | -CH= | -CH- | Difluorome thyl group | Hydrogen atom | Hydrogen atom | 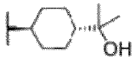 |
| Example 19 | -CH= | -CH- | Fluorine atom | Hydrogen atom | Hydrogen atom | 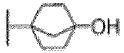 |
| Example 20 | -CH= | -CH- | Fluorine atom | Hydrogen atom | Hydrogen atom | 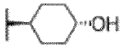 |
| Example 21 | -CH= | -CH- | Fluorine atom | Hydrogen atom | Hydrogen atom | 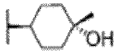 |

**[Table 3-4]**

| | -X= | =Y- | R¹ | R² | R³ | z |
|---|---|---|---|---|---|---|
| Example 22 | -CH= | -CH- | Fluorine atom | Hydrogen atom | Hydrogen atom | 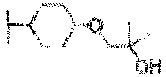 |
| Example 23 | -CH= | -CH- | Fluorine atom | Hydrogen atom | Hydrogen atom | 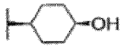 |
| Example 24 | -CH= | -CH- | Fluorine atom | Hydrogen atom | Hydrogen atom | 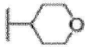 |
| Example 25 | -CH= | -CH- | Fluorine atom | Hydrogen atom | Hydrogen atom | 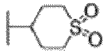 |
| Example 26 | -CH= | -CH- | Fluorine atom | Hydrogen atom | Hydrogen atom | 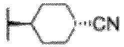 |
| Example 27 | -CH= | -CH- | Fluorine atom | Hydrogen atom | Hydrogen atom | 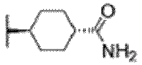 |
| Example 28 | -CH= | -CH- | Chlorine atom | Hydrogen atom | Hydrogen atom | 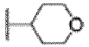 |

**[Table 3-5]**

| | -X= | =Y- | R¹ | R² | R³ | Z |
|---|---|---|---|---|---|---|
| Example 29 | -CH= | -CH- | Chlorine atom | Hydrogen atom | Hydrogen atom | 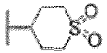 |
| Example 30 | -CH= | -CH- | Chlorine atom | Hydrogen atom | Hydrogen atom | 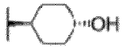 |
| Example 31 | -CH= | -CH- | Chlorine atom | Hydrogen atom | Hydrogen atom | 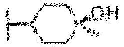 |
| Example 32 | -CH= | -CH- | Chlorine atom | Hydrogen atom | Hydrogen atom | 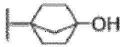 |
| Example 33 | -CH= | -CH- | Chlorine atom | Hydrogen atom | Hydrogen atom | 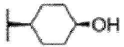 |
| Example 34 | -CH= | -CH- | Chlorine atom | Hydrogen atom | Hydrogen atom | 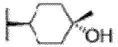 |
| Example 35 | -CH= | -CH- | Fluorine atom | Hydrogen atom | Fluorine atom | 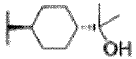 |

**[Table 3-6]**

| | -X= | =Y- | R¹ | R² | R³ | Z |
|---|---|---|---|---|---|---|
| Example 36 | -CH= | -CH- | Fluorine atom | Hydrogen atom | Fluorine atom | 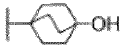 |
| Example 37 | -CH= | -CH- | Fluorine atom | Hydrogen atom | Fluorine atom | 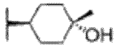 |
| Example 38 | -CH= | -CH- | Fluorine atom | Hydrogen atom | Fluorine atom | 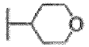 |
| Example 39 | -CH= | -CH- | Fluorine atom | Hydrogen atom | Fluorine atom | 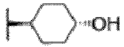 |
| Example 40 | -CH= | -CH- | Fluorine atom | Hydrogen atom | Fluorine atom | 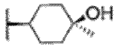 |
| Example 41 | -CH= | -CH- | Fluorine atom | Hydrogen atom | Fluorine atom | 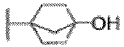 |
| Example 42 | -CH= | -CH- | Fluorine atom | Hydrogen atom | Fluorine atom | 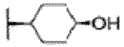 |

**[Table 3-7]**

| | -X= | =Y- | R¹ | R² | R³ | Z |
|---|---|---|---|---|---|---|
| Example 43 | -N= | =CH- | Hydrogen atom | Hydrogen atom | Hydrogen atom | 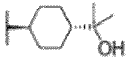 |
| Example 44 | -CH= | =N- | Hydrogen atom | Hydrogen atom | Hydrogen atom | 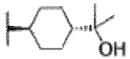 |
| Example 45 | -N= | =N- | Hydrogen atom | Hydrogen atom | Hydrogen atom | 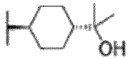 |

**[Table 3-8]**

| | -X= | =Y- | R¹ | R² | R³ | Z |
|---|---|---|---|---|---|---|
| Example 46 | -N= | =CH- | Fluorine atom | Hydrogen atom | Hydrogen atom | |
| Example 47 | -N= | =CH- | Fluorine atom | Hydrogen atom | Hydrogen atom | |
| Example 48 | -N= | =CH- | Chlorine atom | Hydrogen atom | Hydrogen atom | |
| Example 49 | -N= | =CH- | Hydrogen atom | Hydrogen atom | Fluorine atom | |

### Industrial Applicability

The compound of the present invention has H-PGDS inhibitory activity, and thus can be used as a therapeutic agent or a preventive agent for a disease involving H-PGDS.

## Claims

1. A compound represented by Formula (I): 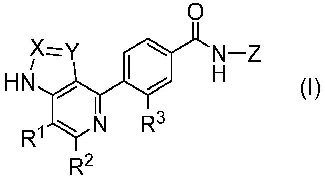 [wherein
-X= represents -CH= or -N=;
=Y- represents =CH- or =N-;
R¹ and R² each independently represent a hydrogen atom, a halogen atom, a cyano group, a hydroxy group, a sulfanyl group, an amino group, a carbamoyl group, a sulfamoyl group, a carboxy group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, or a C₃₋₆ cycloalkyl group;
R³ represents a hydrogen atom or a halogen atom; and
Z is a group represented by Formula (II): 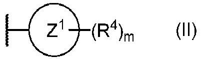 (wherein
the wavy line represents the point of attachment to the nitrogen atom;
ring Z¹ represents a C₆₋₁₀ aryl group, a C₃₋₁₀ cycloalkyl group, a 5- to 10-membered heteroaryl group, or a 4- to 10-membered heterocyclyl group;
m represents 0, 1, or 2; and
R⁴ represents (when there are multiple R⁴'s, each R⁴ independently represents) a halogen atom, a cyano group, a hydroxy group, a sulfanyl group, an amino group, a carbamoyl group, a sulfamoyl group, a carboxy group, a formyl group, an azide group, a hydrazinyl group, a nitro group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, a C₂₋₇ alkanoyl group, a halo C₂₋₇ alkanoyl group, a hydroxy C₂₋₇ alkanoyl group, a C₂₋₇ alkoxycarbonyl group, a mono C₁₋₆ alkylcarbamoyl group, a di C₁₋₆ alkylcarbamoyl group, a C₁₋₆ alkylsulfonyl group, a C₁₋₆ alkylsulfonyloxy group, a halo C₁₋₆ alkylsulfonyloxy group, a mono C₁₋₆ alkylsulfamoyl group, a di C₁₋₆ alkylsulfamoyl group, a mono C₂₋₇ alkanoylamino group, a (C₂₋₇ alkanoyl) C₁₋₆ alkylamino group, a di C₂₋₇ alkanoylamino group, a mono C₁₋₆ alkylsulfonylamino group, a mono C₂₋₇ alkoxycarbonylamino group, or an oxo group.)
or 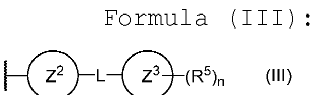 (wherein
the wavy line represents the point of attachment to the nitrogen atom;
ring Z² represents a C₆₋₁₀ aryl group, a C₃₋₁₀ cycloalkyl group, a 5- to 10-membered heteroaryl group, or a 4- to 10-membered heterocyclyl group;
L represents a single bond, a C₁₋₆ alkanediyl group, a hydroxy C₁₋₆ alkanediyl group, a carbonyl group, or a sulfonyl group;
ring Z³ represents a C₆₋₁₀ aryl group, a C₃₋₁₀ cycloalkyl group, a 5- to 10-membered heteroaryl group, or a 4- to 10-membered heterocyclyl group;
n represents 0, 1, or 2; and
R⁵ represents (when there are multiple R⁵'s, each R⁵ independently represents)
a halogen atom, a cyano group, a hydroxy group, a sulfanyl group, an amino group, a carbamoyl group, a sulfamoyl group, a carboxy group, a formyl group, an azide group, a hydrazinyl group, a nitro group, a C₁₋₆ alkyl group, a C₂₋₆ alkenyl group, a halo C₁₋₆ alkyl group, a hydroxy C₁₋₆ alkyl group, a hydroxyhalo C₁₋₆ alkyl group, a C₁₋₆ alkoxy group, a halo C₁₋₆ alkoxy group, a hydroxy C₁₋₆ alkoxy group, a (C₁₋₆ alkoxy) C₁₋₆ alkyl group, a (halo C₁₋₆ alkoxy) C₁₋₆ alkyl group, a C₁₋₆ alkylthio group, a mono C₁₋₆ alkylamino group, a di C₁₋₆ alkylamino group, a mono (halo C₁₋₆ alkyl) amino group, a C₂₋₇ alkanoyl group, a halo C₂₋₇ alkanoyl group, a hydroxy C₂₋₇ alkanoyl group, a C₂₋₇ alkoxycarbonyl group, a mono C₁₋₆ alkylcarbamoyl group, a di C₁₋₆ alkylcarbamoyl group, a C₁₋₆ alkylsulfonyl group, a C₁₋₆ alkylsulfonyloxy group, a halo C₁₋₆ alkylsulfonyloxy group, a mono C₁₋₆ alkylsulfamoyl group, a di C₁₋₆ alkylsulfamoyl group, a mono C₂₋₇ alkanoylamino group, a (C₂₋₇ alkanoyl) C₁₋₆ alkylamino group, a di C₂₋₇ alkanoylamino group, a mono C₁₋₆ alkylsulfonylamino group, a mono C₂₋₇ alkoxycarbonylamino group, an oxo group, a C₃₋₆ cycloalkyl group, a cyclic ether group, a cyclic amino group, or a halo cyclic amino group.)] or a pharmaceutically acceptable salt thereof.

2. The compound according to claim 1 or a pharmaceutically acceptable salt thereof, wherein Z is a group represented by Formula (II): 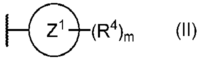 (wherein
the wavy line represents the point of attachment to the nitrogen atom; and
ring Z¹, m, and R⁴ are as defined above.).

3. The compound according to claim 1 or 2 or a pharmaceutically acceptable salt thereof, wherein ring Z¹ is a C₃₋₁₀ cycloalkyl group.

4. The compound according to any one of claims 1 to 3 or a pharmaceutically acceptable salt thereof, wherein ring Z¹ is a
group represented by Formula: 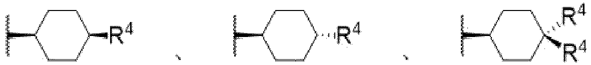 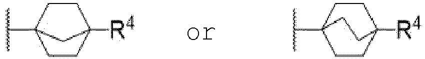 (wherein
the wavy line represents the point of attachment to the nitrogen atom; and
R⁴ is as defined above.).

5. The compound according to any one of claims 1 to 4 or a pharmaceutically acceptable salt thereof, wherein R¹ is a hydrogen atom, a halogen atom, a cyano group, a C₁₋₆ alkyl group, or a halo C₁₋₆ alkyl group, and R² is a hydrogen atom.

6. The compound according to any one of claims 1 to 5 or a pharmaceutically acceptable salt thereof, wherein R⁴ represents (when there are multiple R⁴'s, each R⁴ independently represents) a cyano group, a hydroxy group, a carbamoyl group, a C₁₋₆ alkyl group, a hydroxy C₁₋₆ alkyl group, or a hydroxy C₁₋₆ alkoxy group.

7. The compound according to any one of claims 1 to 6 or a pharmaceutically acceptable salt thereof, wherein -X= is -CH=, and =Y- is =CH-.

8. The compound according to any one of claims 1 to 6 or a pharmaceutically acceptable salt thereof, wherein -X= is -N=, and =Y- is =CH-.

9. The compound according to claim 1 or a pharmaceutically acceptable salt thereof, which is selected from (1) to (49) below:
(1) N-(4-hydroxybicyclo[2.2.2]octan-1-yl)-4-(7-methyl-1H-pyrrolo[3,2-c]pyridin-4-yl)benzamide;
(2) N-(trans-4-hydroxy-4-methylcyclohexyl)-4-(7-methyl-1H-pyrrolo[3,2-c]pyridin-4-yl)benzamide;
(3) N-[trans-4-(1-hydroxycyclopropyl)cyclohexyl]-4-(7-methyl-1H-pyrrolo[3,2-c]pyridin-4-yl)benzamide;
(4) N-[trans-4-(2-hydroxy-2-methylpropoxy)cyclohexyl]-4-(7-methyl-1H-pyrrolo[3,2-c]pyridin-4-yl)benzamide;
(5) 3-fluoro-N-(trans-4-hydroxy-4-methylcyclohexyl)-4-(1H-pyrrolo[3,2-c]pyridin-4-yl)benzamide;
(6) 3-fluoro-N-(4-hydroxybicyclo[2.2.2]octan-1-yl)-4-(1H-pyrrolo[3,2-c]pyridin-4-yl)benzamide;
(7) N-(4-cyanobicyclo[2.2.2]octan-1-yl)-3-fluoro-4-(1H-pyrrolo[3,2-c]pyridin-4-yl)benzamide;
(8) 4-[3-fluoro-4-(1H-pyrrolo[3,2-c]pyridin-4-yl)benzamide]bicyclo[2.2.2]octane-1-carboxamide;
(9) 3-fluoro-N-[trans-4-(2-hydroxy-2-methylpropoxy)cyclohexyl]-4-(1H-pyrrolo[3,2-c]pyridin-4-yl)benzamide;
(10) 3-fluoro-N-(4-hydroxybicyclo[2.2.1]heptan-1-yl)-4-(1H-pyrrolo[3,2-c]pyridin-4-yl)benzamide;
(11) 3-fluoro-N-[trans-4-(1-hydroxycyclopropyl)cyclohexyl]-4-(1H-pyrrolo[3,2-c]pyridin-4-yl)benzamide;
(12) 3-fluoro-N-(trans-4-hydroxycyclohexyl)-4-(1H-pyrrolo[3,2-c]pyridin-4-yl)benzamide;
(13) 4-(7-fluoro-1H-pyrrolo[3,2-c]pyridin-4-yl)-N-[trans-4-(2-hydroxypropan-2-yl)cyclohexyl]benzamide;
(14) 4-(7-chloro-1H-pyrrolo[3,2-c]pyridin-4-yl)-N-[trans-4-(2-hydroxypropan-2-yl)cyclohexyl]benzamide;
(15) 4-(7-cyano-1H-pyrrolo[3,2-c]pyridin-4-yl)-N-[trans-4-(2-hydroxypropan-2-yl)cyclohexyl]benzamide;
(16) 4-(7-fluoro-1H-pyrrolo[3,2-c]pyridin-4-yl)-N-(4-hydroxybicyclo[2.2.2]octan-1-yl)benzamide;
(17) 4-(7-chloro-1H-pyrrolo[3,2-c]pyridin-4-yl)-N-(4-hydroxybicyclo[2.2.2]octan-1-yl)benzamide;
(18) 4-[7-(difluoromethyl)-1H-pyrrolo[3,2-c]pyridin-4-yl]-N-[trans-4-(2-hydroxypropan-2-yl)cyclohexyl]benzamide;
(19) 4-(7-fluoro-1H-pyrrolo[3,2-c]pyridin-4-yl)-N-(4-hydroxybicyclo[2.2.1]heptan-1-yl)benzamide;
(20) 4-(7-fluoro-1H-pyrrolo[3,2-c]pyridin-4-yl)-N-(trans-4-hydroxycyclohexyl)benzamide;
(21) 4-(7-fluoro-1H-pyrrolo[3,2-c]pyridin-4-yl)-N-(trans-4-hydroxy-4-methylcyclohexyl)benzamide;
(22) 4-(7-fluoro-1H-pyrrolo[3,2-c]pyridin-4-yl)-N-[trans-4-(2-hydroxy-2-methylpropoxy)cyclohexyl]benzamide;
(23) 4-(7-fluoro-1H-pyrrolo[3,2-c]pyridin-4-yl)-N-(cis-4-hydroxycyclohexyl)benzamide;
(24) 4-(7-fluoro-1H-pyrrolo[3,2-c]pyridin-4-yl)-N-(tetrahydropyran-4-yl)benzamide;
(25) N-(1,1-dioxotetrahydrothiopyran-4-yl)-4-(7-fluoro-1H-pyrrolo[3,2-c]pyridin-4-yl)benzamide;
(26) N-(trans-4-cyanocyclohexyl)-4-(7-fluoro-1H-pyrrolo[3,2-c]pyridin-4-yl)benzamide;
(27) N-(trans-4-carbamoylcyclohexyl)-4-(7-fluoro-1H-pyrrolo[3,2-c]pyridin-4-yl)benzamide;
(28) 4-(7-chloro-1H-pyrrolo[3,2-c]pyridin-4-yl)-N-(tetrahydropyran-4-yl)benzamide;
(29) 4-(7-chloro-1H-pyrrolo[3,2-c]pyridin-4-yl)-N-(1,1-dioxotetrahydrothiopyran-4-yl)benzamide;
(30) 4-(7-chloro-1H-pyrrolo[3,2-c]pyridin-4-yl)-N-(trans-4-hydroxycyclohexyl)benzamide;
(31) 4-(7-chloro-1H-pyrrolo[3,2-c]pyridin-4-yl)-N-(cis-4-hydroxy-4-methylcyclohexyl)benzamide;
(32) 4-(7-chloro-1H-pyrrolo[3,2-c]pyridin-4-yl)-N-(4-hydroxybicyclo[2.2.1]heptan-1-yl)benzamide;
(33) 4-(7-chloro-1H-pyrrolo[3,2-c]pyridin-4-yl)-N-(cis-4-hydroxycyclohexyl)benzamide;
(34) 4-(7-chloro-1H-pyrrolo[3,2-c]pyridin-4-yl)-N-(trans-4-hydroxy-4-methylcyclohexyl)benzamide;
(35) 3-fluoro-4-(7-fluoro-1H-pyrrolo[3,2-c]pyridin-4-yl)-N-[trans-4-(2-hydroxypropan-2-yl)cyclohexyl]benzamide;
(36) 3-fluoro-4-(7-fluoro-1H-pyrrolo[3,2-c]pyridin-4-yl)-N-(4-hydroxybicyclo[2.2.2]octan-1-yl)benzamide;
(37) 3-fluoro-4-(7-fluoro-1H-pyrrolo[3,2-c]pyridin-4-yl)-N-(trans-4-hydroxy-4-methylcyclohexyl)benzamide;
(38) 3-fluoro-4-(7-fluoro-1H-pyrrolo[3,2-c]pyridin-4-yl)-N-(tetrahydropyran-4-yl)benzamide;
(39) 3-fluoro-4-(7-fluoro-1H-pyrrolo[3,2-c]pyridin-4-yl)-N-(trans-4-hydroxycyclohexyl)benzamide;
(40) 3-fluoro-4-(7-fluoro-1H-pyrrolo[3,2-c]pyridin-4-yl)-N-(cis-4-hydroxy-4-methylcyclohexyl)benzamide;
(41) 3-fluoro-4-(7-fluoro-1H-pyrrolo[3,2-c]pyridin-4-yl)-N-(4-hydroxybicyclo[2.2.1]heptan-1-yl)benzamide;
(42) 3-fluoro-4-(7-fluoro-1H-pyrrolo[3,2-c]pyridin-4-yl)-N-(cis-4-hydroxycyclohexyl)benzamide;
(43) N-[trans-4-(2-hydroxypropan-2-yl)cyclohexyl]-4-(1H-pyrazolo[4,3-c]pyridin-4-yl)benzamide;
(44) N-[trans-4-(2-hydroxypropan-2-yl)cyclohexyl]-4-(1H-imidazo[4,5-c]pyridin-4-yl)benzamide;
(45) 4-(1H-[1,2,3]triazolo[4,5-c]pyridin-4-yl)-N-[trans-4-(2-hydroxypropan-2-yl)cyclohexyl]benzamide;
(46) 4-(7-fluoro-1H-pyrazolo[4,3-c]pyridin-4-yl)-N-[trans-4-(2-hydroxypropan-2-yl)cyclohexyl]benzamide;
(47) 4-(7-fluoro-1H-pyrazolo[4,3-c]pyridin-4-yl)-N-(4-hydroxybicyclo[2.2.2]octan-1-yl)benzamide;
(48) 4-(7-chloro-1H-pyrazolo[4,3-c]pyridin-4-yl)-N-[trans-4-(2-hydroxypropan-2-yl)cyclohexyl]benzamide; and
(49) 3-fluoro-N-[trans-4-(2-hydroxypropan-2-yl)cyclohexyl]-4-(1H-pyrazolo[4,3-c]pyridin-4-yl)benzamide.

10. A pharmaceutical composition, comprising: the compound according to any one of claims 1 to 9 or a pharmaceutically acceptable salt thereof.

11. A pharmaceutical composition for preventing or treating a disease involving H-PGDS, comprising: the compound according to any one of claims 1 to 9 or a pharmaceutically acceptable salt thereof.

12. The pharmaceutical composition according to claim 11, wherein the disease involving H-PGDS is selected from the group consisting of asthma, chronic obstructive pulmonary disease, allergic rhinitis, sinusitis, eosinophilic pneumonia, atherosclerosis, rheumatoid arthritis, cystic fibrosis, actinic keratosis, chronic urticaria, dermatitis, muscular dystrophy, sarcopenia, disuse muscle atrophy, muscle damage, wounds, dermatomyositis, amyotrophic lateral sclerosis, cerebral infarction, myocardial infarction, ischemic bowel disease, ischemic renal disease, ischemic stomach disease, ischemic liver disease, diabetic ischemic limb, and Buerger's disease.

13. An H-PGDS inhibitor, comprising: the compound according to any one of claims 1 to 9 or a pharmaceutically acceptable salt thereof.
